(19) Europäisches Patentamt
European Patent Office
Office européen des brevets

(11) EP 0 854 863 B1

(12) **EUROPEAN PATENT SPECIFICATION**

(45) Date of publication and mention of the grant of the patent:
**30.10.2002 Bulletin 2002/44**

(21) Application number: **96924136.3**

(22) Date of filing: **18.07.1996**

(51) Int Cl.7: **C07C 271/22**, A61K 31/16, A61K 31/21, C07C 235/34, C07C 237/22, C07D 307/80, C07D 209/42, C07D 215/48, C07D 405/12, C07D 401/12, C07D 401/06

(86) International application number:
**PCT/JP96/01995**

(87) International publication number:
**WO 97/003951 (06.02.1997 Gazette 1997/07)**

(54) **ESTERS AND AMIDES AS PLA2 INHIBITORS**

ESTER UND AMIDE ALS PLA2-INHIBITOREN

ESTERS ET AMIDES UTILISES EN TANT QU'INHIBITEURS ANTI-PLA2

(84) Designated Contracting States:
**AT BE CH DE DK ES FI FR GB GR IE IT LI LU NL PT SE**

(30) Priority: **24.07.1995 GB 9515162**
**14.05.1996 AU PN983596**

(43) Date of publication of application:
**29.07.1998 Bulletin 1998/31**

(73) Proprietor: **FUJISAWA PHARMACEUTICAL CO., LTD.**
**Osaka-shi Osaka 541-8514 (JP)**

(72) Inventors:
- **HEMMI, Keiji**
  **Deceased (JP)**
- **FUKAMI, Naoki**
  **Ibaraki 300-34 (JP)**
- **YOSHIMURA, Seiji**
  **Ibaraki 305 (JP)**
- **IMAI, Keisuke**
  **Tsukuba-shi Ibaraki 305 (JP)**

(74) Representative:
**Gille Hrabal Struck Neidlein Prop Roos**
**Patentanwälte**
**Brucknerstrasse 20**
**40593 Düsseldorf (DE)**

(56) References cited:
**WO-A-93/21211**
**WO-A-95/19959**
**US-A- 4 792 555**

Remarks:
The file contains technical information submitted after the application was filed and not included in this specification

EP 0 854 863 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

TECHNICAL FIELD

The present invention relates to a novel fatty acid derivative of general formula (I) and a pharmaceutically acceptable salt thereof which are useful as a medicament.

BACKGROUND ART

A phospholipase $A_2$ inhibitor having the structure of that of the present invention has not been known.
US-A-4 792 555 and WO-A-9321211 disclose fatty acid derivatives useful as phospholipase A2 inhibitors, which , however, are structurally different from those of the present invention.
WO-A-9519952, which is a document according to Article 54(3) and (4)EPC, discloses fatty acid derivatives having the same activity. These compounds are disclaimed from the present invention by the proviso defined in claim 1.

DISCLOSURE OF INVENTION

The present invention relates to novel fatty acid derivative and a pharmaceutically acceptable salt thereof which are phospholipase $A_2$ inhibitors and are useful for the prevention and/or the treatment of pancreatitis, hepatitis, chronic renal failure; shock (e.g. endotoxin shock, gram-negative septic shock), arthritis (e.g. rheumatoid arthritis, osteoarthritis), respiratory disease (e.g. bronchial asthma, bronchitis, adult respiratory distress syndrome), heart disease (e.g. myocardial ischemia), allergic disease, thrombosis, arteriosclerosis, pain, autoimmune disease, dermal disease (e.g. atopic dermatitis, psoriasis, contact dermatitis), inflammatory bowel disease (e.g. Crohn's disease, ulcerative colitis), ophthalmic disease (e.g. allergic ophthalmic disease, inflammatory ophthalmic diseases), nasal diseases (e. g. allergic rhinitis), gout, trauma induced inflammation (e.g. spinal cord injury), liver diseases (e.g. cirrhosis, hepatitis) ; to a process for preparation thereof, to a pharmaceutical composition comprising the same, and to a use of the same for the manufacture of a medicament for the prevention and/or treatment of the aforesaid diseases.
The object fatty acid derivative can be represented by the following formula (I) :

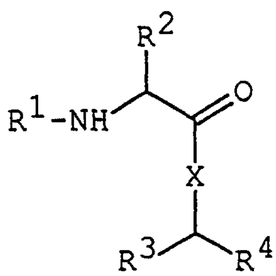

(I)

wherein $R^1$, $R^2$, $R^3$, $R^4$, $R^5$ and X are defined as in claim 1, with proviso that X is

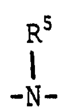

(wherein $R^5$ is as defined above), when $R^3$ is ($C_3$-$C_6$) alkyl or ($C_7$-$C_{16}$) alkyl.
It is to be noted the object compound (I) may include one or more stereoisomers due to asymmetric carbon atom(s) and double bond, and all of such isomers and a mixture thereof are included within the scope of the present invention.
It is further to be noted isomerization or rearrangement of the object compound (I) may occur due to the effect of the light, acid, or base, and the compound obtained as the result of said isomerization or rearrangement is also included within the scope of the present invention.
It is also to be noted that the solvating form of the compound (I) (e.g. hydrate) and any form of the crystal of the compound (I) are included within the scope of the present invention.
The object compound (I) or a salt thereof can be prepared according to the following reaction schemes.

Process 1

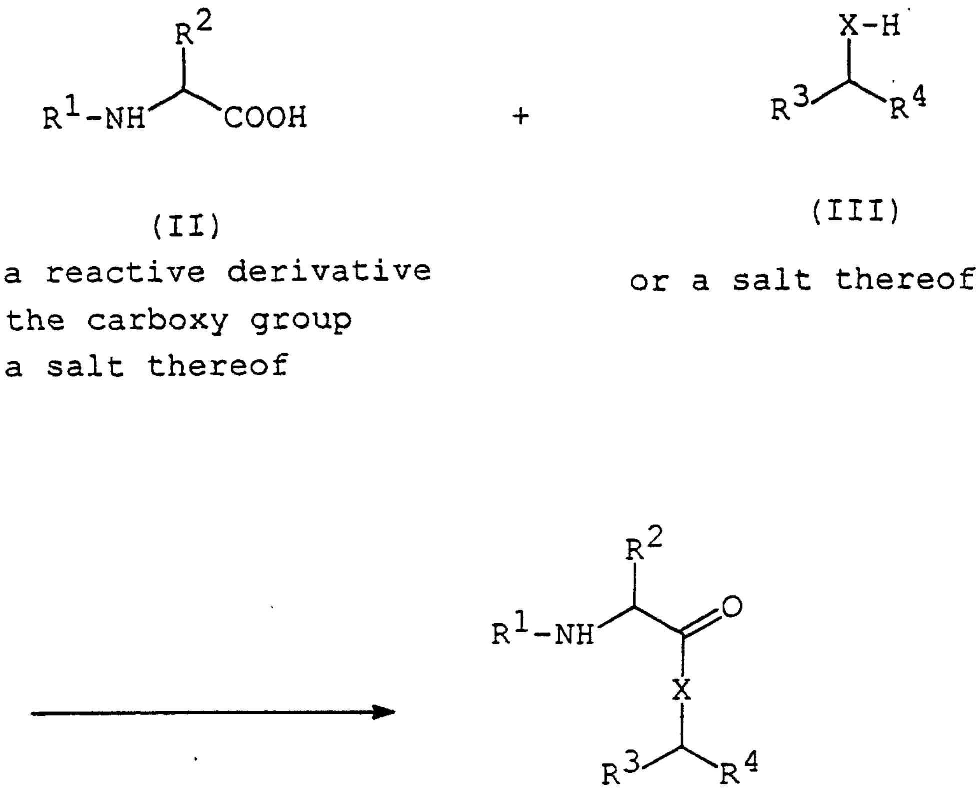

(II)
or a reactive derivative at the carboxy group or a salt thereof

(III)
or a salt thereof

(I)
or a salt thereof

Process 2

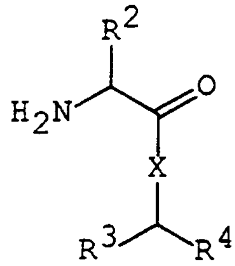

+

$R^1$-OH

(IV)

or a reactive derivative at the amino group or a salt thereof

(V)

or a reactive derivative or a salt thereof

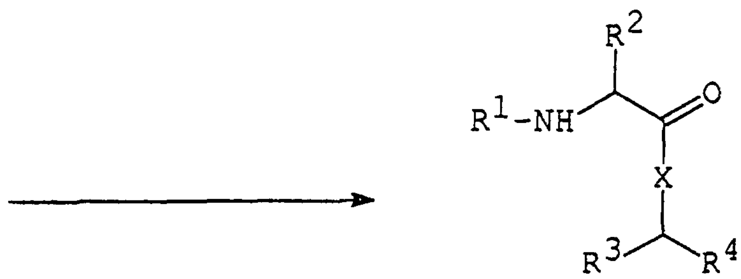

(I)

or a salt thereof

Process 3

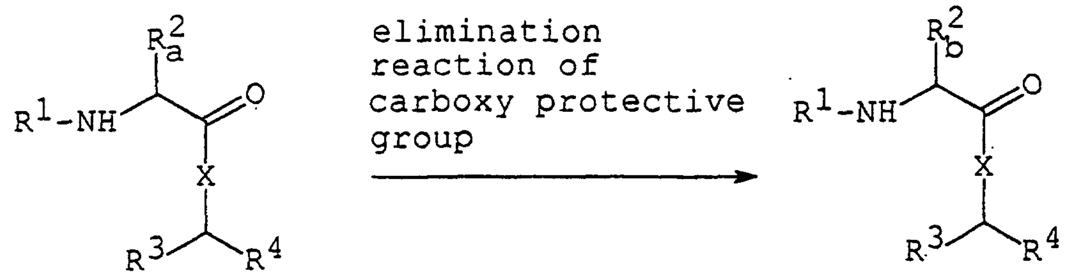


(Ia)
or a salt thereof

(Ib)
or a salt thereof

wherein

$R^1$, $R^2$, $R^3$, $R^4$ and X are each as defined above,
$R^2_a$ is protected carboxy ($C_1$-$C_6$) alkyl,
$R^2_b$ is carboxy($C_1$-$C_6$)alkyl.

The starting compound (IV) or a salt thereof can be prepared according to the following reaction scheme.

Process A

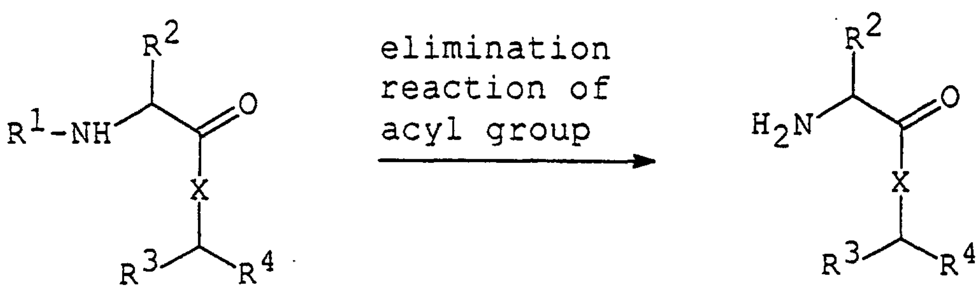


(I)
or a salt thereof

(IV)
or a salt thereof

wherein $R^1$, $R^2$, $R^3$, $R^4$ and X are each as defined above.

Among the starting compounds, there are some novel compounds. They can be prepared according to the methods as described in Preparations in the present specification or the conventional manners in this field of the art.

Suitable pharmaceutically acceptable salts of the object compound (I) are conventional ones and include a metal salt such as an alkali metal salt (e.g. sodium salt, potassium salt) and an alkaline earth metal salt (e.g. calcium salt, magnesium salt), an ammonium salt, an organic base salt (e.g. trimethylamine salt, triethylamine salt, pyridine salt, picoline salt, dicyclohexylamine salt, N,N'-dibenzylethylenediamine salt), an organic acid salt (e.g. acetate, trifluoroacetate, maleate, tartrate, fumarate, methanesulfonate, benzenesulfonate, formate, toluenesulfonate), an inorganic acid salt (e.g. hydrochloride, hydrobromide, hydriodide, sulfate, phosphate ), a salt with an amino acid (e.g. arginine, aspartic acid, glutamic acid).

In the above and following descriptions of the present specification, suitable examples and illustrations of the various definitions which the present invention includes within the scope thereof are explained in detail as follows.

Suitable example of "$C_1$-$C_6$ alkyl" and "$C_1$-$C_6$ alkyl" moiety in the terms used in the present specification may include straight or branched one such as methyl, ethyl, propyl, isopropyl, butyl, isobutyl, t-butyl, pentyl, isopentyl, hexyl .

Suitable "($C_2$-$C_4$) alkenyl" and "($C_2$-$C_4$) alkenyl" moiety in the terms used in the present specification may include vinyl, 1-(or 2-)propenyl, 1-(or 2- or 3-)butenyl, methylvinyl, ethylvinyl, and 1-(or 2- or 3-)methyl-1-(or 2-)-propenyl.

Suitable "$C_7$-$C_{20}$ alkyl" and "$C_7$-$C_{20}$ alkyl" moiety in the terms used in the present specification may include straight or branched one such as heptyl, 2-methylheptyl, octyl, nonyl, decyl, undecyl, dodecyl, tridecyl, 11-methyldodecyl, 12-methyltridecyl, tetradecyl, pentadecyl, hexadecyl, heptadecyl, octadecyl, nonadecyl, eicosyl , in which the preferred one may be ($C_7$-$C_{16}$)alkyl, and the more preferred one may be heptyl, octyl, nonyl, decyl, or tridecyl.

Suitable "halogen" may include fluorine, chlorine, bromine, iodine, in which more preferable one may be chlorine.

$R^1$ may preferably be

(1) ($C_1$-$C_4$)alkoxycarbonyl, and the most preferred one may be t-butoxycarbonyl;

(2) phenyl ($C_1$-$C_4$)alkanoyl or naphthyl ($C_1$-$C_4$)alkanoyl, each of which may have carboxy($C_1$-$C_4$)alkyl (e.g. carboxymethyl, 2-carboxyethyl, 1-carboxypropyl, 4-carboxybutyl, etc), ($C_1$-$C_4$)alkoxycarbonyl ($C_1$-$C_4$) alkyl (e.g. methoxycarbonylmethyl, 2-methoxycarbonylethyl, 2-(t-butoxycarbonyl)ethyl) which may be substituted by phenyl, ($C_1$-$C_4$)alkoxycarbonyl($C_2$-$C_4$)alkenyl (e.g. 2-methoxycarbonylvinyl), carbamoyl($C_1$-$C_4$)alkyl (e.g. 2-carbamoylethyl), and phenyl($C_1$-$C_4$)alkyl (e.g. benzyl, phenethyl), the much more preferred one may be phenyl($C_1$-$C_4$)alkanoyl which may have substituent selected from the group consisting of carboxymethyl, 2-carboxyethyl, methoxycarbonylmethyl, benzyloxycarbonylmethyl, 2-methoxycarbonylethyl, 2-(t-butoxycarbonyl)ethyl, 2-methoxycarbonylvinyl, 2-carbamoylethyl, benzyl, and phenethyl, or naphthyl($C_1$-$C_4$)alkanoyl which may have benzyl, the most preferred one may be benzoyl, 2-(carboxymethyl)benzoyl, 2-(2-carboxyethyl)benzoyl, 2-(methoxycarbonylmethyl) benzoyl, 2-(benzyloxycarbonylmethyl)benzoyl, 2-(2-methoxycarbonylethyl)benzoyl, 2-[2-(t-butoxycarbonyl)ethyl] benzoyl, 2-(2-methoxycarbonylvinyl)benzoyl, 2-(2-carbamoylethyl)benzoyl, 2-benzylbenzoyl, 3-benzylbenzoyl, 2-phenethylbenzoyl, 2-naphthoyl, or 3-benzylnaphthalen-2-ylcarbonyl; or

(3) quinolyl($C_1$-$C_4$)alkanoyl, isoquinolyl($C_1$-$C_4$)alkanoyl, or indolyl($C_1$ - $C_4$)alkanoyl which may have ($C_1$-$C_4$)alkylphenyl($C_1$-$C_4$)alkyl, halophenyl($C_1$-$C_4$)alkyl, or pyridyl($C_1$-$C_4$)alkyl, the much more preferred one may be quinolylcarbonyl, isoquinolylcarbonyl, or indolylcarbonyl which may have benzyl, 1-naphthylmethyl, 4-methylbenzyl, 2-chlorobenzyl, 3-chlorobenzyl, 4-chlorobenzyl or 2-pyridylmethyl, the most preferred one may be 2-(or 3-)quinolylcarbonyl, 1-(or 3-)isoquinolylcarbonyl, 1-benzylindol-2-(or 3-)ylcarbonyl, 1-(1-naphthylmethyl)indol-3-ylcarbonyl, 1-(4-methylbenzyl)indol-2-(or 3-)ylcarbonyl, 1-[2-(or 3- or 4-)chlorobenzyl]indol-3-ylcarbonyl or 1-(2-pyridylmethyl)indol-3-ylcarbonyl.

In the following, some of the preferred embodiments of the fatty acid derivative (I) of the present invention are shown.

(1) the derivative (I), wherein

$R^1$ is ($C_1$-$C_4$)alkoxycarbonyl;

phenyl($C_1$-$C_4$)alkanoyl or naphthyl($C_1$-$C_4$)alkanoyl, each of which may have carboxy($C_1$-$C_4$)-alkyl, ($C_1$-$C_4$)alkoxycarbonyl($C_1$-$C_4$)alkyl which may be substituted by phenyl, ($C_1$-$C_4$)alkoxycarbonyl($C_2$-$C_4$)alkenyl, carbamoyl($C_1$-$C_4$)alkyl or phenyl($C_1$-$C_4$)alkyl;

heterocyclic($C_1$-$C_4$)alkanoyl which may have pyridyl($C_1$-$C_4$)alkyl, naphthyl($C_1$-$C_4$)alkyl or phenyl($C_1$-$C_4$) alkyl which may have 1 to 3 suitable substituent(s) selected from the group consisting of ($C_1$-$C_4$)alkyl and chloro, in which the heterocyclic moiety is indolyl, quinolyl or isoquinolyl,

$R^2$ is carboxy($C_1$-$C_4$)alkyl, methoxycarbonyl($C_1$-$C_4$)alkyl, or benzyloxycarbonyl($C_1$-$C_4$)alkyl,

$R^3$ is phenyl($C_1$-$C_4$)alkyl which may have ($C_1$-$C_4$)alkyl, ($C_7$-$C_{16}$)alkyl or phenyl; or

naphthyl($C_1$-$C_4$)alkyl which may have ($C_1$-$C_4$)alkyl,

$R^4$ is carbamoyl($C_1$-$C_4$)alkyl, and

X is -O-.

(2) the derivative (I), wherein

$R^1$, $R^2$, $R^3$ and $R^4$ are each as defined above in (1), and

X is -NH- or

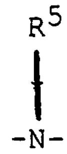

[wherein $R^5$ is $(C_1\text{-}C_5)$alkyl, phenyl$(C_1\text{-}C_4)$alkyl, or pyridyl$(C_1\text{-}C_4)$alkyl].

(3) the derivative (I), wherein

$R^1$, $R^2$, $R^4$ and X are each as defined above in (1), and
$R^3$ is phenyl$(C_7\text{-}C_{16})$alkyl.

(4) the derivative (I), wherein

$R^1$, $R^2$, $R^3$ and $R^4$ are each as defined above in (3), and
X is -NH- or

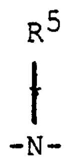

[wherein $R^5$ is as defined above in (2)].

(5) the derivative (I), wherein

$R^1$, $R^2$, $R^4$ and X are each as defined above in (1), and
$R^3$ is benzofuranyl$(C_1\text{-}C_4)$alkyl.

(6) the derivative (I), wherein

$R^1$, $R^2$, $R^3$ and $R^4$ are each as defined above in (5), and
X is -NH- or

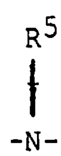

[wherein $R^5$ is as defined above in (2)].

(7) the derivative (I), wherein

$R^1$, $R^2$, $R^4$ and X are each as defined above in (1), and
$R^3$ is $(C_7\text{-}C_{16})$alkoxy$(C_1\text{-}C_4)$alkyl.

(8) the derivative (I), wherein

$R^1$, $R^2$, $R^3$ and $R^4$ are each as defined above in (7), and X is -NH- or

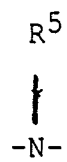

[wherein $R^5$ is as defined above in (2)].

(9) the derivative (I), wherein

$R^1$, $R^2$, $R^4$ and X are each as defined above in (1), and $R^3$ is $(C_3\text{-}C_6)$alkyl.

(10) the derivative (I), wherein

$R^1$, $R^2$, $R^3$ and $R^4$ are each as defined above in (9), and X is

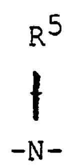

[wherein $R^5$ is as defined above in (2)].

(11) the derivative (I), wherein

$R^1$, $R^2$, $R^4$ and X are each as defined above in (1), and $R^3$ is $(C_7\text{-}C_{16})$alkyl.

(12) the derivative (I), wherein

$R^1$, $R^2$, $R^3$ and $R^4$ are each as defined above in (11), and X is

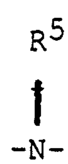

[wherein $R^5$ is as defined above in (4)].

The processes for preparing the object compound (I) of the present invention are explained in detail in the following.

<u>Process 1</u>

The compound (I) or a salt thereof can be prepared by reacting the compound (II) or a reactive derivative at

the carboxy group or a salt thereof with the compound (III) or a salt thereof.

Suitable salts of the compounds (II) and (III) can be referred to the ones as exemplified for the compound (I).

Suitable reactive derivative at the carboxy group of the compound (II) may include an acid halide, an acid anhydride, an activated amide, and an activated ester. Suitable examples of the reactive derivatives may be an acid chloride; an acid azide; a mixed acid anhydride with an acid such as substituted phosphoric acid [e.g. dialkylphosphoric acid, phenylphosphoric acid, diphenylphosphoric acid, dibenzylphosphoric acid, halogenated phosphoric acid], dialkylphosphorous acid, sulfurous acid, thiosulfuric acid, sulfuric acid, sulfonic acid [e.g. methanesulfonic acid ], aliphatic carboxylic acid [e.g. acetic acid, propionic acid, butyric acid, isobutyric acid, pivalic acid, pentanoic acid, isopentanoic acid, 2-ethylbutyric acid, trichloroacetic acid] or aromatic carboxylic acid [e.g. benzoic acid ]; a symmetrical acid anhydride; an activated amide with imidazole, 4-substituted imidazole, dimethylpyrazole, triazole, tetrazole or 1-hydroxy-1H-benzotriazole; or an activated ester [e.g. cyanomethyl ester, methoxymethyl ester, trichlorophenyl ester, pentachlorophenyl ester, mesylphenyl ester, phenylazophenyl ester, phenyl thioester, p-nitrophenyl thioester, p-cresyl thioester, carboxymethyl thioester, pyranyl ester, pyridyl ester, piperidyl ester, 8-quinolyl thioester or an ester with a N-hydroxy compound [e.g. N,N-dimethylhydroxylamine, 1-hydroxy-2-(1H)-pyridone, N-hydroxysuccinimide, N-hydroxyphthalimide, 1-hydroxy-1H-benzotriazole]. These reactive derivatives can optionally be selected from them according to the kind of the compound (II) to be used.

In the case that the group X is

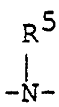

in the compound (III), the compound (III) can be used in the form of its reactive derivative at the amino group.

Suitable said reactive derivative at the amino group may include Schiff's base type imino or its tautomeric enamine type isomer formed by the reaction of the compound (III) with a carbonyl compound such as aldehyde or ketone; a silyl derivative formed by the reaction of the compound (III) with a silyl compound such as bis(trimethylsilyl) acetamide, mono(trimethylsilyl)acetamide or bis(trimethylsilyl)urea; or a derivative formed by reaction of the compound (III) with phosphorus trichloride or phosgene.

The reaction is usually carried out in a conventional solvent such as water, alcohol [e.g. methanol, ethanol], acetone, dioxane, acetonitrile, chloroform, methylene chloride, ethylene chloride, tetrahydrofuran, ethyl acetate, N,N-dimethylformamide, pyridine or any other organic solvent which does not adversely influence the reaction. These conventional solvents may also be used in a mixture with water.

In this reaction, when the compound (II) is used in a free acid form or its salt form, the reaction is preferably carried out in the presence of a conventional condensing agent such as N,N'-dicyclohexylcarbodiimide; 1-ethyl-3-(3-dimethylaminopropyl)carbodiimide; or benzotriazole-1-yloxy-tris-pyrrolidinophosphonium hexafluorophosphate.

The reaction may also be carried out in the presence of an inorganic or organic base such as an alkali metal carbonate, alkali metal bicarbonate, tri($C_1$-$C_6$)alkylamine (e.g. triethylamine), pyridine, di($C_1$-$C_6$)alkylaminopyridine (e.g. N,N-dimethylaminopyridine), N-($C_1$-$C_6$)-alkylmorpholine, or N,N-di($C_1$-$C_6$)alkylbenzylamine.

The reaction temperature is not critical, and the reaction is usually carried out under cooling to warming.

Process 2

The compound (I) or a salt thereof can be prepared by reacting the compound (IV) or a reactive derivative at the amino group or a salt thereof with the compound (V) or a reactive derivative or a salt thereof.

Suitable salts of the compounds (IV) and (V) can be referred to the ones as exemplified for the compound (I).

The reaction of this process can be carried out according to a similar manner to that of Process 1, and so the reaction condition can be referred to the explanation therein.

Process 3

The compound (Ib) or a salt thereof can be prepared by subjecting a compound (Ia) or a salt thereof to elimination reaction of carboxy protective group.

This reaction is carried out in accordance with a conventional method such as hydrolysis, or reduction .

The hydrolysis is preferably carried out in the presence of a base or an acid including Lewis acid.

Suitable base may include an inorganic base and an organic base such as an alkali metal [e.g. sodium, potassium], an alkaline earth metal [e.g. magnesium, calcium], the hydroxide or carbonate or bicarbonate thereof, trialkylamine [e.g. trimethylamine, triethylamine], picoline, 1,5-diazabicyclo[4.3.0]non-5-ene, 1,4-diazabicyclo[2.2.2]octane, or 1,8-diazabicyclo[5.4.0]undec-7-ene.

Suitable acid may include an organic acid [e.g. formic acid, acetic acid, propionic acid, trichloroacetic acid, trifluoroacetic acid] and an inorganic acid [e.g. hydrochloric acid, hydrobromic acid, sulfuric acid, hydrogen chloride, hydrogen bromide].

The elimination using Lewis acid such as trihaloacetic acid [e.g. trichloroacetic acid, trifluoroacetic acid], aluminium halide [e.g. aluminium chloride] is preferably carried out in the presence of cation trapping agents [e.g. anisole, phenol].

The reaction is usually carried out in a solvent such as water, an alcohol [e.g. methanol, ethanol], nitromethane, methylene chloride, tetrahydrofuran, a mixture thereof or any other solvent which does not adversely influence the reaction. A liquid base or acid can be also used as the solvent. The reaction temperature is not critical and the reaction is usually carried out under cooling to warming.

The reduction method applicable for the elimination reaction may include chemical reduction and catalytic reduction.

Suitable reducing agents to be used in chemical reduction are a combination of metal [e.g. tin, zinc, iron ] or metallic compound [e.g. chromium chloride, chromium acetate] and an organic or inorganic acid [e.g. formic acid, acetic acid, propionic acid, trifluoroacetic acid, p-toluenesulfonic acid, hydrochloric acid, hydrobromic acid].

Suitable catalysts to be used in catalytic reduction are conventional ones such as platinum catalysts [e.g. platinum plate, spongy platinum, platinum black, colloidal platinum, platinum oxide, platinum wire], palladium catalysts [e.g. spongy palladium, palladium black, palladium oxide, palladium on carbon, colloidal palladium, palladium on barium sulfate, palladium on barium carbonate], nickel catalysts [e.g. reduced nickel, nickel oxide, Raney nickel ], cobalt catalysts [e.g. reduced cobalt, Raney cobalt ], iron catalysts [e.g. reduced iron, Raney iron], copper catalysts [e.g. reduced copper, Raney copper, Ullman copper].

The reduction is usually carried out in a conventional solvent which does not adversely influence the reaction such as water, methanol, ethanol, propanol, dioxane, N,N-dimethylformamide, or a mixture thereof. Additionally, in case that the above-mentioned acids to be used in chemical reduction are in liquid, they can also be used as a solvent. Further, a suitable solvent to be used in catalytic reduction may be the above-mentioned solvent, and other conventional solvent such as diethyl ether, dioxane, tetrahydrofuran, or a mixture thereof.

The reaction temperature of this reduction is not critical and the reaction is usually carried out under cooling to warming.

It is to be noted the compound (I) or a salt thereof can be prepared by the methods other than aforesaid Processes 1 to 3, for example, by the other methods disclosed in Examples in this specification.

Biological Property of the Compound (I)

In order to show the utility of the object compound (I), the biological test data on phospholipase $A_2$ assay of the representative compound of the compound (I) is shown in the following.

Test on the inhibitory effect against phospholipase $A_2$ ($PLA_2$)

[I] Test Method

$PLA_2$ activity was assayed with a fluorescent phospholipid analogue [1-palmitoyl-2-(10-pyrenyldecanoyl)-*sn*-glycero-3-monomethylphosphatidic acid (10-pyrene PA-monomethyl ester)] as a substrate, according to Radvanyi et al. (1989) with several modifications. Briefly, the reaction medium was prepared by sequential additon of 1160 μl of 50 mM Tris-HCl (pH 7.4) buffer containing 100 mM NaCl and 1 mM EDTA, 10 μl of 120 μM 10-pyrene PA-monomethyl ester in ethanol, 10 μl of drug sample in methanol and 10 μl of 1.2 μg/ml human recombinant $PLA_2$ group II enzyme. The enzymatic reaction was then initiated with 10 μl of 0.84 M $CaCl_2$. Following incubation at room temperature for 10 minutes, the reaction was terminated by addition of 25 μl of 1 M EDTA and 10 μl of 10 mg/ml β-cyclodextrin. Fluorescence measurements were carried out with a JASCO Corporation FP-777 spectrofluorometer. Excitation and emission wavelengths were 345 nm and 380 nm, respectively. All data are the average of at least duplicate determinations corrected for the spontaneous fluorescence of the reaction medium. Data were expressed as percent inhibition.

Reference

F. Radvanyi, L. Jordan, F. Russo-Marie and C. Bon: A sensitive and continuous fluorometric assay for phospholipase $A_2$ using pyrene-labeled phospholipids in the presence of serum albumin. [Anal. Biochem. 177 pages 103-109 (1989)]

[II] Test Compound

(3S)-3-[(2S)-2-(3-Benzylnaphthalen-2-ylcarbonylamino)-5-carboxypentanoyl]oxy-4-(2-naphthyl)butanamide (the compound of Example 24)

[III] Test Result

| Percent inhibition | |
|---|---|
| dose (M) | inhibition (%) |
| 1 x $10^{-6}$ | 100 |

The pharmaceutical composition of the present invention can be used in the form of a pharmaceutical preparation, for example, in solid, semisolid or liquid form, which contains the object compound (I) or a pharmaceutically acceptable salt thereof, as an active ingredient in admixture with an organic or inorganic carrier or excipient suitable for rectal, pulmonary (nasal or buccal inhalation), nasal, ocular, external (topical), oral or parenteral (including subcutaneous, intravenous, intramuscular and intra-articular) administrations or insufflation.
The active ingredient may be compounded, for example, with the usual non-toxic, pharmaceutically acceptable carriers for tablets, pellets, troches, capsules, suppositories, creams, ointments, aerosols, powders for insufflation, solutions, emulsions, suspensions, and any other form suitable for use. And, if necessary, in addition, auxiliary, stabilizing, thickening and coloring agents and perfumes may be used.
The object compound (I) or a pharmaceutically acceptable salt thereof is/are included in the pharmaceutical composition in an amount sufficient to produce the desired effect upon the process or condition of the diseases.
The pharmaceutical composition of the present invention can be manufactured by the conventional method in this field of the art. If necessary, the technique generally used in this field of the art for improving the bioavailability of a drug can be applied to the pharmaceutical composition of the present invention.
For applying the composition to a human being or an animal, it is preferable to apply it by intravenous (including i.v. infusion), intramuscular, pulmonary, or oral administration, or insufflation including aerosols from metered dose inhalator, nebulizer or dry powder inhalator.
While the dosage of therapeutically effective amount of the object compound (I) varies from and also depends upon the age and condition of each individual patient to be treated, in the case of intravenous administration, a daily dose of 0.001-100 mg of the object compound (I) per kg weight of a human being or an animal, in the case of intramuscular administration, a daily dose of 0.001-100 mg of the object compound (I) per kg weight of a human being or an animal, in the case of oral administration, a daily dose of 0.001-200 mg of the object compound (I) per kg weight of a human being or an animal is generally given for the prevention and/or the treatment of aforesaid diseases in a human being or an animal.
The following preparations and examples are given only for the purpose of illustrating the present invention in more detail.

<u>Preparation 1</u>

2-(6-Ethylnaphthalen-2-yl)acetic acid (1.88 g) was dissolved in thionyl chloride (9.6 ml) and the mixture was stirred at room temperature for 1 hour and then the mixture was concentrated in vacuo. The residue was dissolved in methylene chloride (20 ml) and the solution was added dropwise to a stirring solution of Meldrum's acid (1.26 g) and pyridine (1.56 ml) in methylene chloride (20 ml) at room temperature. After being stirred overnight at the same temperature, the mixture was washed with 1N hydrochloric acid and the organic layer was dried over magnesium sulfate and concentrated in vacuo. The residue was dissolved in methanol (40 ml) and refluxed for 2 hours and the mixture was concentrated in vacuo. The residue was dissolved in ethyl acetate and the solution was washed with 1N hydrochloric acid, water, aqueous sodium bicarbonate and brine, successively. The organic layer was dried over magnesium sulfate and concentrated in vacuo. The residue was purified by silica gel column chromatography (ethyl acetate : hexane = 1:9, as an eluent) to give methyl 4-(6-ethyl-2-naphthyl)-3-oxobutanoate (0.61 g).

NMR ($CDCl_3$, δ) : 7.74 (2H, dd, J=8.0, 7.5Hz), 7.64 (1H, s), 7.60 (1H, s), 7.36 (1H, d, J=8.0Hz), 7.28 (1H, d, J=8.0Hz), 3.96 (2H, s), 3.70 (3H, s), 3.46 (2H, s), 2.80 (2H, q, J=7.5Hz), 1.30 (3H, t, J=7.5Hz)
ESI-MS : 271 [M+H]

Preparation 2

Methyl 4-(2-naphthyl)-3-oxobutanoate was obtained according to a similar manner to that of Preparation 1.

NMR ($CDCl_3$, δ) : 7.82 (3H, m), 7.70 (1H, s), 7.48 (2H, m), 7.32 (1H, m), 4.00 (2H, s), 3.70 (3H, s), 3.48 (2H, s)

Preparation 3

Methyl 4-(6-ethyl-2-naphthyl)-3-oxobutanoate (0.60 g), D-camphorsulfonic acid (4.1 mg) and [Ru2Cl2((S)-BINAP)2]NEt3 (di[(S)-2,2'-bis(diphenylphosphino)-1,1'-binaphthyl]-dichlorodirhutenium triethylamine complex) (4.5 mg) in methanol (6 ml) was hydrogenated at 65°C under hydrogen atmosphere under 10 atmosphere for 5 hours. After cooling at room temperature, the solvent was removed in vacuo and the residue was purified by silica gel column chromatography (ethyl acetate : hexane = 1:4, as an eluent) to give methyl (3S)-4-(6-ethyl-2-naphthyl)-3-hydroxybutanoate (0.55 g).

NMR ($CDCl_3$, δ) : 7.72 (2H, dd, J=8.0, 6.0Hz), 7.64 (1H, s), 7.60 (1H, s), 7.32 (2H, t, J=7.5Hz), 4.36 (1H, br s), 3.70 (3H, s), 2.96 (2H, ABX), 2.80 (2H, q, J=7.5Hz), 2.52 (2H, ABX), 1.30 (3H, t, J=7.5Hz)
ESI-MS : 273 [M+H]

The following compounds (Preparations 4 and 5) were obtained according to a similar manner to that of Preparation 3.

Preparation 4

Methyl (3R)-3-hydroxy-4-(2-naphthyl)butanoate

NMR ($CDCl_3$, δ) : 7.74-7.84 (3H, m), 7.66 (1H, s), 7.4-7.5 (2H, m), 7.34 (1H, d, J=8.0Hz), 4.36 (1H, m), 3.70 (3H, s), 2.98 (2H, ABX), 2.87 (1H, d, J=5.0Hz), 2.52 (2H, ABX)
ESI-MS : 245 [M+H]

Preparation 5

Methyl (3S)-3-hydroxy-4-(2-naphthyl)butanoate

NMR ($CDCl_3$, δ) : 7.74-7.84 (3H, m), 7.66 (1H, s), 7.4-7.5 (2H, m), 7.34 (1H, d, J=8.0Hz), 4.36 (1H, m), 3.70 (3H, s), 2.98 (2H, ABX), 2.87 (1H, d, J=5.0Hz), 2.52 (2H, ABX)
ESI-MS : 245 [M+H]

Preparation 6

To a solution of methyl (3R)-3-hydroxy-4-(2-naphthyl)butanoate (3.02 g) and methanesulfonyl chloride (2.12 g) in methylene chloride (60 ml) was added triethylamine (2.5 g) at 0°C. After being stirred at room temperature for 1.5 hours, the mixture was diluted with ethyl ether and the mixture was washed with 0.5N hydrochloric acid, water, aqueous sodium bicarbonate and brine, successively. The organic layer was dried over magnesium sulfate and concentrated in vacuo. The residue was dissolved in DMF (50 ml) and sodium azide (2.02 g) was added to this solution. After being stirred at 60°C for 1 hour, the mixture was diluted with ethyl ether and the mixture was washed with water and brine. The organic layer was dried with magnesium sulfate and concentrated in vacuo. The residue was dissolved in methanol (100 ml) and hydrogenated at room temperature under hydrogen atmosphere under atmospheric pressure for 6 hours. The catalyst was filtered off and the solvent was removed under reduced pressure. The residue was dissolved in 4N hydrogen chloride in ethyl acetate (50 ml) at room temperature. After being stirred at the same temperature for 10 minutes, the mixture was concentrated in vacuo and the residue was triturated with ethyl ether to give methyl (3S)-3-amino-4-(2-naphthyl)butanoate hydrochloride (0.44 g).

NMR ($CDCl_3$-$CD_3OD$, δ) : 7.74-7.84 (3H, m), 7.72 (1H, s), 7.42-7.52 (2H, m), 7.34 (1H, d, J=8.0Hz), 3.90 (1H, m), 3.46 (1H, dd, J=15.0, 5.0Hz), 3.12 (1H, dd, J=15.0, 10.0Hz), 2.80 (2H, ABX)

Preparation 7

Methyl (3S)-4-(6-ethyl-2-naphthyl)-3-hydroxybutanoate (0.54 g) was dissolved in 15N ammonia in methanol (5 ml) at room temperature and the mixture was allowed to stand for six days. The solvent was evaporated in vacuo and the residue was triturated with isopropyl ether to give (3S)-4-(6-ethyl-2-naphthyl)-3-hydroxybutanamide (0.49 g).

NMR (DMSO-$d_6$, δ) : 7.76 (2H, dd, J=8.0, 4.0Hz), 7.64 (2H, s), 7.34 (2H, d, J=8.0Hz), 7.28 (1H, br s), 6.80 (1H, br s), 4.86 (1H, d, J=4.0Hz), 4.14 (1H, m), 2.80 (2H, d, J=7.5Hz), 2.74 (2H, q, J=7.5Hz), 2.16 (2H, d, J=7.5Hz), 1.26 (3H, t, J=7.5Hz)

The following compounds (Preparations 8 and 9) were obtained according to a similar manner to that of Preparation 7.

Preparation 8

(3S)-3-Amino-4-(2-naphthyl)butanamide

NMR ($CDCl_3$, δ) : 7.8 (4H, m), 7.64 (1H, s), 7.44 (3H, m), 7.30 (1H, d, J=10.0Hz), 3.5 (1H, m), 3.00 (1H, dd, J=12.0, 7.5Hz), 2.76 (1H, dd, J=12.0, 10.0Hz), 2.48 (1H, dd, J=15.0, 5.0Hz), 2.25 (1H, dd, J=15.0, 10.0Hz)
ESI-MS : 229 [M+H]

Preparation 9

(3S)-3-Hydroxy-4-(2-naphthyl)butanamide

NMR (DMSO-$d_6$, δ) : 7.78-7.90 (3H, m), 7.70 (1H, s), 7.34-7.52 (3H, m), 7.30 (1H, br s), 6.82 (1H, br s), 4.90 (1H, d, J=5.0Hz), 4.14 (1H, m), 2.74-2.90 (2H, m), 2.15 (2H, d, J=5.0Hz)

Preparation 10

A mixture of 2-acetyl-6-ethylnaphthalene (9.09 g) and morpholine (6 ml) and sulfur (2.2 g) was heated at 120°C for one hour and then refluxed for ten hours. The mixture was cooled to room temperature and diluted with ethyl acetate. The mixture was washed with 1N hydrochloric acid, aqueous sodium bicarbonate and brine, successively. The organic layer was dried over magnesium sulfate and concentrated in vacuo. The residue was purified by silica gel column chromatography (ethyl acetate : hexane = 1:1, as an eluent) to give 6-ethyl-2-naphthylacetothiomorpholide. The thiomorpholide thus obtained was dissolved in acetic acid (20 ml), concentrated sulfuric acid (3 ml) and water (4.5 ml) and the mixture was refluxed for five hours. The mixture was cooled to room temperature and poured into ethyl acetate and the mixture was washed with water and brine, successively. The organic layer was dried over magnesium sulfate and concentrated in vacuo. The residue was triturated with isopropyl ether to give 2-(6-ethylnaphthalen-2-yl) acetic acid (1.90 g).

NMR ($CDCl_3$, δ) : 7.74 (2H, t, J=7.5Hz), 7.70 (1H, s), 7.60 (1H, s), 7.36 (2H, t, J=7.5Hz), 3.80 (2H, s), 2.80 (2H, q, J=7.5Hz), 1.30 (3H, t, J=7.5Hz)
ESI-MS : 213 [M-H]

Preparation 11

To an ice-cooled suspension of sodium hydride (60% in oil dispersion, 6.75 g) in tetrahydrofuran (50 ml) was added 5-hydroxy-1-pentene (10.1 g) in tetrahydrofuran (50 ml). After stirring for 30 minutes, 1-bromononane (31.1 g) in tetrahydrofuran (100 ml) was added. This mixture was refluxed overnight, poured into saturated aqueous ammonium chloride (300 ml), and extracted with diethyl ether (300 ml). The organic phase was separated, washed with water (300 ml) and brine (200 ml), dried over magnesium sulfate, and evaporated to dryness. The residue was chromatographed on a silica gel (1000 cc), eluting with ethyl acetate in n-hexane (0-10%) to give 4-pentenyl nonyl ether (17.2 g).

NMR ($CDCl_3$, δ) : 5.83 (1H, m), 4.92-5.07 (2H, m), 3.35-3.45 (4H, m), 2.12 (2H, m), 1.48-1.73 (4H, m), 1.17-1.39 (12H, m), 0.87 (3H, t, J=7Hz)

Preparation 12

A solution of 4-pentenyl nonyl ether (7.0 g) in a mixture of methanol (150 ml) and dichloromethane (50 ml) was cooled to -78°C. Ozone was passed through this solution keeping temperature below -60°C until the color turned to be light blue. Then, methylsulfide (12.1 ml) was added dropwise, and this solution was warmed to room temperature over 3 hours. The resulting solution was concentrated and partitioned between diethyl ether (150 ml) and water (100 ml). The ethereal solution was dried over magnesium sulfate and evaporated to dryness to give a crude product of 1,1-dimethoxy-4-nonyloxybutane (7.76 g).

NMR ($CDCl_3$, δ) : 4.38 (1H, t, J=5Hz), 3.42 (2H, t, J=6Hz), 3.38 (2H, t, J=6Hz), 3.32 (6H, s), 1.37-1.73 (6H, m), 1.17-1.41 (12H, m), 0.88 (3H, t, J=7Hz)

Preparation 13

To an ice-cooled solution of 1,1-dimethoxy-4-nonyloxybutane (3.00 g) in acetone (150 ml) was added 2N Jones' reagent drop by drop. After stirring for 1 hour at 4°C, isopropyl alcohol was added until the orange color disappeared. This solution was neutralized with 1N aqueous sodium hydroxide, concentrated in vacuo, acidified with 1N hydrochloric acid, saturated with ammonium chloride, and extracted with ethyl acetate (50 ml). The organic phase was washed with brine, dried over magnesium sulfate, and evaporated to dryness to give 4-nonyloxybutyric acid (2.68 g).

NMR ($CDCl_3$, δ) : 3.35-3.52 (4H, m), 2.48 (2H, t, J=7Hz), 1.90 (2H, m), 1.47-1.66 (2H, m), 1.16-1.41 (12H, m), 0.88 (3H, t, J=7Hz)

Preparation 14

To a solution of 4-nonyloxybutyric acid (2.66 g) and a drop of dimethylformamide in dichloromethane (50 ml) was added oxalyl chloride (1.11 ml). This solution was stirred for 1 hour and concentrated under reduced pressure. The residue was dissolved in dichloromethane (10 ml), and added to a solution of Meldrum's acid (1.66 g) and pyridine (1.87 ml) in dichloromethane (25 ml) at 4°C. This solution was stirred at room temperature overnight. The resulting mixture was washed with 10% hydrochloric acid (50 ml x 3) and water, dried over magnesium sulfate, and concentrated in vacuo. The residue was dissolved in methanol, and refluxed for 3 hours. Then, the mixture was evaporated in dryness, and chromatographed on a silica gel (150 cc) eluting with 10% ethyl acetate in n-hexane to give methyl 6-nonyloxy-3-oxohexanoate (0.96 g).

NMR ($CDCl_3$, δ) : 3.74 (3H, s), 3.47 (2H, s), 3.41 (2H, t, J=6Hz), 3.36 (2H, t, J=6Hz), 2.63 (2H, t, J=7Hz), 1.86 (2H, m), 1.47-1.61 (2H, m), 1.18-1.40 (12H, m), 0.88 (3H, t, J=7Hz)

Preparation 15

Methyl (3S)-3-hydroxy-6-nonyloxyhexanoate was obtained according to a similar manner to that of Preparation 3.

NMR ($CDCl_3$, δ) : 4.04 (1H, m), 3.71 (3H, s), 3.49 (1H, d, J=3Hz), 3.45 (2H, t, J=6Hz), 3.41 (2H, t, J=7Hz), 2.41-2.53 (2H, m), 1.46-1.80 (6H, m), 1.17-1.38 (12H, m), 0.88 (3H, t, J=7Hz)

Preparation 16

(3S)-3-Hydroxy-6-nonyloxyhexanamide was obtained according to a similar manner to that of Preparation 7.

NMR ($CDCl_3$, δ) : 6.37 (1H, br s), 5.33 (1H, br s), 4.39 (1H, d, J=2Hz), 3.99 (1H, m), 3.40-3.53 (4H, m), 2.30-2.44 (2H, m), 1.49-1.82 (6H, m), 1.18-1.41 (12H, m), 0.87 (3H, t, J=7Hz)

Preparation 17

To an ice-cooled solution of methyl (3R)-3-hydroxyhexadecanoate (5.35 g) and triethylamine (5.21 ml) in dichloromethane (50 ml) was added methanesulfonyl chloride (2.17 ml). After stirring in an ice-water bath for 35 minutes, this solution was poured into a mixture of ethyl acetate (150 ml) and 1N hydrochloric acid (150 ml). The organic phase was separated and washed with 1N hydrochloric acid (100 ml), saturated aqueous sodium bicarbonate (100

ml), and brine (100 ml). Dryness over magnesium sulfate and evaporation gave methyl (3R)-3-methanesulfonyloxyhexadecanoate (6.77 g).

NMR ($CDCL_3$, δ) : 5.04 (1H, m), 3.72 (3H, s), 3.02 (3H, m), 2.78 (1H, dd, J=16, 8Hz), 2.65 (1H, dd, J=16, 5Hz), 1.77 (2H, m), 1.15-1.55 (22H, m), 0.88 (3H, t, J=7Hz)

Preparation 18

A solution of methyl (3R)-3-methanesulfonyloxyhexadecanoate (6.77 g) and sodium azide (2.33 g) in dimethylformamide (60 ml) was heated to 60°C for 40 minutes. This solution was poured into a mixture of ethyl acetate (300 ml) and water (500 ml). The organic phase was separated and washed with water (500 ml) and brine (300 ml). The resulting solution was dried over magnesium sulfate and evaporated to dryness to give methyl (3S)-3-azidohexadecanoate and some by-products. This crude product (5.0 g) was used in the next step without any further purification.

Preparation 19

Methyl (3S)-3-azidohexadecanoate (5.0 g) in methanol (25 ml) was hydrogenated over 10% palladium on carbon (0.50 g) under atmospheric pressure of hydrogen for 4 hours at room temperature. Then, the catalyst was filtered off with celite and the filtrate was concentrated under reduced pressure. The residue was dissolved with 4N hydrogen chloride in ethyl acetate (20 ml), evaporated, and triturated with diisopropyl ether (20 ml) to give methyl (3S)-3-aminohexadecanoate hydrochloride (930 mg).

NMR ($CDCl_3$, δ) : 3.75 (3H, s), 3.60 (1H, m), 2.74-2.93 (2H, m), 1.57-1.98 (4H, m), 1.14-1.51 (20H, m), 0.87 (3H, t, J=7Hz)

Preparation 20

To a suspension of methyl (3S)-3-aminohexadecanoate hydrochloride (890 mg) in water (1.8 ml) was added formalin (0.67 ml) and cyclopentadiene (1.14 ml) successively. The mixture was sonicated for 15 minutes, and stirred for 20 hours. The resulting mixture was washed with n-hexane, made basic with saturated sodium bicarbonate, and extracted with chloroform (x3). The combined organic phase was dried over magnesium sulfate, and concentrated under reduced pressure. To the residue in dichloromethane (12 ml) and trifluoroacetic acid (12 ml) was added triethylsilane (1.32 ml). This mixture was stirred overnight, and evaporated. This residue was dissolved in ethyl acetate (30 ml), washed with saturated aqueous sodium bicarbonate (20 ml), and dried over magnesium sulfate. After evaporation, the residue was purified on a silica gel (20 cc) to give methyl (3S)-3-(methylamino)hexadecanoate (686 mg).

NMR ($CDCl_3$, δ) : 3.69 (3H, s), 2.98 (1H, m), 2.89 (1H, br s), 2.50 (2H, m), 2.45 (3H, s), 1.18-1.63 (24H, m), 0.87 (3H, t, J=7Hz)

Preparation 21

Methyl 4-(3-benzo[b]furanyl)-3-oxobutanoate was obtained according to a similar manner to that of Preparation 1.

NMR ($CDCl_3$, δ) : 7.64 (1H, s), 7.43-7.57 (2H, m), 7.21-7.36 (2H, m), 3.92 (2H, s), 3.71 (3H, s), 3.52 (2H, s)

Preparation 22

Methyl (3S)-4-(3-benzo[b]furanyl)-3-hydroxybutanoate was obtained according to a similar manner to that of Preparation 3.

NMR ($CDCl_3$, δ) : 7.58 (1H, d, J=7Hz), 7.52 (1H, s), 7.47 (1H, d, J=7Hz), 7.19-7.34 (2H, m), 4.39 (1H, m), 3.68 (3H, s), 2.80-3.08 (3H, m), 2.42-2.65 (2H, m)

Preparation 23

To an ice-cooled solution of methyl (3S)-4-(3-benzo[b]furanyl)-3-hydroxybutanoate (250 mg) in methanol (2 ml) was added 1N aqueous sodium hydroxide (1.1 ml). This solution was stirred at room temperature overnight. Then

it was diluted with water (20 ml), washed with diethyl ether (10 ml), acidified with 1N hydrochloric acid (1.4 ml), extracted with ethyl acetate (10 ml x 3), and dried over magnesium sulfate. After evaporation, the residue was dissolved in dimethylformamide (2 ml). To this solution, HOBt (1-hydroxybenzotriazole) (136 mg), WSCD·HCl [1-ethyl-3-(3-dimethylaminopropyl)carbodiimide hydrochloride] (193 mg) was added successively. After 30 minutes, 28% ammonium hydroxide (91 µl) was added, and the mixture was stirred overnight. Then, the resulting mixture was diluted with 1N hydrochloric acid (20 ml) and extracted with ethyl acetate (20 ml). The organic phase was washed with 1N hydrochloric acid (20 ml), saturated aqueous sodium bicarbonate (20 ml) and brine. Dried over magnesium sulfate, evaporated to dryness, and chromatographed on a silica gel (20 cc) to give (3S)-4-(3-benzo[b]furanyl)-3-hydroxybutanamide (110 mg).

NMR (DMSO-$d_6$, δ) : 7.76 (1H, s), 7.65 (1H, d, J=7Hz), 7.53 (1H, d, J=8Hz), 7.19-7.40 (3H, m), 6.82 (1H, br s), 4.94 (1H, d, J=6Hz), 4.17 (1H, m), 2.79 (1H, dd, J=15, 5Hz), 2.70 (1H, dd, J=15, 7Hz), 2.11 (2H, d, J=7Hz)

Preparation 24

To a suspension of methyl triphenylphosphoranylideneacetate (2.45 g) in tetrahydrofuran (20 ml) was added 2-carboxybenzaldehyde (1.0 g) at 4°C. The resulting clear solution was stirred at room temperature for 30 minutes, and concentrated under reduced pressure. The residue was diluted with chloroform (20 ml) and extracted with saturated aqueous sodium bicarbonate (20 ml x 2). The combined aqueous phase was washed with diethyl ether (20 ml), acidified with 1N hydrochloric acid (pH 5~6), and extracted with ethyl acetate (20 ml x 2). The combined organic phase was washed with water (20 ml), brine (20 ml), dried over magnesium sulfate, and evaporated to dryness. The residue was triturated with diisopropyl ether to give methyl 2-carboxycinnamate (350 mg).

NMR ($CDCl_3$, δ) : 8.55 (1H, d, J=16Hz), 8.12 (1H, d, J=8Hz), 7.56-7.67 (2H, m), 7.49 (1H, m), 6.34 (1H, d, J=16Hz), 3.83 (3H, s)

Preparation 25

t-Butyl 2-carboxycinnamate was obtained according to a similar manner to that of Preparation 24.

NMR ($CDCl_3$, δ) : 8.47 (1H, d, J=16Hz), 8.10 (1H, d, J=8Hz), 7.54-7.67 (2H, m), 7.47 (1H, m), 6.27 (1H, d, J=16Hz), 1.55 (9H, s)

Preparation 26

A solution of methyl 2-carboxycinnamate (100 mg), palladium(II) acetate (5 mg) and potassium formate (108 mg) in dimethylformamide (1 ml) was stirred at 60°C under nitrogen flow. The mixture was diluted with saturated aqueous ammonium chloride (20 ml), and extracted with ethyl acetate (20 ml). The organic phase was washed with water (20 ml) and brine (20 ml), dried over magnesium sulfate, and evaporated to dryness. The residue was triturated with diisopropyl ether to give methyl 3-(2-carboxyphenyl)propionate (82 mg).

NMR ($CDCl_3$, δ) : 8.06 (1H, m), 7.49 (1H, m), 7.33 (3H, m), 3.34 (2H, t, J=8Hz), 2.72 (2H, t, J=8Hz)

Preparation 27

t-Butyl 3-(2-carboxyphenyl)propionate was obtained according to a similar manner to that of Preparation 26.

NMR ($CDCl_3$, δ) : 8.04 (1H, d, J=7Hz), 7.47 (1H, t, J=7Hz), 7.27 (2H, m), 3.29 (2H, t, J=7Hz), 2.53 (2H, t, J=7Hz), 1.42 (9H, s)

Preparation 28

3-(2-Carboxyphenyl)propionamide was obtained according to a similar manner to that of Preparation 7.

NMR (DMSO-$d_6$, δ) : 7.77 (1H, d, J=8Hz), 7.45 (1H, dd, J=7, 6Hz), 7.17-7.38 (3H, m), 6.74 (1H, br s), 3.11 (2H, t, J=8Hz), 2.37 (2H, t, J=8Hz)

Preparation 29

In a three-necked flask, under nitrogen flow, was placed magnesium turnings (1.26 g). In this flask, was added a solution of 1-bromohexane (8.59 g) in tetrahydrofuran (100 ml) dropwise. When the addition was completed, the whole was stirred for 30 minutes. The resulting mixture was added to an ice-cooled mixture of 4-bromobenzyl bromide (10.0 g) in tetrahydrofuran (100 ml) and 0.1M dilithium tetrachlorocuprate in tetrahydrofuran (10 ml). This mixture was stirred at 4°C for 1.5 hours and at room temperature overnight. Then, it was poured into a mixture of ice and 1N hydrochloric acid (300 ml), and extracted with diethyl ether (300 ml). The etheral solution was washed with water (300 ml), saturated aqueous sodium bicarbonate (150 ml), and brine, dried over magnesium sulfate, and concentrated under reduced pressure. The residue was purified on a silica gel (200 cc) eluting with n-hexane to give 1-bromo-4-heptylbenzene (7.61 g).

NMR ($CDCl_3$, δ) : 7.38 (2H, d, J=8Hz), 7.04 (2H, d, J=8Hz), 2.55 (2H, dd, J=7, 8Hz), 1.48-1.67 (2H, m), 1.17-1.38 (8H, m), 0.88 (3H, t, J=7Hz)

Preparation 30

1-Allyl-4-heptylbenzene was obtained according to a similar manner to that of Preparation 29.

NMR ($CDCl_3$, δ) : 7.12 (2H, d, J=8Hz), 7.08 (2H, d, J=8Hz), 5.97 (1H, m), 5.02-5.13 (2H, m), 3.36 (2H, d, J=7Hz), 2.58 (2H, t, J=8Hz), 1.60 (2H, m), 1.19-1.40 (8H, m), 0.88 (3H, t, J=7Hz)

Preparation 31

To a mixture of 1-allyl-4-heptylbenzene (2.4 g), acetone (40 ml) and water (40 ml) was added sodium metaperiodate (11.9 g) and potassium permanganate (70 mg). This mixture was stirred at room temperature overnight. Then, the mixture was filtered and the filtrate was concentrated under reduced pressure. The resulting aqueous solution was extracted with ethyl acetate (60 ml), and the organic phase was washed with water (60 ml) and brine (30 ml). This solution was dried over magnesium sulfate, and evaporated to dryness. The residue was purified on a silica gel (40 cc) eluting with 0%~5% methanol in chloroform to give 1-allyl-4-heptylbenzene (1.82 g) and 2-(4-heptylphenyl)acetic acid (300 mg).

NMR ($CDCl_3$, δ) : 7.11-7.23 (4H, m), 3.62 (2H, s), 2.57 (2H, dd, J=8, 7Hz), 1.59 (2H, m), 1.18-1.42 (8H, m), 0.87 (3H, t, J=7Hz)

Preparation 32

Methyl (3S)-4-(4-heptylphenyl)-3-hydroxybutanoate was obtained according to a similar manner to that of Preparation 3.

NMR ($CDCl_3$, δ) : 7.12 (4H, s), 4.25 (1H, m), 3.69 (3H, s), 2.70-2.88 (3H, m), 2.39-2.61 (4H, m), 1.59 (2H, m), 1.20-1.39 (8H, m), 0.87 (3H, t, J=7Hz)

Preparation 33

Methyl 4-(4-heptylphenyl)-3-oxobutanoate was obtained according to a similar manner to that of Preparation 1.

NMR ($CDCl_3$, δ) : 7.15 (2H, d, J=8Hz), 7.10 (2H, d, J=8Hz), 3.78 (2H, s), 3.70 (3H, s), 3.45 (2H, s), 2.58 (2H, m), 1.59 (2H, m), 1.20-1.40 (8H, m), 0.88 (3H, t, J=7Hz)

Preparation 34

(3S)-4-(4-Heptylphenyl)-3-hydroxybutanamide was obtained according to a similar manner to that of Preparation 7.

NMR ($CDCl_3$, δ) : 7.12 (4H, s), 5.88 (1H, br s), 5.41 (1H, br s), 4.23 (1H, m), 3.24 (1H, d, J=3Hz), 2.84 (1H, dd, J=14, 7Hz), 2.76 (1H, dd, J=14, 7Hz), 2.57 (2H, dd, J=8, 7Hz), 2.44 (1H, dd, J=15, 3Hz), 2.33 (1H, dd, J=15, 8Hz),

1.51-1.67 (2H, m), 1.18-1.40 (8H, m), 0.88 (3H, t, J=7Hz)

Example 1

(2S)-5-Benzyloxycarbonyl-2-(tert-butoxycarbonylamino)-pentanoic acid dicyclohexylammonium salt (1.00 g) was suspended with ethyl acetate and the mixture was washed with 0.5N sulfuric acid, water and brine, successively. The organic layer was dried over magnesium sulfate and concentrated in vacuo. The residue was dissolved in methylene chloride (10 ml) and to this was added DMAP (N,N-dimethylaminopyridine) (469 mg), PyBOP (benzotriazole-1-yloxy-tris-pyrrolidinophosphonium hexafluorophosphate) (1.07 g) and (3S)-3-hydroxy-4-(2-naphthyl)butanamide (430 mg) at room temperature. After being stirred overnight at the same temperature, the mixture was diluted with ethyl acetate and washed with 1N hydrochloric acid, aqueous ammonium chloride, aqueous sodium bicarbonate and brine, successively. The organic layer was dried over magnesium sulfate and concentrated in vacuo. The residue was purified by silica gel column chromatography (2% methanol in chloroform, as an eluent) to give (3S)-3-[(2S)-5-benzyloxycarbonyl-2-(tert-butoxycarbonylamino)pentanoyl]oxy-4-(2-naphthyl)butanamide (0.96 g).

NMR ($CDCl_3$, δ) : 7.80 (3H, m), 7.66 (1H, s), 7.44 (2H, m), 7.34 (9H, m), 5.86 (1H, br s), 5.54 (1H, m), 5.30 (1H, br s), 5.06 (2H, s), 5.00 (1H, d, J=10Hz), 4.18 (1H, m), 3.14 (2H, dd, J=8.0, 3.0Hz), 2.46 (2H, m), 2.22 (2H, m), 1.5-1.6 (4H, m), 1.42 (9H, s)

The following compounds (Examples 2 to 5) were obtained according to a similar manner to that of Example 1.

Example 2

(3S)-4-(3-Benzo[b]furanyl)-3-[(2S)-5-benzyloxycarbonyl-2-(tert-butoxycarbonylamino)pentanoyl]oxybutanamide

NMR ($CDCl_3$, δ) : 7.64 (1H, m), 7.53 (1H, s), 7.48 (1H, d, J=8Hz) 7.22-7.41 (7H, m), 5.82 (1H, br s), 5.64 (1H, m), 5.27 (1H, br s), 5.10 (2H, s), 4.97 (1H, m), 4.22 (1H, m), 3.10 (2H, d, J=6Hz), 2.50 (2H, d, J=7Hz), 2.35 (2H, m), 1.62-1.84 (4H, m), 1.44 (9H, s)

Example 3

(3S)-3-[(2S)-5-Benzyloxycarbonyl-2-(tert-butoxycarbonylamino)pentanoyl]oxy-4-(4-heptylphenyl)-butanamide

NMR ($CDCl_3$, δ) : 7.30-7.39 (5H, m), 7.09 (4H, s), 5.82 (1H, br s), 5.43 (1H, m), 5.26 (1H, br s), 5.10 (2H, s), 5.01 (2H, s), 4.22 (1H, m), 2.97 (1H, dd, J=14, 7Hz), 2.89 (1H, dd, J=14, 7Hz), 2.55 (2H, dd, J=8, 7Hz), 2.32-2.45 (4H, m), 1.50-1.72 (6H, m), 1.44 (9H, s), 1.21-1.36 (8H, m), 0.88 (3H, t, J=7Hz)

Example 4

(3S)-3-[(2S)-5-Benzyloxycarbonyl-2-tert-butoxycarbonylaminopentanoyl]oxy-6-nonyloxyhexanamide

NMR ($CDCl_3$, δ) : 7.31-7.42 (5H, m), 5.90 (1H, br s), 5.25-5.34 (2H, m), 5.11 (2H, s), 5.05 (1H, d, J=8Hz), 4.23 (1H, m), 3.40 (2H, t, J=6Hz), 3.37 (2H, t, J=7Hz), 2.47 (2H, d, J=6Hz), 2.41 (2H, m), 1.49-1.92 (6H, m), 1.44 (9H, s), 1.21-1.37 (12H, m), 0.88 (3H, t, J=7Hz)

Example 5

(3S)-3-[N-Methyl-{(2S)-5-benzyloxycarbonyl-2-(tert-butoxycarbonylamino)pentanoyl}amino]hexadecanamide

NMR ($CDCl_3$, δ) : 7.25-7.4 (5H, m), 6.22 (1H, br s), 5.40 (1H, br s), 5.25 (1H, d, J=8.0Hz), 5.10 (2H, s), 4.75 (1H, m), 4.52 (1H, m), 2.90 (3H, s), 2.3-2.5 (4H, m), 1.45-1.75 (6H, m), 1.43 (9H, s), 1.15-1.35 (22H, m), 0.88 (3H, t, J=7.5Hz)
ESI-MS : 618 [M+H]

Preparation 35

(3S)-3-[(2S)-5-Benzyloxycarbonyl-2-(tert-butoxycarbonylamino)pentanoyl]oxy-4-(2-naphthyl)butanamide (0.94 g) was dissolved in 4N hydrogen chloride in ethyl acetate (50 ml) at room temperature. After being stirred for 1 hour at the same temperature, the mixture was diluted with ethyl acetate and the resulting solid was collected by filtration to give (3S)-3-[(2S)-2-amino-5-benzyloxycarbonylpentanoyl]oxy-4-(2-naphthyl)butanamide hydrochloride (0.67 g).

NMR (DMSO-$d_6$, δ) : 8.60 (3H, br s), 7.88 (3H, m), 7.80 (1H, s), 7.3-7.55 (9H, m), 6.90 (1H, br s), 5.50 (1H, m), 5.10 (2H, s), 3.94 (1H, m), 3.10 (2H, m), 2.42 (2H, d, J=7.5Hz), 2.28 (2H, m), 1.72 (2H, m), 1.54 (2H, m)

The following compounds (Preparations 36 to 41) were obtained according to a similar manner to that of Preparation 35.

Preparation 36

(3S)-4-(3-Benzo[b]furanyl)-3-[(2S)-5-benzyloxycarbonyl-2-aminopentanoyl]oxybutanamide

NMR (DMSO-$d_6$, δ) : 8.52 (2H, br s), 7.88 (1H, s), 7.72 (1H, dd, J=6, 3Hz), 7.56 (1H, d, J=8Hz), 7.47 (1H, br s), 7.21-7.41 (7H, m), 6.91 (1H, br s), 5.48 (1H, m), 5.08 (2H, s), 3.98 (1H, m), 2.96-3.09 (2H, m), 2.45 (2H, d, J=6Hz), 2.36 (2H, t, J=7Hz), 1.47-1.89 (4H, m)

Preparation 37

(3S)-3-[(2S)-5-Benzyloxycarbonyl-2-aminopentanoyl]oxy-4-(4-heptylphenyl)butanamide hydrochloride

NMR (DMSO-$d_6$, δ) : 8.42 (2H, br s), 7.43 (1H, br s), 7.31-7.41 (5H, m), 7.11 (4H, s), 6.87 (1H, br s), 5.38 (1H, m), 5.07 (2H, s), 3.97 (1H, m), 2.87 (2H, m), 2.29-2.40 (4H, m), 1.42-1.83 (6H, m), 1.16-1.35 (8H, m), 0.85 (3H, m)

Preparation 38

(3S)-3-[(2S)-5-Benzyloxycarbonyl-2-aminopentanoyl]oxy-6-nonyloxyhexanamide hydrochloride

NMR (DMSO-$d_6$, δ) : 8.42 (2H, br s), 7.45 (1H, br s), 7.28-7.41 (5H, m), 6.87 (1H, br s), 5.23 (1H, m), 5.09 (2H, s), 3.99 (2H, s), 3.25-3.36 (4H, m), 2.31-2.44 (4H, m), 1.35-1.90 (10H, m), 1.10-1.32 (12H, m), 0.85 (3H, m)

Preparation 39

(3S)-3-[(2S)-2-Amino-5-benzyloxycarbonylpentanoyl]amino-4-(2-naphthyl)butanamide hydrochloride

NMR ($CDCl_3$, δ) : 8.66 (1H, br s), 8.46 (3H, br s), 7.56 (5H, br s), 7.1-7.4 (9H, m), 4.86 (2H, s), 4.42 (1H, m), 4.06 (1H, m), 3.22 (1H, m), 2.94 (1H, m), 2.76 (1H, m), 2.36 (1H, m), 1.16-2.0 (6H, m)

Preparation 40

(3S)-3-[N-Methyl-{(2S)-2-amino-5-benzyloxycarbonylpentanoyl}amino]hexadecanamide hydrochloride

NMR ($CDCl_3$, δ) : 8.3-8.5 (3H, m), 8.14 (1H, br s), 7.2-7.4 (5H, m), 7.14 (1H, br s), 5.08 (2H, s), 4.85 (1H, m), 4.14 (1H, m), 2.85 (3H, s), 1.4-2.8 (10H, m), 1.1-1.3 (22H, m), 0.88 (3H, t, J=7.5Hz)

Preparation 41

(3S)-3-[(2S)-5-Benzyloxycarbonyl-2-aminopentanoyl]aminohexadecanamide hydrochloride

NMR (DMSO-$d_6$, δ) : 8.25 (1H, d, J=8Hz), 8.12 (2H, m), 7.29-7.34 (6H, m), 6.80 (1H, br s), 5.08 (2H, s), 4.04 (1H, m), 3.80 (1H, m), 2.38 (2H, m), 2.21 (2H, d, J=7Hz), 1.51-1.86 (4H, m), 1.10-1.47 (24H, m), 0.85 (3H, t, J=7Hz)

Preparation 42

(2S)-5-Benzyloxycarbonyl-2-(tert-butoxycarbonylamino)pentanoic acid dicyclohexylammonium salt (1.07 g) was suspended with ethyl acetate and the mixture was washed with 0.5N sulfuric acid, water and brine, successively. The organic layer was dried over magnesium sulfate and concentrated in vacuo. The residue was dissolved in methylene chloride (10 ml) and to this was added DMAP (469 mg), PyBOP (1.05 g) and (3S)-4-(6-ethyl-2-naphthyl)-3-hydroxybutanamide (0.47 g) at room temperature. After being stirred overnight at the same temperature, the mixture was diluted with ethyl acetate and washed with 1N hydrochloric acid, aqueous ammonium chloride, aqueous sodium bicarbonate and brine, successively. The organic layer was dried over magnesium sulfate and concentrated in vacuo. The residue was dissolved in 4N hydrogen chloride in ethyl acetate (20 ml) at room temperature. After being stirred for 1 hour at the same temperature, the mixture was diluted with ethyl acetate and the resulting solid was collected by filtration to give (3S)-3-[(2S)-2-amino-5-benzyloxycarbonylpentanoyl]oxy-4-(6-ethyl-2-naphthyl)butanamide hydrochloride (0.70 g).

NMR (DMSO-$d_6$, δ) : 8.52 (3H, br s), 7.80 (2H, dd, J=8.0, 6.0Hz), 7.72 (1H, s), 7.66 (1H, s), 7.48 (1H, br s), 7.3-7.4 (7H, m), 6.90 (1H, br s), 5.50 (1H, m), 5.08 (2H, s), 3.94 (1H, m), 3.06 (2H, m), 2.74 (2H, q, J=7.5Hz), 2.40 (2H, d, J=7.5Hz), 2.28 (2H, m), 1.4-1.8 (4H, m), 1.24 (3H, t, J=7.5Hz)

Example 6

To a stirring solution of (3S)-3-[(2S)-2-amino-5-benzyloxycarbonylpentanoyl]oxy-4-(6-ethyl-2-naphthyl)butanamide hydrochloride (0.20 g), 1-benzylindole-3-carboxylic acid (105 mg) and HOBt (62 mg) in DMF (2 ml) was added WSCD (71 mg) at 0°C. After being stirred at room temperature overnight, the mixture was diluted with ethyl acetate and washed with 1N hydrochloric acid, aqueous ammonium chloride, aqueous sodium bicarbonate and brine, successively. The organic layer was dried over magnesium sulfate and concentrated in vacuo to give (3S)-3-[(2S)-2-(1-benzylindol-3-ylcarbonylamino)-5-benzyloxycarbonylpentanoyl]oxy-4-(6-ethyl-2-naphthyl)-butanamide (0.26 g).

NMR ($CDCl_3$, δ) : 8.20 (1H, s), 8.1 (2H, m), 7.1-7.8 (19H, m), 6.66 (1H, d, J=10Hz), 6.1 (1H, br s), 5.6 (1H, m), 5.34 (2H, s), 5.08 (2H, s), 4.7 (1H, m), 3.1 (2H, m), 2.74 (2H, q, J=7.5Hz), 2.1-2.6 (4H, m), 1.4-1.9 (4H, m), 1.28 (3H, t, J=7.5Hz)
ESI-MS : 724 [M+H]

The following compounds (Examples 7 to 20) were obtained according to a similar manner to that of Example 6.

Example 7

(3S)-3-[(2S)-2-(1-Benzylindol-2-ylcarbonylamino)-5-benzyloxycarbonylpentanoyl]oxy-4-(2-naphthyl)butanamide

NMR ($CDCl_3$, δ) : 7.72 (4H, m), 7.62 (1H, s), 7.1-7.45 (14H, m), 7.02 (3H, m), 6.92 (1H, d, J=7.5Hz), 5.82 (1H, br s), 5.78 (2H, ABq), 5.54 (1H, m), 5.20 (1H, br s), 5.10 (2H, s), 4.54 (1H, m), 3.08 (2H, m), 2.05-2.45 (4H, m), 1.70 (2H, m), 1.44 (2H, m)
ESI-MS : 696 [M+H]

Example 8

(3S)-3-[(2S)-2-(1-Benzylindol-3-ylcarbonylamino)-5-benzyloxycarbonylpentanoyl]oxy-4-(2-naphthyl)butanamide

NMR ($CDCl_3$, δ) : 8.06 (1H, m), 7.7 (4H, m), 7.62 (1H, s)., 7.2-7.4 (15H, m), 7.16 (1H, d, J=7.5Hz), 6.66 (1H, d, J=7.5Hz), 6.10 (1H, br s), 5.60 (1H, m), 5.34 (2H, s), 5.26 (1H, br s), 5.08 (2H, s), 4.66 (1H, m), 3.14 (2H, d, J=7.5Hz), 2.52 (2H, ABX), 2.1-2.3 (2H, m), 1.65-1.85 (2H, m), 1.45-1.6 (2H, m)
ESI-MS : 696 [M+H]

Example 9

(3S)-3-[(2S)-5-Benzyloxycarbonyl-2-(2-quinolylcarbonylamino)pentanoyl]oxy-4-(2-naphthyl)butanamide

NMR ($CDCl_3$, δ) : 8.70 (1H, d, J=10Hz), 7.3-8.5 (18H, m), 5.92 (1H, br s), 5.62 (1H, m), 5.32 (1H, br s), 5.10 (2H, s), 4.74 (1H, m), 3.16 (2H, d, J=7.5Hz), 2.52 (2H, m), 2.30 (2H, m), 1.6-2.0 (4H, m)
ESI-MS : 618 [M+H]

Example 10

(3S)-3-[(2S)-2-(3-Benzylnaphthalen-2-ylcarbonylamino)-5-benzyloxycarbonylpentanoyl]oxy-4-(2-naphthyl) butanamide

NMR ($CDCl_3$, δ) : 7.05-7.86 (23H, m), 6.34 (1H, d, J=8.0Hz), 5.80 (1H, br s), 5.58 (1H, m), 5.28 (1H, br s), 5.04 (2H, s), 4.50 (1H, m), 4.34 (2H, ABq), 3.16 (2H, d, J=7.5Hz), 2.48 (2H, m), 2.16 (2H, m), 1.44-1.70 (2H, m), 1.28-1.40 (2H, m)
ESI-MS : 707 [M+H]

Example 11

(3S)-4-(2-Naphthyl)-3-[(2S)-5-benzyloxycarbonyl-2-[2-(2-methoxycarbonylethyl)benzoylamino]pentanoyl] oxybutanamide

NMR ($CDCl_3$, δ) : 7.68-7.82 (3H, m), 7.65 (1H, br s), 7.21-7.46 (12H, m), 6.93 (1H, d, J=8Hz), 6.02 (1H, br s), 5.61 (1H, m), 5.31 (1H, br s), 5.08 (2H, s), 4.59 (1H, m), 3.59 (3H, s), 3.18 (2H, m), 3.05 (2H, m), 2.72 (2H, m), 2.54 (2H, d, J=7Hz), 2.16-2.34 (2H, m), 1.48-1.86 (4H, m)

Example 12

(3S)-4-(2-Naphthyl)-3-[(2S)-5-benzyloxycarbonyl-2-[2-(2-t-butoxycarbonylethyl)benzoylamino]pentanoyl]oxybutanamide

NMR ($CDCl_3$, δ) : 7.68-7.81 (3H, m), 7.65 (1H, br s), 7.10-7.48 (13H, m), 6.10 (1H, br s), 5.60 (1H, m), 5.28 (1H, br s), 5.06 (2H, s), 4.56 (1H, m), 3.18 (2H, m), 2.93-3.13 (2H, m), 2.50-2.69 (4H, m), 2.22 (2H, m), 1.50-1.84 (4H, m), 1.36 (9H, s)

Example 13

(3S)-4-(2-Naphthyl)-3-[(2S)-5-benzyloxycarbonyl-2-[2-(2-carbamoylethyl)benzoylamino]pentanoyl]oxybutanamide

NMR (DMSO-$d_6$, δ) : 8.85 (1H, d, J=7Hz), 7.79-7.88 (3H, m), 7.75 (1H, br s), 7.18-7.50 (12H, m), 6.68 (1H, br s), 6.82 (1H, br s), 5.42 (1H, m), 5.06 (2H, s), 4.31 (1H, m), 3.13 (1H, dd, J=14Hz, 5Hz), 3.02 (1H, dd, J=14, 6Hz), 2.92 (2H, dd, J=16, 8Hz), 2.33-2.46 (4H, m), 2.23 (2H, m), 1.44-1.73 (4H, m)

Example 14

(3S)-3-[(2S)-5-Benzyloxycarbonyl-2-(2-quinolylcarbonylamino)pentanoyl]oxy-4-(4-heptylphenyl)-butanamide

NMR ($CDCl_3$, δ) : 8.69 (1H, d, J=8Hz), 8.33 (1H, d, J=8Hz), 8.26 (1H, d, J=8Hz), 8.18 (1H, d, J=8Hz), 7.90 (1H, d, J=8Hz), 7.80 (1H, m), 7.75 (1H, m), 7.28-7.38 (5H, m), 7.11 (2H, d, J=8Hz), 7.02 (1H, d, J=8Hz), 5.85 (1H, br s), 5.49 (1H, m), 5.30 (1H, br s), 5.10 (2H, s), 4.76 (1H, m), 2.53-2.86 (2H, m), 2.35-2.53 (6H, m), 1.40-2.10 (6H, m), 1.15-1.34 (8H, m), 0.88 (3H, t, J=7Hz)

Example 15

(3S)-4-(3-Benzo[b]furanyl)-3-[(2S)-5-benzyloxycarbonyl-2-(2-quinolylcarbonylamino)pentanoyl]oxybutanamide

NMR ($CDCl_3$, δ) : 8.68 (1H, d, J=8Hz), 8.34 (1H, d, J=8Hz), 8.27 (1H, d, J=8Hz), 8.19 (1H, d, J=8Hz), 7.91 (1H, d, J=8Hz), 7.81 (1H, d, J=8Hz, 7Hz), 7.62-7.71 (2H, m), 7.54 (1H, s), 7.43 (1H, m), 7.23-7.37 (7H, m), 5.86 (1H,

br s), 5.58 (1H, m), 5.28 (1H, br s), 5.09 (2H, s), 4.75 (1H, m), 3.10 (2H, d, J=7Hz), 2.53 (2H, m), 2.38 (2H, m), 1.64-2.03 (4H, m)

Example 16

(3S)-3-[(2S)-5-Benzyloxycarbonyl-2-(2-quinolylcarbonylamino)pentanoyl]oxy-6-nonyloxyhexanamide

NMR ($CDCl_3$, δ) : 8.74 (1H, d, J=8Hz), 8.32 (1H, d, J=8Hz), 8.25 (1H, d, J=8Hz), 8.16 (1H, d, J=9Hz), 7.88 (1H, d, J=8Hz), 7.77 (1H, t, J=8Hz), 7.62 (1H, t, J=8Hz), 7.28-7.37 (5H, m), 5.93 (1H, br s), 5.36 (1H, m), 5.28 (1H, br s), 5.11 (2H, s), 4.78 (1H, m), 3.30-3.41 (4H, m), 2.41-2.53 (4H, m), 1.45-2.15 (10H, m), 1.15-1.33 (12H, m), 0.86 (3H, t, J=7Hz)

Example 17

(3S)-3-[(2S)-5-Benzyloxycarbonyl-2-(tert-butoxycarbonylamino)pentanoyl]amino-4-(2-naphthyl)butanamide

NMR ($CDCl_3$, δ) : 7.74-7.82 (3H, m), 7.64 (1H, s), 7.3-7.5 (8H, m), 7.14 (1H, d, J=10Hz), 5.76 (1H, br s), 5.56 (1H, br s), 5.08 (1H, d, J=8.0Hz), 5.06 (2H, s), 4.50 (1H, m), 4.00 (1H, m), 3.16 (1H, dd, J=12.0, 7.5Hz), 3.02 (1H, dd, J=12.0, 7.5Hz), 2.42 (2H, ABX), 2.26 (2H, m), 1.6-1.75 (2H, m), 1.45-1.6 (2H, m), 1.4 (9H, s)
ESI-MS : 562 [M+H]

Example 18

(3S)-3-[(2S)-2-(1-Benzylindol-3-ylcarbonylamino)-5-benzyloxycarbonylpentanoyl]amino-4-(2-naphthyl)butanamide

NMR (DMSO-$d_6$, δ) : 8.26 (1H, s), 8.20 (1H, d, J=8.0Hz), 8.02 (1H, d, J=8.0Hz), 7.86 (1H, d, J=8.0Hz), 7.64-7.74 (4H, m), 7.54 (1H, d, J=8.0Hz), 7.1-7.4 (16H, m), 6.84 (1H, br s), 5.46 (2H, s), 5.06 (2H, s), 4.44 (1H, m), 4.30 (1H, m), 2.82-3.0 (2H, m), 2.2-2.4 (4H, m), 1.4-1.8 (4H, m)
ESI-MS : 695 [M+H]

Example 19

(3S)-3-[N-Methyl-{(2S)-5-benzyloxycarbonyl-2-(2-quinolylcarbonylamino)pentanoyl}amino]hexadecanamide

NMR ($CDCl_3$, δ) : 8.90 (1H, d, J=8.0Hz), 8.1-8.35 (3H, m), 7.85 (1H, d, J=8.0Hz), 7.75 (1H, t, J=8.0Hz), 7.62 (1H, t, J=8.0Hz), 7.25-7.35 (5H, m), 6.32 (1H, br s), 5.52 (1H, br s), 5.16 (1H, m), 5.10 (2H, s), 4.82 (1H, m), 3.00 (3H, s), 2.4-2.55 (4H, m), 1.4-2.05 (6H, m), 1.05-1.4 (22H, m), 0.88 (3H, t, J=7.5Hz)
ESI-MS : 673 [M+H]

Example 20

(3S)-3-[(2S)-5-Benzyloxycarbonyl-2-(2-quinolylcarbonylamino)pentanoyl]aminohexadecanamide

NMR ($CDCl_3$, δ) : 8.71 (1H, d, J=8Hz), 8.32 (1H, d, J=8Hz), 8.25 (1H, d, J=8Hz), 8.15 (1H, d, J=9Hz), 7.88 (1H, d, J=8Hz), 7.78 (1H, t, J=8Hz), 7.63 (1H, t, J=8Hz), 7.26-7.36 (5H, m), 6.82 (1H, d, J=8Hz), 6.06 (1H, br s), 5.35 (1H, br s), 5.12 (2H, s), 4.62 (1H, m), 4.18 (1H, m), 2.37-2.54 (4H, m), 1.48-2.18 (6H, m), 1.02-1.36 (22H, m), 0.87 (3H, t, J=7Hz)

Example 21

To a stirring solution of (3S)-3-[(2S)-2-(1-benzylindol-3-ylcarbonylamino)-5-benzyloxycarbonylpentanoyl]oxy-4-(6-ethyl-2-naphthyl)butanamide (0.24 g) and anisole (717 mg) in methylene chloride (2.5 ml) was added aluminum chloride (442 mg) in nitromethane (2.5 ml) at room temperature. After being stirred for two hours at the same temperature, the mixture was diluted with ethyl acetate and washed with 1N hydrochloric acid, water and brine, successively. The organic layer was dried over magnesium sulfate and concentrated in vacuo. The resulting solid was triturated with ethyl ether to give (3S)-3-[(2S)-2-(1-benzylindol-3-ylcarbonylamino)-5-carboxypentanoyl]oxy-4-(6-ethyl-2-naphthyl) butanamide (113 mg).

NMR (DMSO-$d_6$, δ) : 8.28 (1H, s), 8.16 (2H, dd, J=8.0, 3.0Hz), 7.1-7.8 (14H, m), 6.86 (1H, br s), 5.50 (2H, s), 5.40 (1H, m), 4.40 (1H, m), 3.04 (2H, ABX), 2.74 (2H, q, J=7.5Hz), 2.34 (2H, d, J=7.5Hz), 2.18 (2H, t, J=7.5Hz), 1.5-1.8 (4H, m), 1.24 (3H, t, J=7.5Hz)

The following compounds (Examples 22 to 32) were obtained according to a similar manner to that of Example 21.

Example 22

(3S)-3-[(2S)-2-(1-Benzylindol-3-ylcarbonylamino) -5-carboxypentanoyl]oxy-4-(2-naphthyl)butanamide

NMR (DMSO-$d_6$, δ) : 8.28 (1H, s), 8.20 (2H, d, J=7.5Hz), 7.8 (3H, m), 7.54 (1H, d, J=7.5Hz), 7.1-7.6 (11H, m), 6.86 (1H, br s), 5.46 (2H, s), 5.40 (1H, m), 4.40 (1H, m), 3.05 (2H, ABX), 2.32 (2H, d, J=7.5Hz), 2.16 (2H, t, J=7.5Hz), 1.70 (2H, m), 1.55 (2H, m)
ESI-MS : 606 [M+H]

Example 23

(3S)-3-[(2S)-5-Carboxy-2-(2-quinolylcarbonylamino)-pentanoyl]oxy-4-(2-naphthyl)butanamide

NMR ($CDCl_3$, δ) : 8.72 (1H, d, J=10Hz), 7.15-8.3 (13H, m), 7.04 (1H, br s), 6.50 (1H, br s), 5.60 (1H, m), 4.76 (1H, m), 2.9-3.2 (2H, m), 2.44 (2H, m), 2.24 (2H, m), 1.35-2.1 (4H, m)
ESI-MS : 528 [M+H]

Example 24

(3S)-3-[(2S)-2-(3-Benzylnaphthalen-2-ylcarbonylamino)-5-carboxypentanoyl]oxy-4-(2-naphthyl)butanamide

NMR (DMSO-$d_6$, δ) : 8.84 (1H, d, J=8.0Hz), 7.06-7.98 (19H, m), 6.88 (1H, s), 5.42 (1H, m), 4.34 (1H, m), 4.28 (2H, ABq), 3.10 (2H, ABX), 2.36 (2H, d, J=7.5Hz), 2.10 (2H, t, J=7.5Hz), 1.4-1.75 (4H, m)
ESI-MS : 617 [M+H]

Example 25

(3S)-4-(2-Naphthyl)-3-[(2S)-5-carboxy-2-[2-(2-methoxycarbonylethyl)benzoylamino]pentanoyl]oxybutanamide

NMR (DMSO-$d_6$, δ) : 8.75 (1H, d, J=8Hz), 7.80-7.91 (3H, m), 7.78 (1H, br s), 7.22-7.50 (8H, m), 6.86 (1H, br s), 5.41 (1H, m), 4.32 (1H, m), 3.50 (3H, s), 2.90-3.18 (4H, m), 2.63 (2H, dd, J=8, 7Hz), 2.35 (2H, d, J=7Hz), 2.13 (2H, t, J=7Hz), 1.44-1.72 (4H, m)

Example 26

(3S)-4-(2-Naphthyl)-3-[(2S)-5-carboxy-2-[2-(2-carboxyethyl)benzoylamino]pentanoyl]oxybutanamide

NMR (DMSO-$d_6$, δ) : 8.74 (1H, d, J=7Hz), 7.80-7.92 (3H, m), 7.77 (1H, br s), 7.22-7.53 (8H, m), 6.87 (1H, br s), 5.42 (1H, m), 4.31 (1H, m), 3.15 (1H, dd, J=14, 6Hz), 2.86-3.08 (3H, m), 2.57 (2H, m), 2.37 (2H, d, J=7Hz), 2.13 (2H, t, J=7Hz), 1.42-1.72 (4H, m)

Example 27

(3S)-4-(2-Naphthyl)-3-[(2S)-5-carboxy-2-[2-(2-carbamoylethyl)benzoylamino]pentanoyl]oxybutanamide

NMR (DMSO-$d_6$, δ) : 8.86 (1H, d, J=7Hz), 7.80-7.92 (3H, m), 7.75 (1H, br s), 7.15-7.53 (10H, m), 6.88 (1H, br s), 6.73 (1H, br s), 5.42 (1H, m), 4.32 (1H, m), 2.82-3.18 (4H, m), 2.35-2.47 (4H, m), 2.11 (2H, t, J=7Hz), 1.42-1.77 (4H, m)

Example 28

(3S)-3-[(2S)-5-Carboxy-2-(2-quinolylcarbonylamino)-pentanoyl]oxy-4-(4-heptylphenyl)butanamide

NMR (DMSO-$d_6$, δ) : 8.95 (1H, d, J=8Hz), 8.62 (1H, d, J=8Hz), 8.20 (1H, d, J=9Hz), 8.17 (1H, d, J=8Hz), 8.12 (1H, d, J=8Hz), 7.90 (1H, t, J=8Hz), 7.74 (1H, t, J=8Hz), 7.37 (1H, br s), 7.08 (1H, d, J=8Hz), 6.92 (1H, d, J=8Hz), 6.86 (1H, br s), 5.33 (1H, m), 4.51 (1H, m), 2.87 (1H, dd, J=14, 6Hz), 2.78 (1H, dd, J=14, 7Hz), 2.19-2.40 (4H, m), 1.84 (2H, m), 1.53 (2H, m), 1.36 (2H, m), 1.10-1.30 (8H, m), 0.83 (3H, t, J=7Hz)

Example 29

(3S)-3-[(2S)-5-Carboxy-2-(2-quinolylcarbonylamino)-pentanoyl]oxy-6-nonyloxyhexanamide

NMR ($CDCl_3$, δ) : 8.84 (1H, d, J=8Hz), 8.32 (1H, d, J=8Hz), 8.26 (1H, d, J=8Hz), 8.17 (1H, d, J=8Hz), 7.88 (1H, d, J=8Hz), 7.78 (1H, m), 7.63 (1H, m), 7.13 (1H, br s), 6.25 (1H, br s), 5.43 (1H, m), 4.83 (1H, m), 3.31-3.46 (4H, m), 2.34-2.77 (4H, m), 1.90-2.20 (2H, m), 1.40-1.88 (10H, m), 1.14-1.37 (12H, m), 0.86 (3H, t, J=7Hz)

Example 30

(3S)-3-[(2S)-2-(1-Benzylindol-3-ylcarbonylamino)-5-carboxypentanoyl]amino-4-(2-naphthyl)butanamide

NMR (DMSO-$d_6$, δ) : 8.24 (1H, s), 8.20 (1H, d, J=8.0Hz), 8.04 (1H, d, J=8.0Hz), 7.84 (1H, d, J=8.0Hz), 7.64-7.74 (4H, m), 7.54 (1H, d, J=8.0Hz), 7.1-7.4 (11H, m), 6.84 (1H, br s), 5.44 (2H, s), 4.42 (1H, m), 4.30 (1H, m), 2.8-3.0 (2H, m), 2.26 (2H, d, J=7.5Hz), 2.16 (2H, t, J=7.5Hz), 1.4-1.75 (4H, m)
ESI-MS : 605 [M+H]

Example 31

(3S)-3-[N-Methyl-{(2S)-5-carboxy-2-(2-quinolylcarbonylamino)pentanoyl}amino]hexadecanamide

NMR ($CDCl_3$, δ) : 9.00 (1H, d, J=8.0Hz), 8.26 (2H, ABq), 8.16 (1H, d, J=8.0Hz), 7.86 (1H, d, J=8.0Hz), 7.75 (1H, t, J=8.0Hz), 7.60 (1H, t, J=8.0Hz), 7.10 (1H, br s), 6.80 (1H, br s), 5.20 (1H, m), 5.10 (1H, m), 3.05 (3H, s), 2.58 (1H, dd, J=15.0, 5Hz), 2.34-2.50 (3H, m), 1.4-2.1 (6H, m), 1.0-1.35 (22H, m), 0.86 (3H, t, J=7.5Hz)
ESI-MS : 583 [M+H]

Example 32

(3S)-3-[(2S)-5-Carboxy-2-(2-quinolylcarbonylamino)-pentanoyl]aminohexadecanamide

NMR (DMSO-$d_6$, δ) : 8.75 (1H, d, J=9Hz), 8.09 (1H, d, J=9Hz), 8.17 (2H, d, J=8Hz), 8.10 (1H, d, J=8Hz), 8.07 (1H, d, J=9Hz), 7.88 (1H, t, J=8Hz), 7.73 (1H, t, J=8Hz), 7.27 (1H, br s), 6.79 (1H, br s), 4.55 (1H, m), 4.06 (1H, m), 2.12-2.30 (4H, m), 1.32-1.91 (6H, m), 0.95-1.30 (18H, m), 0.83 (3H, t, J=7Hz)

Example 33

A solution of (3S)-3-[(2S)-2-(1-benzylindol-2-ylcarbonylamino)-5-benzyloxycarbonylpentanoyl]oxy-4-(2-naphthyl)butanamide (0.22 g) in water (0.4 ml) and methanol (4 ml) was hydrogenated over 10% palladium on carbon (40 mg) under atmospheric pressure of hydrogen for two days at room temperature. Then the catalyst was filtered off with celite and filtrate was concentrated under reduced pressure. The residue was triturated with ether and chloroform to give (3S)-3-[(2S)-2-(1-benzylindol-2-ylcarbonylamino)-5-carboxypentanoyl]oxy-4-(2-naphthyl)butanamide (168 mg).

NMR (DMSO-$d_6$, δ) : 8.92 (1H, d, J=8.0Hz), 7.05-7.85 (18H, m), 6.86 (1H, br s), 5.80 (2H, ABq), 5.40 (1H, m), 4.32 (1H, m), 3.02 (2H, ABX), 2.50 (2H, s), 2.32 (2H, d, J=7.5Hz), 2.15 (2H, t, J=7.5Hz), 1.65-1.8 (2H, m), 1.4-1.65 (2H, m)
ESI-MS : 606 [M+H]

Example 34

A mixture of (3S)-4-(3-benzo[b]furanyl)-3-[(2S)-5-benzyloxycarbonyl-2-(2-quinolylcarbonylamino)pentanoyl]-oxybutanamide (184 mg), cyclohexene (0.31 ml), and 10% palladium on carbon (130 mg) in dioxane (1 ml) was refluxed for 2.5 hours. After filtration with celite, and filtrate was partitioned between ethyl acetate (20 ml) and water (20 ml). The organic phase was washed with brine, dried over magnesium sulfate, and concentrated in vacuo. The residue was triturated in diethyl ether (5 ml) to give (3S)-4-(3-benzo[b]furanyl)-3-[(2S)-5-carboxy-2-(2-quinolylcarbonylamino) pentanoyl]oxybutanamide (107 mg).

NMR (DMSO-$d_6$, δ) : 9.02 (1H, d, J=8Hz), 8.61 (1H, d, J=8Hz), 8.21 (1H, d, J=8Hz), 8.17 (1H, d, J=8Hz), 8.12 (1H, d, J=8Hz), 7.91 (1H, t, J=8Hz), 7.70-7.79 (2H, m), 7.52 (1H, dd, J=7, 2Hz), 7.41 (1H, br s), 7.12-7.34 (2H, m), 6.89 (1H, br s), 5.45 (1H, m), 4.53 (1H, m), 3.03 (2H, m), 2.42 (2H, m), 2.23 (2H, t, J=7Hz), 1.86 (2H, m), 1.55 (2H, m)

Example 35

A solution of (3S)-4-(2-naphthyl)-3-[(2S)-5-benzyloxycarbonyl-2-{2-(2-t-butoxycarbonylethyl)-benzoylamino} pentanoyl]oxybutanamide (160 mg) in 4N hydrogen chloride in ethyl acetate (1 ml) was stirred at room temperature for four hours. Then, the mixture was concentrated in vacuo, dissolved in ethyl acetate (20 ml), washed with water (20 ml x 2) and brine (20 ml), and dried over magnesium sulfate. After evaporation, the residue was triturated in diisopropyl ether to give (3S)-4-(2-naphthyl)-3-[(2S)-5-benzyloxycarbonyl-2-{2-(2-carboxyethyl)-benzoylamino}pentanoyl]oxybutanamide (91 mg).

NMR ($CDCl_3$, δ) : 7.66-7.80 (3H, m), 7.60 (1H, br s), 7.17-7.46 (12H, m), 7.04 (1H, d, J=8Hz), 6.33 (1H, br s), 6.21 (1H, br s), 5.60 (1H, m), 5.05 (2H, s), 4.62 (1H, m), 2.92-3.20 (4H, m), 2.50-2.69 (4H, m), 2.19-2.32 (2H, m), 1.45-1.81 (4H, m)

Preparation 43

(3R)-3-Hydroxyhexadecanamide was obtained according to a similar manner to that of Preparation 23.

NMR (DMSO-d6,δ) : 7.26 (1H, br s), 6.88 (1H, br s), 4.58 (1H, d, J=5Hz), 3.75 (1H, m), 2.11 (2H, d, J=6 Hz), 1.14-1.40 (24H, m), 0.84 (3H, t, J=7Hz)

Preparation 44

To a solution of (3R)-3-hydroxyhexadecanamide (6.4 g) and triethylamine (6.57 ml) in dichloromethane (130 ml) and dimethylsulfoxide (130 ml) was added methanesulfonyl chloride (2.74 ml). This mixture was stirred at room temperature for 6 hours, and the resulting precipitate was filtered off. The liquor was concentrated, and dissolved in ethyl acetate (500 ml). This solution was washed with 1N-hydrochloric acid (800 ml x 2), saturated sodium carbonate (500 ml), and brine (200 ml). Drying over magnesium sulfate and concentration, the residue was chromatographed on a silica gel, eluting with a mixture of dichloromethane and methanol (50 : 1) to give (3R)-3-(methanesulfonyloxy)hexadecanamide (4.16 g).

NMR ($CDCl_3$, δ) : 5.68 (1H, br s), 5.45 (1H, br s), 5.04 (1H, m), 3.04 (3H, s), 2.62 (2H, d, J=7Hz), 1.81 (2H, m), 1.35-1.50 (22H, m), 0.87 (3H, t, J=7Hz)

Preparation 45

A solution of (3R)-3-(methanesulfonyloxy)hexadecanamide (4.16 g) and sodium azide (1.55 g) in dimethylformamide (50 ml) was heated at 60°C for 2 hours. The cooled mixture was partitioned between ethyl acetate (300 ml) and water (500 ml). The organic phase was washed with water (300 ml), and brine (200 ml). Dried, concentrated under vacuum, the residue was purified on a silica gel (200 cc) to give (3S)-3-azidohexadecanamide (2.20 g).

NMR ($CDCl_3$, δ) : 5.62 (1H, br s), 5.40 (1H, br s), 3.85 (1H, m), 2.42 (1H, dd, J=15Hz, 6Hz), 2.34 (1H, dd, J=15Hz, 8Hz), 1.18-1.64 (24H, m), 0.88 (3H, t, J=7 Hz)

Preparation 46

A mixture of (3S)-3-azidohexadecanamide (2.18 g) and 10 % palladium on carbon (320 mg) was hydrogenated at atmospheric pressure for 10 hours. The catalyst was filtered off with celite, and the liquor was concentrated under reduced pressure. The residue was triturated in diisopropyl ether (30 ml) to give (3S)-3-aminohexadecanamide.

NMR (DMSO-d6,δ) : 7.36 (1H, br s), 6.72 (1H, br s), 2.89 (1H, m), 2.08 (1H, dd, J=14Hz, 5Hz), 1.94 (1H, dd, J=14Hz, 8Hz), 1.13-1.52 (24H, m), 0.85 (3H, t, J=7Hz)

(3S)-3-aminohexadecanamide thus obtained was dissolved in 4N hydrogen chloride in ethyl acetate (15 ml), and concentrated under reduced pressure. The residue was triturated in diisopropyl ether to give (3S)-3-aminohexadecanamide hydrochloride (1.55 g).

Preparation 47

To an ice-cooled solution of (3S)-3-aminohexadecanamide (300 mg) and propionaldehyde (71 μl) in methanol (6 ml) was added sodium cyanoborohydride (61 mg). After 2 hours, propionaldehyde (71 μl) and sodium cyanoborohydride (61 mg) was added. The solution was stirred overnight. Then, the solvent was evaporated, diluted with water (20 ml), and extracted with chloroform (20 ml). The organic phase was washed with brine (20 ml), dried over magnesium sulfate, and evaporated to dryness. The residue was purified on a silica gel (20 cc) eluting with 1~10 % methanol in chloroform to give (3S)-3-(propylamino)hexadecanamide (240 mg).

NMR ($CDCl_3$, δ) : 7.52 (1H, br s), 5.61 (1H, br s), 3.05 (1H, m), 2.34-2.88 (5H, m), 1.49-1.73 (4H, m), 1.16 -1.45 (22H, m), 0.99 (3H, t, J=7Hz), 0.88 (3H, m)

Example 36

(3S)-3-[N-Propyl-{(2S)-5-benzyloxycarbonyl-2-(tert-butoxycarbonylamino)pentanoyl}amino]hexadecanamide was obtained according to a similar manner to that of Example 1.

Preparation 48

(3S)-3-[N-Propyl-{(2S)-2-amino-5-benzyloxycarbonylpentanoyl}amino]hexadecanamide was obtained according to a similar manner to that of Preparation 35.

Example 37

(3S)-3-[N-Propyl-{(2S)-5-benzyloxycarbonyl-2-(2-quinolylcarbonylamino)pentanoyl}amino]hexadecanamide was obtained according to a similar manner to that of Example 6.

Example 38

(3S)-3-[N-Propyl-{(2S)-5-carboxy-2-(2-quinolylcarbonylamino)pentanoyl}amino]hexadecanamide was obtained according to a similar manner to that of Example 21.

NMR ($CDCl_3$, δ) : 8.83 (1H, d, J=9Hz), 8.12-8.34 (3H, m), 7.86 (1H, d, J=8Hz), 7.76 (1H, t, J=7Hz), 7.61 (1H, t, J=7Hz), 6.52 (2H, br s), 5.10 (2H, m), 3.08-3.50 (2H, m), 2.33-2.72 (4H, m), 1.47-2.16 (8H, m), 1.05-1.40 (22H, m), 1.00 (3H, t, J=7Hz), 0.87 (3H, t, J=7Hz)

The following compounds (Preparations 49 and 50) were obtained according to a similar manner to that of Preparation 1.

Preparation 49

Methyl 4-(4-biphenylyl)-3-oxobutanoate
mp : 80-82°C

Preparation 50

Methyl 3-oxo-10-phenyldecanoate

NMR ($CDCl_3$, δ) : 7.1-7.25 (5H, m), 3.74 (3H, s), 3.44 (2H, s), 2.5-2.7 (4H, m), 1.5-1.7 (4H, m), 1.2-1.4 (6H, m)
FAB-MS : 277 [M+H]

The following compounds (Preparations 51 to 60) were obtained according to a similar manner to that of Preparation 3.

Preparation 51

Methyl (3R)-4-(4-n-heptyl)phenyl-3-hydroxybutanoate

NMR ($CDCl_3$, δ) : 7.12 (4H, s), 4.25 (1H, m), 3.70 (3H, s), 2.7-2.9 (3H, m), 2.4-2.6 (4H, m), 1.55-1.65 (2H, m), 1.2-1.4 (8H, m), 0.88 (3H, t, J=7Hz)

Preparation 52

Methyl (3R)-4-(4-n-butyl)phenyl-3-hydroxybutanoate

NMR ($CDCl_3$, δ) : 7.1 (4H, s), 4.24 (1H, m), 3.70 (3H, s), 2.7-2.85 (3H, m), 2.4-2.6 (4H, m), 1.5-1.65 (2H, m), 1.25-1.45 (2H, m), 0.90 (3H, t, J=7Hz)

Preparation 53

Methyl (3R)-4-(4-methylphenyl)-3-hydroxybutanoate

NMR ($CDCl_3$, δ) : 7.1 (4H, s), 4.22 (1H, m), 3.68 (3H, s), 2.76 (2H, ABX), 2.48 (2H, ABX), 2.32 (3H, s)

Preparation 54

Methyl (3S)-3-hydroxy-5-(2-naphthyl)pentanoate

NMR ($CDCl_3$, δ) : 7.74-7.83 (3H, m), 7.64 (1H, s), 7.38-7.50 (2H, m), 7.35 (1H, d, J=8Hz), 4.06 (1H, m), 3.71 (3H, s), 2.82-3.06 (3H, m), 2.42-2.60 (2H, m), 1.77-2.03 (2H, m)

Preparation 55

Methyl (3S)-5-(n-decyloxy)-3-hydroxypentanoate

NMR ($CDCl_3$, δ) : 4.24 (1H, m), 3.71 (3H, s), 3.56-3.68 (2H, m), 3.53 (1H, d, J=3Hz), 3.42 (2H, t, J=7Hz), 2.48-2.54 (2H, m), 1.69-1.86 (2H, m), 1.50-1.61 (2H, m), 1.18-1.38 (14H, m), 0.87 (3H, t, J=7Hz)

Preparation 56

Methyl (3S)-4-(4-biphenylyl)-3-hydroxybutanoate

Preparation 57

Methyl (3R)-3-hydroxydodecanoate

NMR ($CDCl_3$, δ) : 4.0 (1H, m), 3.8 (3H, s), 2.82 (1H, d, J=3Hz), 2.44 (2H, ABX), 1.2-1.55 (14H, m), 0.88 (3H, t, J=7Hz)

Preparation 58

Methyl (3R)-3-hydroxynonanoate

NMR ($CDCl_3$, δ) : 4.0 (1H, m), 3.72 (3H, s), 2.85 (1H, d, J=2Hz), 2.45 (2H, ABX), 1.2-1.6 (10H, m), 0.86 (3H, t, J=7Hz)

Preparation 59

Methyl (3R)-3-hydroxyheptanoate

NMR ($CDCl_3$, δ) : 4.0 (1H, m), 3.7 (3H, s), 2.82 (1H, d, J=3Hz), 2.44 (2H, ABX), 1.2-1.55 (6H, m), 0.88 (3H, t, J=7Hz)

Preparation 60

Methyl (3R)-4-(6-ethyl-2-naphthyl)-3-hydroxybutanoate

NMR ($CDCl_3$, δ) : 7.74-7.84 (3H, m), 7.66 (1H, s), 7.4-7.5 (2H, m), 7.34 (1H, d, J=8Hz), 4.36 (1H, m), 3.7 (2H, ABX), 2.87 (1H, d, J=5Hz), 2.52 (2H, ABX)
ESI-MS : 245 [M+H]

The following compounds (Preparations 61 to 72) were obtained according to a similar manner to that of Preparation 7.

Preparation 61

(3R)-4-(4-n-Heptyl)phenyl-3-hydroxybutanamide

NMR ($CDCl_3$, δ) : 7.12 (4H, s), 5.88 (1H, br s), 5.46 (1H, br s), 4.22 (1H, m), 3.26 (1H, d, J=3Hz), 2.7-2.9 (2H, m), 2.54-2.62 (2H, m), 2.38 (2H, ABX), 2.54-2.66 (2H, m), 1.2-1.4 (8H, m), 0.88 (3H, t, J=7Hz)

Preparation 62

(3R)-4-(4-n-Butyl)phenyl-3-hydroxybutanamide

NMR (DMSO-$d_6$, δ) : 7.26 (1H, br s), 7.06 (4H, s), 6.78 (1H, br s), 4.76 (1H, d, J=5Hz), 4.02 (1H, m), 2.60 (2H, d, J=7Hz), 2.5 (2H, m), 2.10 (2H, d, J=7Hz), 1.45-1.6 (2H, m), 1.2-1.35 (2H, m), 0.88 (3H, t, J=7Hz)

Preparation 63

(3R)-4-(4-Methylphenyl)-3-hydroxybutanamide

NMR (DMSO-$d_6$, δ) : 7.26 (1H, br s), 7.06 (4H, s), 6.78 (1H, br s), 4.76 (1H, d, J=5Hz), 4.00 (1H, m), 2.60 (2H, d, J=7Hz), 2.24 (3H, s), 2.10 (2H, d, J=7Hz)

Preparation 64

(3R)-3-Hydroxy-4-(2-naphthyl)butanamide

NMR (DMSO-$d_6$, δ) : 7.78-7.90 (3H, m), 7.70 (1H, s), 7.34-7.52 (3H, m), 7.30 (1H, br s), 6.82 (1H, br s), 4.90 (1H, d, J=5.0Hz), 4.14 (1H, m), 2.74-2.90 (2H, m), 2.15 (2H, d, J=5.0Hz)

Preparation 65

(3S)-3-Hydroxy-5-(2-naphthyl)pentanamide

NMR (DMSO-$d_6$, δ) : 7.80-7.90 (3H, m), 7.69 (1H, s), 7.36-7.52 (3H, m), 7.28 (1H, s), 6.80 (1H, br s), 4.79 (1H, d, J=5Hz), 3.85 (1H, m), 2.68-2.96 (2H, m), 2.21 (2H, d, J=6Hz), 1.60-1.84 (2H, m)

Preparation 66

(3S)-5-(n-Decyloxy)-3-hydroxypentanamide

Rf = 0.16 (2% methanol in chloroform)

Preparation 67

(3S)-3-Hydroxy-10-phenyldecanamide

NMR ($CDCl_3$, δ) : 7.1-7.35 (5H, m), 5.80 (1H, br s), 5.50 (1H, br s), 3.98 (1H, m), 3.30 (1H, d, J=3Hz), 2.5-2.65 (2H, m), 2.2-2.45 (2H, m), 1.2-1.7 (10H, m)
FAB-MS : 264 [M+H]

Preparation 68

(3S)-4-(4-Biphenylyl)-3-hydroxybutanamide

NMR (DMSO-$d_6$, δ) : 7.25-7.7 (10H, m), 6.85 (1H, br s), 4.86 (1H, d, J=7.5Hz), 4.10 (1H, m), 2.52 (2H, s), 2.15 (2H, d, J=8.0Hz)
FAB-MS : 256 [M+H]

Preparation 69

(3R)-3-Hydroxydodecanamide

NMR ($CDCl_3$, δ) : 5.85 (1H, br s), 5.52 (1H, br s), 4.0 (1H, m), 3.3 (1H, d, J=3Hz), 2.4 (2H, ABX), 1.2-1.6 (16H, m), 0.90 (3H, t, J=7Hz)

Preparation 70

(3R)-3-Hydroxynonanamide

NMR ($CDCl_3$, δ) : 5.85 (1H, br s), 5.52 (1H, br s), 4.0 (1H, m), 3.3 (1H, br s), 2.38 (2H, ABX), 1.2-1.7 (10H, m), 0.88 (3H, t, J=7Hz)

Preparation 71

(3R)-3-Hydroxyheptanamide

NMR ($CDCl_3$, δ) : 5.85 (1H, br s), 5.6 (1H, br s), 4.0 (1H, m), 3.32 (1H, br s), 2.38 (2H, ABX), 1.2-1.8 (8H, m), 0.88 (3H, t, J=7Hz)

Preparation 72

(3R)-4-(6-Ethyl-2-naphthyl)-3-hydroxybutanamide

NMR (DMSO-$d_6$, δ) : 7.76 (2H, dd, J=8, 4Hz), 7.64 (2H, s), 7.34 (2H, d, J=8Hz), 7.28 (2H, br s), 6.80 (1H, br s), 4.86 (1H, d, J=4Hz), 4.14 (1H, m), 2.8 (2H, d, J=7.5Hz), 2.74 (2H, q, J=7.5Hz), 2.16 (2H, d, J=7.5Hz), 1.26 (3H, t, J=7.5Hz)

Preparation 73

[4-(n-Butyl)phenyl]acetic acid was obtained according to a similar manner to that of Preparation 10.

NMR ($CDCl_3$, δ) : 7.10-7.22 (4H, m), 3.60 (2H, s), 2.58 (2H, dd, J=8, 7Hz), 1.41-1.64 (2H, m), 1.27-1.42 (2H, m), 0.92 (3H, t, J=7Hz)

The following compounds (Preparations 74 to 77) were obtained according to a similar manner to that of Preparation 14.

Preparation 74

Methyl 4-(4-n-butyl)phenyl-3-oxobutanoate

NMR ($CDCl_3$, δ) : 7.05-7.15 (4H, m), 3.76 (2H, s), 3.70 (3H, s), 3.42 (2H, s), 2.58 (2H, t, J=7Hz), 1.5-1.65 (2H, m), 1.25-1.4 (2H, m), 0.92 (3H, t, J=7Hz)

Preparation 75

Methyl 4-(4-methylphenyl)-3-oxobutanoate

NMR ($CDCl_3$, δ) : 7.0-7.15 (4H, m), 3.74 (2H, s), 3.68 (3H, s), 3.42 (2H, s), 2.3 (3H, s)

Preparation 76

Methyl 5-(2-naphthyl)-3-oxopentanoate

NMR ($CDCl_3$, δ) : 7.74-7.83 (3H, m), 7.63 (1H, br s), 7.38-7.49 (2H, m), 7.32 (1H, d, J=8Hz), 3.72 (3H, s), 3.46 (2H, s), 3.09 (2H, t, J=7Hz), 2.97 (2H, t, J=7Hz)

Preparation 77

Methyl 5-(n-decyloxy)-3-oxopentanoate

NMR ($CDCl_3$, δ) : 3.74 (3H, s), 3.67 (2H, t, J=7Hz), 3.51 (2H, s), 3.39 (2H, t, J=7Hz), 2.78 (2H, t, J=7Hz), 1.46-1.58 (2H, m), 1.18-1.36 (14H, m), 0.87 (3H, t, J=7Hz)

The following compounds (Preparations 78 to 85) were obtained according to a similar manner to that of Preparation 17.

Preparation 78

(3R)-4-(4-n-Heptyl)phenyl-3-methanesulfonyloxybutanamide

NMR ($CDCl_3$, δ) : 7.05-7.2 (4H, m), 5.6 (1H, br s), 5.4 (1H, br s), 5.12 (1H, m), 3.08 (2H, d, J=7Hz), 2.70 (3H, s), 2.52-2.62 (4H, m), 1.5-1.65 (2H, m), 1.2-1.35 (8H, m), 0.88 (3H, t, J=7Hz)

Preparation 79

(3R)-4-(4-n-Butyl)phenyl-3-methanesulfonyloxybutanamide

NMR ($CDCl_3$, δ) : 7.05-7.2 (4H, m), 5.64 (1H, br s), 5.54 (1H, br s), 5.14 (1H, m), 3.08 (2H, d, J=7Hz), 2.70 (3H, s), 2.54-2.64 (4H, m), 1.5-1.65 (2H, m), 1.25-1.4 (2H, m), 0.92 (3H, t, J=7Hz)

Preparation 80

(3R)-4-(4-Methylphenyl)-3-methanesulfonyloxybutanamide

NMR ($CDCl_3$, δ) : 7.05-7.15 (4H, m), 5.60 (1H, br s), 5.44 (1H, br s), 5.14 (1H, m), 3.08 (2H, d, J=7Hz), 2.70 (3H, s), 2.64 (2H, d, J=7Hz), 2.30 (3H, s)

Preparation 81

(3R)-3-Methanesulfonyloxy-4-(2-naphthyl)butanamide

Preparation 82

(3R)-3-Methanesulfonyloxydodecanamide

NMR ($CDCl_3$, δ) : 5.72 (1H, br s), 5.52 (1H, br s), 5.05 (1H, m), 3.04 (3H, s), 2.62 (2H, d, J=7Hz), 1.83 (2H, m), 1.2-1.5 (14H, m), 0.90 (3H, t, J=7Hz)

Preparation 83

(3R)-3-Methanesulfonyloxynonanamide

NMR ($CDCl_3$, δ) : 5.84 (1H, br s), 5.76 (1H, br s), 5.02 (1H, m), 3.02 (3H, s), 2.6 (2H, d, J=7Hz), 1.7-1.9 (2H, m), 1.2-1.45 (8H, m), 0.88 (3H, t, J=7Hz)

Preparation 84

(3R)-3-Methanesulfonyloxyheptanamide

NMR ($CDCl_3$, δ) : 5.9 (1H, br s), 5.8 (1H, br s), 5.04 (1H, m), 3.04 (3H, s), 2.62 (2H, d, J=7Hz), 1.7-1.9 (2H, m), 1.2-1.4 (4H, m), 0.90 (3H, t, J=7Hz)

Preparation 85

(3R)-4-(6-Ethyl-2-naphthyl)-3-methanesulfonyloxybutanamide

NMR (DMSO-$d_6$, δ) : 7.8 (2H, d, J=8, 4Hz), 7.7 (2H, d, J=8Hz), 7.48 (1H, br s), 7.28 (2H, dd, J=8, 3Hz), 7.04 (1H, br s), 5.22 (1H, m), 3.2 (2H, ABX), 2.95 (3H, s), 2.76 (2H, q, J=7Hz), 2.46 (2H, d, J=7Hz), 1.26 (3H, t, J=7Hz)

The following compounds (Preparations 86 to 93) were obtained according to a similar manner to that of Preparation 18.

Preparation 86

(3S)-3-Azido-4- (4-n-heptylphenyl)butanamide

Preparation 87

(3S)-3-Azido-4-(4-n-butylphenyl)butanamide

Preparation 88

(3S)-3-Azido-4-(4-methylphenyl)butanamide

Preparation 89

(3S)-3-Azido-4-(2-naphthyl)butanamide

Preparation 90

(3S)-3-Azidododecanamide

NMR ($CDCl_3$, δ) : 5.66 (1H, br s), 5.55 (1H, br s), 3.84 (1H, m), 2.3-2.5 (2H, m), 1.58 (2H, t, J=7Hz), 1.2-1.5 (14H, m), 0.88 (3H, t, J=7Hz)

Preparation 91

(3S)-3-Azidononanamide

NMR ($CDCl_3$, δ) : 5.65 (1H, br s), 5.55 (1H, br s), 3.86 (1H, m), 2.4 (2H, ABX), 1.2-1.65 (10H, m), 0.88 (3H, t, J=7Hz)

Preparation 92

(3S)-3-Azidoheptanamide

NMR ($CDCl_3$, δ) : 5.65 (1H, br s), 5.55 (1H, br s), 3.82 (1H, m), 2.4 (2H, ABX), 1.2-1.7 (6H, m), 0.9 (3H, t, J=7Hz)

Preparation 93

(3S)-3-Azido-4-(6-ethyl-2-naphthyl)butanamide

The following compounds (Preparations 94 to 101) were obtained according to a similar manner to that of Preparation 19.

Preparation 94

(3S)-3-Amino-4-(4-n-heptylphenyl)butanamide hydrochloride

NMR (DMSO-$d_6$, δ) : 8.04 (3H, br s), 7.62 (1H, br s), 7.05-7.2 (5H, m), 3.56 (1H, m), 2.96 (1H, dd, J=12, 5Hz), 2.74 (1H, dd, J=12, 8Hz), 2.5-2.6 (2H, m), 2.35 (2H, d, J=7Hz), 1.5-1.65 (2H, m), 1.2-1.35 (8H, m), 0.86 (3H, t, J=7Hz)

Preparation 95

(3S)-3-Amino-4-(4-n-butylphenyl)butanamide hydrochloride

NMR (DMSO-$d_6$, δ) : 8.06 (3H, br s), 7.62 (1H, br s), 7.1-7.2 (5H, m), 3.58 (1H, m), 2.96 (1H, dd, J=12, 8Hz), 2.74 (1H, dd, J=12, 5Hz), 2.54 (2H, t, J=7Hz), 2.36 (2H, d, J=7Hz), 1.48-1.62 (2H, m), 1.22-1.38 (2H, m), 0.90 (3H, t, J=7Hz)

Preparation 96

(3S)-3-Amino-4-(4-methylphenyl)butanamide hydrochloride

NMR (DMSO-$d_6$, δ) : 8.06 (3H, br s), 7.62 (1H, br s), 7.05-7.2 (5H, m), 3.56 (1H, m), 2.96 (1H, dd, J=12, 5Hz), 2.74 (1H, dd, J=12, 8Hz), 2.74 (2H, t, J=7Hz), 2.34 (2H, d, J=7Hz), 2.28 (3H, s)

Preparation 97

(3S)-3-Amino-4-(2-naphthyl)butanamide

NMR ($CD_3OD$-$CDCl_3$, δ) : 7.75-7.87 (3H, m), 7.64 (1H, s), 7.42-7.54 (2H, m), 7.32 (1H, d, J=8Hz), 7.11 (0.25H, br s), 5.62 (0.25H, br s), 3.54 (1H, m), 2.98 (1H, dd, J=14, 6Hz), 2.76 (1H, dd, J=14, 8Hz), 2.46 (1H, dd, J=15, 3Hz), 2.26 (1H, dd, J=15, 8Hz)

Preparation 98

(3S)-3-Aminododecanamide hydrochloride

NMR (DMSO-$d_6$, δ) : 8.04 (3H, br s), 7.66 (1H, br s), 7.12 (1H, br s), 3.32 (1H, m), 2.42 (2H, d, J=7Hz), 1.38-1.6 (2H, m), 1.15-1.38 (14H, m), 0.84 (3H, t, J=7Hz)

Preparation 99

(3S)-3-Aminononanamide hydrochloride

NMR (DMSO-$d_6$, δ) : 8.08 (3H, br s), 7.68 (1H, br s), 7.12 (1H, br s), 3.32 (1H, m), 2.44 (2H, d, J=7Hz), 1.4-1.65 (2H, m), 1.2-1.4 (8H, m), 0.86 (3H, t, J=7Hz)

Preparation 100

(3S)-3-Aminoheptanamide

NMR (DMSO-$d_6$, δ) : 7.36 (1H, br s), 6.72 (1H, br s), 2.94 (1H, m), 2.10 (1H, dd, J=12, 5Hz), 1.96 (1H, dd, J=12, 8Hz), 1.15-1.4 (6H, m), 0.84 (3H, t, J=7Hz)

Preparation 101

(3S)-3-Amino-4-(6-ethyl-2-naphthyl)butanamide

NMR (DMSO-$d_6$, δ) : 7.8 (2H, m), 7.7 (2H, d, J=7Hz), 7.62 (1H, br s), 7.36 (2H, m), 7.1 (1H, br s), 3.7 (1H, m), 3.16 (1H, dd, J=12, 5Hz), 2.95 (1H, dd, J=12, 7Hz), 2.76 (2H, q, J=7Hz), 2.4 (2H, d, J=7Hz), 1.26 (3H, t, J=7Hz)

The following compounds (Preparations 102 to 127) were obtained according to a similar manner to that of Preparation 47.

Preparation 102

(3S)-4-(2-Naphthyl)-3-(n-propylamino)butanamide

NMR ($CDCl_3$, δ) : 8.20 (1H, br s), 7.76-7.86 (3H, m), 7.60 (1H, s), 7.4-7.5 (2H, m), 7.30 (1H, d, J=8Hz), 5.34 (1H, br s), 3.26 (1H, m), 2.98 (2H, dd, J=7, 2Hz), 2.65 (2H, t, J=7Hz), 2.52 (1H, dd, J=12, 3Hz), 2.26 (1H, dd, J=12, 5Hz), 1.4-1.6 (2H, m), 0.88 (3H, t, J=7Hz)
ESI-MS : 271 [M+H]

Preparation 103

(3S)-3-(n-Butyl)amino-4-(4-n-heptylphenyl)butanamide

NMR ($CDCl_3$, δ) : 8.26 (1H, br s), 7.0-7.2 (4H, m), 5.34 (1H, br s), 3.12 (1H, m), 2.76 (2H, d, J=7Hz), 2.54-2.7 (4H, m), 2.48 (1H, dd, J=12, 3Hz), 2.20 (1H, dd, J=12, 5Hz), 1.5-1.7 (6H, m), 1.2-1.5 (8H, m), 0.85-0.95 (6H, m)

Preparation 104

(3S)-3-(n-Butyl)amino-4-(4-n-butylphenyl)butanamide

NMR ($CDCl_3$, δ) : 8.08 (1H, br s), 7.0-7.2 (4H, m), 5.38 (1H, br s), 3.15 (1H, m), 2.0-2.9 (9H, m), 1.25-1.65 (8H, m), 0.85-0.95 (6H, m)

Preparation 105

(3S)-4-(4-n-Butyl)phenyl-3-(n-propylamino)butanamide

NMR ($CDCl_3$, δ) : 7.82 (1H, br s), 7.0-7.2 (4H, m), 5.46 (1H, br s), 3.20 (1H, m), 2.25-2.95 (9H, m), 1.25-1.65 (6H, m), 0.85-0.95 (6H, m)

Preparation 106

(3S)-4-(4-Methylphenyl)-3-(n-propylamino)butanamide

NMR ($CDCl_3$, δ) : 8.22 (1H, br s), 7.0-7.2 (4H, m), 5.34 (1H, br s), 3.12 (1H, m), 2.76 (2H, dd, J=7, 3Hz), 2.62 (2H, dt, J=7, 2Hz), 2.46 (1H, dd, J=12, 3Hz), 2.32 (3H, s), 2.20 (1H, dd, J=12, 7Hz), 1.4-1.55 (2H, m), 0.88 (3H, t, J=7Hz)

Preparation 107

(3S)-3-(n-Butyl)amino-4- (2-naphthyl)butanamide

NMR ($CDCl_3$, δ) : 8.20 (1H, br s), 7.76-7.86 (3H, m), 7.60 (1H, s), 7.4-7.5 (2H, m), 7.30 (1H, d, J=8Hz), 5.34 (1H, br s), 3.24 (1H, m), 2.98 (2H, dd, J=7, 2Hz), 2.65 (2H, t, J=7Hz), 2.52 (1H, dd, J=12, 3Hz), 2.24 (1H, dd, J=12, 5Hz), 1.2-1.5 (4H, m), 0.88 (3H, t, J=7Hz)

Preparation 108

(3S)-3-Ethylamino-4-(2-naphthyl)butanamide

Preparation 109

(3S)-3-Isopentylaminohexadecanamide

NMR ($CDCl_3$, δ) : 8.12 (1H, br s), 5.38 (1H, br s), 2.86 (1H, m), 2.1-2.25 (2H, m), 2.50 (1H, dd, J=12, 3Hz), 2.26 (1H, dd, J=12, 8Hz), 1.2-1.7 (27H, m), 0.8-0.9 (9H, m)
ESI-MS : 341 [M+H]

Preparation 110

(3S)-3-Isobutylaminohexadecanamide

NMR ($CDCl_3$, δ) : 8.22 (1H, br s), 5.32 (1H, br s), 2.84 (1H, m), 2.35-2.6 (3H, m), 2.22 (1H, dd, J=12, 8Hz), 1.2-1.8 (25H, m), 0.92-0.98 (6H, m), 0.86 (3H, t, J=7Hz)
ESI-MS : 327 [M+H]

Preparation 111

(3S)-4-(2-Naphthyl)-3-(n-pentylamino)butanamide

NMR ($CDCl_3$, δ) : 8.20 (1H, br s), 7.76-7.87 (3H, m), 7.62 (1H, s), 7.42-7.52 (1H, m), 7.31 (1H, dd, J=8, 3Hz), 5.35 (1H, br s), 3.25 (1H, m), 2.98 (2H, d, J=7Hz), 2.67 (2H, t, J=7Hz), 2.53 (1H, dd, J=16, 3Hz), 2.24 (1H, dd, J=16, 7Hz), 1.36-1.50 (2H, m), 1.18-1.34 (4H, m), 0.84 (3H, t, J=7Hz)
ESI-MS : 299 [M+H]

Preparation 112

(3S)-3-Ethylaminohexadecanamide

Preparation 113

(3S)-3-(n-Butylamino)hexadecanamide

NMR ($CDCl_3$, δ) : 7.48 (1H, s), 5.65 (1H, s), 3.09 (1H, m), 2.87 (1H, m), 2.43-2.79 (3H, m), 1.08-1.76 (28H, m), 0.96 (3H, t, J=7Hz), 0.88 (3H, t, J=7Hz)
ESI-MS : 327 [M+H]

Preparation 114

(3S)-3-Phenethylamino)hexadecanamide

NMR ($CDCl_3$, δ) : 7.95 (1H, br s), 7.15-7.35 (5H, m), 5.08 (1H, br s), 2.74-3.02 (4H, m), 2.38 (1H, dd, J=16, 3Hz), 2.24 (1H, dd, J=16, 8Hz), 1.08-1.69 (24H, m), 0.88 (3H, t, J=7Hz)
ESI-MS : 375 [M+H]

Preparation 115

(3S)-3-(2-Pyridylmethylamino)hexadecanamide

NMR ($CDCl_3$, δ) : 8.56 (1H, m), 8.12 (1H, br s), 7.65 (1H, dd, J=8, 7Hz), 7.16-7.29 (2H, m), 5.31 (1H, br s), 3.95

(1H, s), 2.95 (1H, m), 2.52 (1H, dd, J=16, 4Hz), 2.24 (1H, dd, J=16, 7Hz), 1.16-1.68 (24H, m), 0.88 (3H, t, J=7Hz)
ESI-MS : 362 [M+H]

Preparation 116

(3S)-3-Benzylaminohexadecanamide

NMR ($CDCl_3$, δ) : 8.09 (1H, br s), 7.23-7.39 (5H, m), 5.28 (1H, br s), 3.83 (1H, d, J=14Hz), 3.76 (1H, d, J=14Hz), 2.95 (1H, m), 2.51 (1H, dd, J=13, 4Hz), 2.24 (1H, dd, J=13, 8Hz), 1.17-1.67 (24H, m), 0.88 (3H, t, J=7Hz)
ESI-MS : 361 [M+H]

Preparation 117

(3S)-3-(n-Pentylamino) dodecanamide

Preparation 118

(3S)-3-(n-Propylamino)dodecanamide

Preparation 119

(3S)-3-(n-Pentylamino)nonanamide

Preparation 120

(3S)-3-(n-Butylamino)nonanamide

Preparation 121

(3S)-3-(n-Propylamino)nonanamide

Preparation 122

(3S)-3-Ethylaminononanamide
ESI-MS : 201 [M+H]

Preparation 123

(3S)-3-(n-Propylamino)heptanamide

Preparation 124

(3S)-3-(n-Butylamino)heptanamide
ESI-MS : 201 [M+H]

Preparation 125

(3S)-3-(n-Propyl)amino-4-(6-ethyl-2-naphthyl)butanamide

Preparation 126

(3S)-3-(n-Butyl)amino-4-(6-ethyl-2-naphthyl)butanamide

Preparation 127

(3S)-3-(n-Pentylamino)hexadecanamide

NMR ($CDCl_3$, δ) : 7.80 (1H, br s), 5.52 (1H, br s), 3.00 (1H, m), 2.63-2.86 (2H, m), 2.57 (1H, dd, J=13, 8Hz), 2.38

(1H, dd, J=13, 8Hz), 1.18-1.68 (30H, m), 0.84-0.98 (6H, m)
ESI-MS : 341 [M+H]

The following compounds (Preparations 128 to 157) were obtained according to a similar manner to that of Preparation 35.

Preparation 128

(3S)-3-[N-(n-Butyl)-{(2S)-2-amino-5-methoxycarbonylpentanoyl}amino]-4-(4-n-heptylphenyl)butanamide hydrochloride
ESI-MS : 490 [M+H]

Preparation 129

(3S)-3-[N-(n-Propyl)-{(2S)-2-amino-4-benzyloxycarbonylbutanoyl}amino]-4-(2-naphthyl)butanamide hydrochloride
ESI-MS : 490 [M(free)+H]

Preparation 130

(3S)-3-[N-(n-Butyl)-{(2S)-2-amino-5-benzyloxycarbonylpentanoyl}amino]-4-(4-n-butylphenyl)butanamide hydrochloride
ESI-MS : 524 [M(free)+H]

Preparation 131

(3S)-3-[N-(n-Propyl)-{(2S)-2-amino-5-benzyloxycarbonylpentanoyl}amino]-4-(4-n-butylphenyl)butanamide hydrochloride
ESI-MS : 510 [M(free)+H]

Preparation 132

(3S)-3-[N-(n-Propyl)-{(2S)-2-amino-5-benzyloxycarbonylpentanoyl}amino]-4-(4-methylphenyl)butanamide hydrochloride
ESI-MS : 468 [M(free)+H]

Preparation 133

(3S)-3-[N-(n-Butyl)-{(2S)-2-amino-5-benzyloxycarbonylpentanoyl}amino]-4-(2-naphthyl)butanamide hydrochloride
ESI-MS : 518 [M(free)+H]

Preparation 134

(3S)-3-[N-Ethyl-{(2S)-2-amino-5-benzyloxycarbonylpentanoyl}amino]-4-(2-naphthyl)butanamide hydrochloride
ESI-MS : 490 [M(free)+H]

Preparation 135

(3S)-3-[N-Isopropyl-{(2S)-2-amino-5-benzyloxycarbonylpentanoyl}amino]hexadecanamide hydrochloride
ESI-MS : 574 [M(free)+H]

Preparation 136

(3S)-3-[N-Isobutyl-{(2S)-2-amino-5-benzyloxycarbonylpentanoyl}amino]hexadecanamide hydrochloride
ESI-MS : 560 [M(free)+H]

Preparation 137

(3S)-3-[N-(n-Pentyl)-{(2S)-2-amino-5-benzyloxycarbonylpentanoyl}amino]-4-(2-naphthyl)butanamide hydrochloride
ESI-MS : 532 [M(free)+H]

Preparation 138

(3S)-3-[N-(n-Propyl)-{(2S)-2-amino-5-benzyloxycarbonylpentanoyl}amino]-4-(2-naphthyl)butanamide hydrochloride
ESI-MS : 504 [M(free)+H]

Preparation 139

(3S)-3-[N-Ethyl-{(2S)-2-amino-5-benzyloxycarbonylpentanoyl}amino]hexadecanamide hydrochloride
ESI-MS : 532 [M(free)+H]

Preparation 140

(3S)-3-[N-(n-Propyl)-{(2S)-2-amino-5-benzyloxycarbonylpentanoyl}amino]hexadecanamide hydrochloride
ESI-MS : 560 [M+H]

Preparation 141

(3S)-3-[N-Phenethyl-{(2S)-2-amino-5-benzyloxycarbonylpentanoyl}amino]hexadecanamide hydrochloride
ESI-MS : 608 [M+H]

Preparation 142

(3S)-3-[N-(n-Pentyl)-{(2S)-2-amino-5-benzyloxycarbonylpentanoyl}amino]hexadecanamide hydrochloride
ESI-MS : 574 [M(free)+H]

Preparation 143

(3S)-3-[N-Benzyl-{(2S)-2-amino-5-benzyloxycarbonylpentanoyl}amino]hexadecanamide hydrochloride
ESI-MS : 594 [M(free)+H]

Preparation 144

(3S)-3-(N-(2-Pyridylmethyl)-{(2S)-2-amino-5-benzyloxycarbonylpentanoyl}amino]hexadecanamide dihydrochloride
ESI-MS : 595 [M(free)+H]

Preparation 145

(3S)-3-[(2S)-2-Amino-5-benzyloxycarbonylpentanoyl]-oxy-5-(2-naphthyl)pentanamide hydrochloride

NMR (DMSO-$d_6$, δ) : 8.57 (2H, br s), 7.80-7.92 (3H, m), 7.73 (1H, s), 7.30-7.45 (8H, m), 6.92 (1H, br s), 5.30 (1H, m), 5.09 (2H, s), 4.04 (1H, m), 2.72-2.92 (2H, m), 2.23-2.50 (4H, m), 1.52-2.07 (6H, m)

Preparation 146

(3S)-3-[(2S)-2-Amino-5-benzyloxycarbonylpentanoyl]-oxy-10-phenyldecanamide hydrochloride

NMR ($CDCl_3$, δ) : 8.2 (2H, br), 7.1-7.4 (11H, m), 6.75 (1H, s), 5.3 (1H, m), 5.08 (2H, s), 4.02 (1H, t, J=5.0Hz), 2.54 (3H, m), 2.40 (3H, m), 1.5-2.15 (8H, m), 1.2-1.35 (8H, m)

Preparation 147

3-[N-Methyl-{(2S)-2-amino-5-benzyloxycarbonylpentanoyl}amino]propanamide hydrochloride

Preparation 148

(3S)-3-[N-(n-Pentyl)-{(2S)-2-amino-5-benzyloxycarbonylpentanoyl}amino]dodecanamide hydrochloride

Preparation 149

(3S)-3-[N-(n-Propyl)-{(2S)-2-amino-5-methoxycarbonylpentanoyl}amino]dodecanamide hydrochloride
ESI-MS : 414 [M(free)+H]

Preparation 150

(3S)-3-[N-(n-Pentyl)-{(2S)-2-amino-5-benzyloxycarbonylpentanoyl}amino]nonanamide hydrochloride

Preparation 151

(3S)-3-[N-(n-Butyl)-{(2S)-2-amino-5-benzyloxycarbonylpentanoyl}amino]nonanamide hydrochloride

Preparation 152

(3S)-3-[N-(n-Propyl)-{(2S)-2-amino-5-benzyloxycarbonylpentanoyl}amino]nonanamide hydrochloride

Preparation 153

(3S)-3-[N-Ethyl-{(2S)-2-amino-5-methoxycarbonylpentanoyl}amino]nonanamide hydrochloride
ESI-MS : 358 [M(free)+H]

Preparation 154

(3S)-3-[N-(n-Propyl)-{(2S)-2-amino-5-benzyloxycarbonylpentanoyl}amino]heptanamide hydrochloride

Preparation 155

(3S)-3-[N-(n-Butyl)-{(2S)-2-amino-5-methoxycarbonylpentanoyl}amino]heptanamide hydrochloride
ESI-MS : 358 [M(free)+H]

Preparation 156

(3S)-3-[N-(n-Propyl)-{(2S)-2-amino-5-benzyloxycarbonylpentanoyl}amino]-4-(6-ethyl-2-naphthyl)butanamide hydrochloride

Preparation 157

(3S)-3-[N-(n-Butyl)-{(2S)-2-amino-5-benzyloxycarbonylpentanoyl}amino]-4-(6-ethyl-2-naphthyl)butanamide hydrochloride

The following compounds (Examples 39 to 43) were obtained according to a similar manner to that of Example 1.

Example 39

(3S)-3-[(2S)-5-Benzyloxycarbonyl-2-(tert-butoxycarbonylamino)pentanoyl]oxy-10-phenyldecanamide

NMR ($CDCl_3$, $\delta$) : 7.1-7.4 (5H, m), 5.9 (1H, br s), 5.3 (1H, br s), 5.22 (1H, m), 5.1 (2H, s), 5.06 (1H, d, J=10.0Hz), 4.22 (1H, m), 2.08 (2H, m), 2.3-2.5 (4H, m), 1.2-1.9 (30H, m)
FAB-MS : 619 [M+Na]

Example 40

(3S)-3-[(2S)-5-Benzyloxycarbonyl-2-(tert-butoxycarbonylamino)pentanoyl]oxy-4-(4-biphenylyl)butanamide mp : 94-98°C

Example 41

(35)-3-[N-(n-Butyl)-{(2S)-5-benzyloxycarbonyl-2-(tert-butoxycarbonylamino)pentanoyl}amino]-4-(4-n-butylphenyl)-butanamide
ESI-MS : 624 [M+H]

Example 42

(3S)-3-[(2S)-5-Benzyloxycarbonyl-2-(tert-butoxycarbonylamino)pentanoyl]oxy-5-(2-naphthyl)pentanamide

NMR ($CDCl_3$, δ) : 7.74-7.84 (3H, m), 7.63 (1H, s), 7.28-7.51 (8H, m), 5.93 (1H, br s), 5.33 (1H, m), 5.30 (1H, br s), 5.12 (2H, s), 4.97 (1H, d, J=7Hz), 4.23 (1H, m), 2.84 (2H, t, J=7Hz), 2.52 (2H, d, J=6Hz), 2.33-2.42 (2H, m), 2.04-2.18 (2H, m), 1.60-1.90 (4H, m), 0.95 (9H, s)

Example 43

(3S)-3-[(2S)-5-Benzyloxycarbonyl-2-(tert-butoxycarbonylamino)pentanoyl]oxy-5-(n-decyloxy)pentanamide

NMR ($CDCl_3$, δ) : 7.31-7.40 (5H, m), 6.02 (1H, br s), 5.36 (1H, m), 5.28 (1H, br s), 5.12 (2H, s), 5.06 (1H, d, J=8Hz), 4.23 (1H, m), 3.34-3.57 (4H, m), 2.59 (1H, dd, J=15, 6Hz), 2.51 (1H, dd, J=15, 7Hz), 1.49-2.01 (8H, m), 1.44 (9H, s), 1.21-1.36 (14H, m), 0.88 (3H, t, J=7Hz)

The following compounds (Examples 44 to 136) were obtained according to a similar manner to that of Example 6.

Example 44

(3S)-3-[(2S)-5-Benzyloxycarbonyl-2-[2-(methoxycarbonylmethyl)benzoylamino]pentanoyl]oxy-4-(2-naphthyl)butanamide

NMR ($CDCl_3$, δ) : 7.72-7.83 (3H, m), 7.65 (1H, s), 7.52 (1H, d, J=7Hz), 7.22-7.47 (12H, m), 5.93 (1H, br s), 5.59 (1H, m), 5.25 (1H, br s), 5.08 (2H, s), 4.59 (1H, m), 3.93 (1H, d, J=15Hz), 3.80 (1H, d, J=15Hz), 3.67 (3H, s), 3.18 (2H, d, J=7Hz), 2.43-2.59 (2H, m), 2.16-2.34 (2H, m), 1.48-1.83 (4H, m)
ESI-MS : 639 [M+H]

Example 45

(3S)-3-[(2S)-5-Benzyloxycarbonyl-2-[2-(benzyloxy-carbonylmethyl)benzoylamino]pentanoyl]oxy-4-(2-naphthyl)-butanamide

NMR ($CDCl_3$, δ) : 7.72-7.82 (3H, m), 7.66 (1H, s), 7.52 (1H, d, J=7Hz), 7.22-7.47 (17H, m), 5.89 (1H, br s), 5.57 (1H, m), 5.22 (1H, br s), 5.12 (2H, s), 5.07 (2H, s), 4.57 (1H, m), 3.97 (1H, d, J=16Hz), 3.86 (1H, d, J=16Hz), 3.17 (2H, d, J=7Hz), 2.42-2.57 (2H, m), 2.13-2.31 (2H, m), 1.48-1.79 (4H, m)
ESI-MS : 715 [M+H]

Example 46

(3S)-3-[(2S)-5-Benzyloxycarbonyl-2-[2-((2E)-2-methoxycarbonylvinyl]benzoylamino]pentanoyl]oxy-4-(2-naphthyl)butanamide

NMR ($CDCl_3$, δ) : 7.98 (1H, d, J=16Hz), 7.60-7.77 (5H, m), 7.29-7.53 (11H, m), 6.45 (1H, d, J=8Hz), 6.34 (1H, d, J=16Hz), 6.18 (1H, br s), 5.63 (1H, m), 5.37 (1H, br s), 5.09 (2H, s), 4.61 (1H, m), 3.75 (3H, s), 3.10-3.26 (2H, m), 2.54-2.69 (2H, m), 2.12-2.36 (2H, m), 1.46-1.86 (4H, m)

ESI-MS : 651 [M+H]

Example 47

(3S)-3-[(2S)-5-Benzyloxycarbonyl-2-(3-benzylnaphthalen-2-ylcarbonylamino)pentanoyl]oxy-4-(4-n-heptylphenyl)butanamide

NMR ($CDCl_3$, δ) : 7.93 (1H, s), 7.75-7.88 (2H, m), 7.66 (1H, s), 7.46-7.58 (2H, m), 7.27-7.38 (5H, m), 7.06-7.24 (9H, m), 6.33 (1H, d, J=7Hz), 5.75 (1H, br s), 5.46 (1H, m), 5.23 (1H, br s), 5.08 (2H, s), 4.53 (1H, m), 4.47 (1H, d, J=16Hz), 4.28 (1H, d, J=16Hz), 2.88-3.04 (2H, m), 2.47-2.56 (2H, m), 2.37-2.45 (2H, m), 2.22-2.32 (2H, m), 1.38-1.82 (6H, m), 1.15-1.34 (8H, m), 0.82-0.92 (3H, m)
ESI-MS : 755 [M+H]

Example 48

(3S)-3-[(2S)-5-Benzyloxycarbonyl-2-(2-quinolylcarbonylamino)pentanoyl]oxy-5-(2-naphthyl)pentanamide

NMR ($CDCl_3$, δ) : 8.73 (1H, d, J=8Hz), 8.33 (1H, d, J=8Hz), 8.26 (1H, d, J=8Hz), 8.18 (1H, d, J=8Hz), 7.90 (1H, d, J=8Hz), 7.61-7.83 (5H, m), 7.59 (1H, s), 7.25-7.46 (7H, m), 5.96 (1H, br s), 5.41 (1H, m), 5.29 (1H, br s), 5.12 (2H, s), 4.77 (1H, m), 2.86 (2H, t, J=8Hz), 2.57 (2H, d, J=6Hz), 2.45 (2H, d, J=7Hz), 1.75-2.22 (6H, m)

Example 49

(3S)-3-[(2S)-5-Benzyloxycarbonyl-2-[1-(4-methylbenzyl)-indol-2-ylcarbonylamino]pentanoyl]oxy-4-(2-naphthyl)-butanamide

NMR ($CDCl_3$, δ) : 7.67-7.78 (4H, m), 7.63 (1H, s), 7.27-7.45 (9H, m), 7.17 (1H, m), 6.86-7.05 (6H, m), 5.84 (1H, br s), 5.81 (1H, d, J=16Hz), 5.68 (1H, d, J=16Hz), 5.57 (1H, m), 5.16 (1H, br s), 5.11 (2H, s), 4.55 (1H, m), 3.08 (2H, m), 2.07-2.48 (4H, m), 2.25 (3H, s), 1.38-1.82 (4H, m)
ESI-MS : 710 [M+H]

Example 50

(3S)-3-[(2S)-5-Benzyloxycarbonyl-2-[1-(4-chlorobenzyl)-indol-3-ylcarbonylamino]pentanoyl]oxy-4-(2-naphthyl)-butanamide

NMR ($CDCl_3$, δ) : 8.07 (1H, m), 7.67-7.78 (4H, m), 7.53 (1H, s), 7.21-7.47 (13H, m), 7.07 (2H, d, J=8Hz), 6.86 (1H, d, J=7Hz), 6.07 (1H, br s), 5.62 (1H, m), 5.32 (1H, br s), 5.30 (2H, s), 5.08 (2H, s), 4.70 (1H, m), 3.16 (2H, d, J=7Hz), 2.57 (1H, dd, J=15, 4Hz), 2.48 (1H, dd, J=15, 7Hz), 2.11-2.36 (2H, m), 1.45-1.90 (4H, m)
ESI-MS : 730 [M+H]

Example 51

(3S)-3-[(2S)-5-Benzyloxycarbonyl-2-[1-(4-methylbenzyl)-indol-3-ylcarbonylamino]pentanoyl]oxy-4-(2-naphthyl)-butanamide

NMR ($CDCl_3$, δ) : 8.06 (1H, m), 7.67-7.77 (4H, m), 7.62 (1H, br s), 7.20-7.43 (11H, m), 7.13 (2H, d, J=8Hz), 7.07 (2H, d, J=8Hz), 6.73 (1H, d, J=7Hz), 6.13 (1H, br s), 5.59 (1H, m), 5.29 (2H, s), 5.27 (1H, br s), 5.07 (2H, s), 4.67 (1H, m), 3.15 (2H, d, J=7Hz), 2.57 (1H, dd, J=14, 4Hz), 2.48 (1H, dd, J=14, 7Hz), 2.32 (3H, s), 2.07-2.28 (2H, m), 1.43-1.88 (4H, m)
ESI-MS : 710 [M+H]

Example 52

(3S)-3-[N-(2-Pyridylmethyl)-{(2S)-5-benzyloxycarbonyl-2-(2-quinolylcarbonylamino)pentanoyl}amino]hexadecanamide
ESI-MS : 750 [M+H]

Example 53

(3S)-3-[N-Benzyl-{(2S)-5-benzyloxycarbonyl-2-(2-quinolylcarbonylamino)pentanoyl}amino]hexadecanamide
ESI-MS : 749 [M+H]

Example 54

(3S)-3-[N-(n-Pentyl)-{(2S)-5-benzyloxycarbonyl-2-(2-quinolylcarbonylamino)pentanoyl}amino]hexadecanamide
ESI-MS : 729 [M+H]

Example 55

(3S)-3-[N-Phenethyl-{(2S)-5-benzyloxycarbonyl-2-(2-quinolylcarbonylamino)pentanoyl}amino]hexadecanamide
ESI-MS : 763 [M+H]

Example 56

(3S)-3-[N-(n-Butyl)-{(2S)-5-benzyloxycarbonyl-2-(2-quinolylcarbonylamino)pentanoyl}amino]hexadecanamide
ESI-MS : 737 [M+H]

Example 57

(3S)-3-[N-Ethyl-{(2S)-5-benzyloxycarbonyl-2-(2-quinolylcarbonylamino)pentanoyl}amino]hexadecanamide
ESI-MS : 687 [M+H]

Example 58

(3S)-3-[N-(n-Propyl)-{(2S)-2-(1-benzylindol-3-ylcarbonylamino)-5-benzyloxycarbonylpentanoyl}amino]-4-(2-naphthyl)butanamide
ESI-MS : 737 [M+H]

Example 59

(3S)-3-[N-(n-Propyl)-{(2S)-5-benzyloxycarbonyl-2-(1-(1-naphthylmethyl)indol-3-ylcarbonylamino)pentanoyl}amino]-4-(2-naphthyl)butanamide
ESI-MS : 787 [M+H]

Example 60

(3S)-3-[N-(n-Pentyl)-{(2S)-5-benzyloxycarbonyl-2-(1-benzylindol-3-ylcarbonylamino)pentanoyl}amino]-4-(2-naphthyl)butanamide
ESI-MS : 765 [M+H]

Example 61

(3S)-3-[N-(n-Pentyl)-{(2S)-5-benzyloxycarbonyl-2-(1-(1-naphthylmethyl)indol-3-ylcarbonylamino)pentanoyl}amino]-4-(2-naphthyl)butanamide
ESI-MS : 815 [M+H]

Example 62

(3S)-3-[N-Isobutyl-{(2S)-5-benzyloxycarbonyl-2-(2-quinolylcarbonylamino)pentanoyl}amino]hexadecanamide
ESI-MS : 715 [M+H]

Example 63

(3S)-3-[N-Isopentyl-{(2S)-5-benzyloxycarbonyl-2-(2-quinolylcarbonylamino)pentanoyl}amino]hexadecanamide
ESI-MS : 729 [M+H]

Example 64

(3S)-3-[N-Ethyl-{(2S)-5-benzyloxycarbonyl-2-(1-(1-benzylindol-3-ylcarbonylamino)pentanoyl)amino]-4-(2-naphthyl)butanamide
ESI-MS : 723 [M+H]

Example 65

(3S)-3-[N-Ethyl-{(2S)-5-benzyloxycarbonyl-2-(1-naphthylmethyl) indol-3-ylcarbonylamino)pentanoyl}amino]-4-(2-naphthyl)butanamide
ESI-MS : 773 [M+H]

Example 66

(3S)-3-[N-Ethyl-{(2S)-5-benzyloxycarbonyl-2-(1-(2-chlorobenzyl)indol-3-ylcarbonylamino)pentanoyl}amino]-4-(2-naphthyl)butanamide
ESI-MS : 757 [M+H]

Example 67

(3S)-3-[N-(n-Butyl)-{(2S)-5-benzyloxycarbonyl-2-(1-benzylindol-3-ylcarbonylamino)pentanoyl}amino]-4-(2-naphthyl)butanamide
ESI-MS : 751 [M+H]

Example 68

(3S)-3-[N-(n-Butyl)-{(2S)-5-benzyloxycarbonyl-2-(1-(2-chlorobenzyl)indol-3-ylcarbonylamino)pentanoyl}amino]-4-(2-naphthyl)butanamide
ESI-MS : 785 [M+H]

Example 69

(3S)-3-[N-(n-Propyl)-{(2S)-2-(1-benzylindol-3-ylcarbonylamino)-5-benzyloxycarbonylpentanoyl}amino]-4-(4-methylphenyl)butanamide

Example 70

(3S)-3-[N-(n-Propyl)-{(2S)-5-benzyloxycarbonyl-2-(1-(2-chlorobenzyl)indol-3-ylcarbonylamino)pentanoyl} amino]-4-(4-methylphenyl)butanamide

Example 71

(3S)-3-[N-(n-Propyl)-{(2S)-5-benzyloxycarbonyl-2-(1-benzylindol-3-ylcarbonylamino)pentanoyl}amino]-4-(4-n-butylphenyl)butanamide
ESI-MS : 743 [M+H]

Example 72

(3S)-3-[N-(n-Propyl)-{(2S)-5-benzyloxycarbonyl-2-(1-(2-chlorobenzyl)indol-3-ylcarbonylamino)pentanoyl} amino]-4-(4-n-butylphenyl)butanamide
ESI-MS : 777 [M+H]

Example 73

(3S)-3-[N-(n-Butyl)-{(2S)-5-benzyloxycarbonyl-2-(1-benzylindol-3-ylcarbonylamino)pentanoyl}amino]-4-(4-n-butylphenyl)butanamide

ESI-MS : 757 [M+H]

Example 74

(3S)-3-[N-(n-Butyl)-{(2S)-5-benzyloxycarbonyl-2-(1-(2-chlorobenzyl)indol-3-ylcarbonylamino)pentanoyl}amino]-4-(4-n-butylphenyl)butanamide

ESI-MS : 791 [M+H]

Example 75

(3S)-3-[N-(n-Propyl)-{(2S)-4-benzyloxycarbonyl-2-(1-(2-chlorobenzyl)indol-3-ylcarbonylamino}butanoyl}amino]-4-(2-naphthyl)butanamide

ESI-MS : 757 [M+H]

Example 76

(3S)-3-[N-(n-Butyl)-{(2S)-5-methoxycarbonyl-2-(2-quinolylcarbonylamino)pentanoyl}amino]-4-(4-n-heptylphenyl)butanamide

ESI-MS : 667 [M+H]

Example 77

(3S)-3-[N-(n-Butyl)-{(2S)-5-methoxycarbonyl-2-(1-(2-chlorobenzyl)indol-3-ylcarbonylamino)pentanoyl}amino]-4-(4-n-heptylphenyl)butanamide

ESI-MS : 757 [M+H]

Example 78

(3S)-3-[(2S)-5-Benzyloxycarbonyl-2-(3-quinolylcarbonylamino)pentanoyl]oxy-10-phenyldecanamide

NMR ($CDCl_3$, δ) : 9.34 (1H, s), 8.62 (1H, s), 8.16 (1H, d, J=10Hz), 7.90 (1H, d, J=10Hz), 7.82 (1H, m), 7.64 (1H, m), 7.1-7.4 (11H, m), 5.94 (1H, br s), 5.50 (1H, br s), 5.35 (1H, m), 5.12 (2H, s), 4.80 (1H, m), 2.4-2.7 (6H, m), 1.5-2.15 (8H, m), 1.2-1.4 (8H, m)
FAB-MS : 652 [M+H]

Example 79

(3S)-3-[(2S)-5-Benzyloxycarbonyl-2-(3-quinolylcarbonylamino)pentanoyl]oxy-4-(2-naphthyl)butanamide

NMR ($CDCl_3$, δ) : 9.32 (1H, d, J=2Hz), 8.56 (1H, d, J=2Hz), 8.15 (1H, d, J=15Hz), 7.6-7.9 (7H, m), 7.25-7.4 (8H, m), 5.9 (1H, br s), 5.62 (1H, m), 5.40 (1H, br s), 5.10 (2H, s), 4.68 (1H, m), 3.15 (2H, d, J=7Hz), 2.45-2.6 (2H, m), 2.15-2.4 (2H, m), 1.75-1.9 (2H, m), 1.5-1.7 (2H, m)
FAB-MS : 618 [M+H]

Example 80

(3S)-3-[(2S)-5-Benzyloxycarbonyl-2-(isoquinolin-3-ylcarbonylamino)pentanoyl]oxy-4-(2-naphthyl)butanamide

NMR ($CDCl_3$, δ) : 9.2 (1H, s), 8.66 (1H, d, J=15Hz), 8.56 (1H, s), 8.1 (1H, d, J=15Hz), 8.0 (1H, d, J=15Hz), 7.6-7.85 (5H, m), 7.25-7.4 (8H, m), 6.0 (1H, br s), 5.62 (1H, m), 5.35 (1H, br s), 5.10 (2H, s), 4.75 (1H, m), 3.15 (2H, d, J=7Hz), 2.45-2.6 (2H, m), 2.15-2.4 (2H, m), 1.75-1.9 (2H, m), 1.5-1.7 (2H, m)
FAB-MS : 618 [M+H]

Example 81

(3S)-3-[(2S)-5-Benzyloxycarbonyl-2-(isoquinolin-1-ylcarbonylamino)pentanoyl]oxy-4-(2-naphthyl)butanamide

NMR ($CDCl_3$, δ) : 9.5 (1H, d, J=15Hz), 8.65 (1H, d, J=15Hz), 8.5 (1H, d, J=10Hz), 7.6-7.9 (10H, m), 7.2-7.4 (5H, m), 6.04 (1H, br s), 5.58 (1H, m), 5.42 (1H, br s), 5.05 (2H, s), 4.7 (1H, m), 3.12 (2H, m), 2.5 (2H, m), 2.2-2.3 (2H, m), 1.5-1.9 (2H, m)
FAB-MS : 618 [M+H]

Example 82

(3S)-3-[(2S)-2-(2-Benzylbenzoyl)amino-5-benzyloxycarbonylpentanoyl]oxy-4-(2-naphthyl)butanamide

NMR ($CDCl_3$, δ) : 7.05-7.8 (21H, m), 6.22 (1H, d, J=10Hz), 5.82 (1H, br s), 5.55 (1H, m), 5.26 (1H, m), 5.05 (2H, s), 4.5 (1H, m), 4.2 (2H, ABq), 3.15 (2H, t, J=7Hz), 2.4-2.55 (2H, m), 2.05-2.25 (2H, m), 1.3-1.7 (4H, m)
ESI-MS : 657 [M+H]

Example 83

(3S)-3-[(2S)-5-Benzyloxycarbonyl-2-(2-naphthylcarbonylamino)pentanoyl]oxy-4-(2-naphthyl)butanamide

NMR (DMSO-$d_6$, δ) : 8.9 (1H, d, J=10Hz), 8.52 (1H, s), 7.3-8.05 (14H, m), 6.85 (1H, br s), 5.46 (1H, m), 5.08 (2H, s), 4.44 (1H, m), 3.0-3.2 (2H, m), 2.3-2.4 (4H, m), 1.5-1.9 (4H, m)
ESI-MS : 617 [M+H]

Example 84

(3S)-3-[(2S)-2-Benzoylamino-5-benzyloxycarbonylpentanoyl]oxy-4-(2-naphthyl)butanamide

NMR ($CDCl_3$, δ) : 8.16 (1H, d, J=10Hz), 7.3-7.85 (11H, m), 6.9 (1H, d, J=8Hz), 5.94 (1H, br s), 5.6 (1H, m), 5.35 (1H, br s), 5.1 (2H, s), 4.12 (1H, m), 3.12 (2H, d, J=7Hz), 2.1-2.6 (4H, m), 1.45-1.9 (4H, m)
ESI-MS : 567 [M+H]

Example 85

(3S)-3-[(2S)-5-Benzyloxycarbonyl-2-(2-phenethylbenzoyl)aminopentanoyl]oxy-4-(2-naphthyl)butanamide

NMR ($CDCl_3$, δ) : 7.1-7.8 (21H, m), 6.25 (1H, d, J=10Hz), 5.78 (1H, br s), 5.6 (1H, m), 5.26 (1H, br s), 5.08 (2H, s), 4.6 (1H, m), 3.14 (2H, d, J=7Hz), 3.0-3.1 (2H, m), 2.85-2.95 (2H, m), 2.44 (2H, d, J=7Hz), 2.1-2.3 (2H, m), 1.45-1.8 (4H, m)
ESI-MS : 671 [M+H]

Example 86

(3S)-3-[(2S)-2-(3-Benzylbenzoyl)amino-5-benzyloxycarbonylpentanoyl]oxy-4-(2-naphthyl)butanamide

NMR ($CDCl_3$, δ) : 8.1 (1H, m), 7.1-7.75 (20H, m), 6.92 (1H, d, J=8Hz), 5.92 (1H, br s), 5.55 (1H, m), 5.36 (1H, br s), 5.05 (2H, s), 4.56 (1H, m), 4.02 (2H, s), 3.1 (2H, d, J=7Hz), 2.4-2.55 (2H, m), 2.05-2.3 (2H, m), 1.4-1.8 (4H, m)
ESI-MS : 657 [M+H]

Example 87

(3S)-3-[(2S)-2-(3-Benzylnaphthalen-2-ylcarbonyl)amino-5-benzyloxycarbonylpentanoyl]oxy-4-(6-ethyl-2-naphthyl)-butanamide

NMR ($CDCl_3$, δ) : 7.05-7.85 (22H, m), 6.32 (1H, d, J=10Hz), 5.82 (1H, br s), 5.55 (1H, m), 5.26 (1H, m), 5.05 (2H, s), 4.45 (1H, m), 4.32 (2H, ABq), 3.15 (2H, t, J=7Hz), 2.75 (2H, q, J=7Hz), 2.4-2.55 (2H, m), 2.05-2.25 (2H, m),

1.3-1.7 (4H, m), 1.26 (3H, t, J=7Hz)
ESI-MS : 735 [M+H]

Example 88

3-[N-Methyl-{(2S)-2-(3-benzylnaphthalen-2-ylcarbonyl)-amino-5-benzyloxycarbonylpentanoyl}amino]propanamide
ESI-MS : 580 [M+H]

Example 89

(3S)-3-[N-(n-Pentyl)-{(2S)-2-(1-benzylindol-3-ylcarbonyl)amino-5-benzyloxycarbonylpentanoyl}amino]-dodecanamide
ESI-MS : 751 [M+H]

Example 90

(3S)-3-[N-(n-Propyl)-{(2S)-2-(1-benzylindol-3-yl-carbonyl) amino-5-methoxycarbonylpentanoyl}amino]dodecanamide
ESI-MS : 647 [M+H]

Example 91

(3S)-3-[N-(n-Propyl)-{(2S)-2-(1-(2-chlorobenzyl)indol-3-ylcarbonyl)amino-5-methoxycarbonylpentanoyl}amino]-dodecanamide
ESI-MS : 681 [M+H]

Example 92

(3S)-3-[N-(n-Pentyl)-{(2S)-2-(1-benzylindol-3-yl-carbonyl)amino-5-benzyloxycarbonylpentanoyl}amino]nonanamide
ESI-MS : 709 [M+H]

Example 93

(3S)-3-[N-(n-Butyl)-{(2S)-2-(1-benzylindol-3-yl-carbonyl)amino-5-benzyloxycarbonylpentanoyl}amino]nonanamide
ESI-MS : 695 [M+H]

Example 94

(3S)-3-[N-(n-Butyl)-{(2S)-5-benzyloxycarbonyl-2-(1-(2-chlorobenzyl)indol-3-ylcarbonylamino)pentanoyl}amino]-nonanamide
ESI-MS : 729 [M+H]

Example 95

(3S)-3-[N-(n-Propyl)-{(2S)-2-(1-benzylindol-3-yl-carbonyl)amino-5-benzyloxycarbonylpentanoyl}amino]nonanamide
ESI-MS : 681 [M+H]

Example 96

(3S)-3-[N-(n-Propyl)-{(2S)-5-benzyloxycarbonyl-2-[(1-(1-naphthylmethyl)indol-3-ylcarbonyl)amino]pentanoyl}amino]-nonanamide
ESI-MS : 731 [M+H]

Example 97

(3S)-3-[N-(n-Propyl)-{(2S)-5-benzyloxycarbonyl-2-[(1-(2-pyridylmethyl)indol-3-ylcarbonyl)amino]pentanoyl}amino]-nonanamide

ESI-MS : 682 [M+H]

Example 98

(3S)-3-[N-(n-Propyl)-{(2S)-5-benzyloxycarbonyl-2-[(1-(2-chlorobenzyl)indol-3-ylcarbonyl)amino]pentanoyl}amino]-nonanamide

ESI-MS : 715 [M+H]

Example 99

(3S)-3-[N-(n-Propyl)-{(2S)-5-benzyloxycarbonyl-2-[(1-(3-chlorobenzyl)indol-3-ylcarbonyl)amino]pentanoyl}amino]-nonanamide

ESI-MS : 715 [M+H]

Example 100

(3S)-3-[N-Ethyl-{(2S)-2-(1-(2-chlorobenzyl)indol-3-ylcarbonyl)amino-5-methoxycarbonylpentanoyl}amino]nonanamide

ESI-MS : 625 [M+H]

Example 101

(3S)-3-[N-Ethyl-{(2S)-2-(1-benzylindol-3-ylcarbonyl)amino-5-methoxycarbonylpentanoyl}amino]nonanamide

ESI-MS : 591 [M+H]

Example 102

(3S)-3-[N-(n-Propyl)-{(2S)-2-(1-(2-chlorobenzyl)indol-3-ylcarbonyl)amino-5-benzyloxycarbonylpentanoyl}amino]-heptanamide

ESI-MS : 687 [M+H]

Example 103

(3S)-3-[N-(n-Propyl)-{(2S)-2-(1-(1-naphthylmethyl)indol-3-ylcarbonyl)amino-5-benzyloxycarbonylpentanoyl}amino]-heptanamide

ESI-MS : 703 [M+H]

Example 104

(3S)-3-[N-(n-Butyl)-{(2S)-2-(1-(2-chlorobenzyl)indol-3-ylcarbonyl)amino-5-methoxycarbonylpentanoyl}amino]-heptanamide

ESI-MS : 625 [M+H]

Example 105

(3S)-3-[N-(n-Butyl)-{(2S)-2-(1-(1-naphthylmethyl)indol-3-ylcarbonyl)amino-5-methoxycarbonylpentanoyl}amino]-heptanamide

ESI-MS : 641 [M+H]

Example 106

(3S)-3-[N-(n-Propyl)-{(2S)-5-benzyloxycarbonyl-2-(1-(2-pyridylmethyl)indol-3-ylcarbonylamino)pentanoyl}amino]-4-(6-ethyl-2-naphthyl)butanamide

ESI-MS : 766 [M+H]

Example 107

(3S)-3-[N-(n-Propyl)-{(2S)-2-(1-benzylindol-3-ylcarbonyl)amino-5-benzyloxycarbonylpentanoyl}amino]-4-(6-ethyl-2-naphthyl)butanamide
ESI-MS : 765 [M+H]

Example 108

(3S)-3-[N-(n-Butyl)-{(2S)-5-benzyloxycarbonyl-2-[(1-benzylindol-3-ylcarbonyl)amino]pentanoyl}amino]-4-(6-ethyl-2-naphthyl)butanamide
ESI-MS : 779 [M+H]

Example 109

(3S)-3-[N-(n-Butyl)-{(2S)-5-benzyloxycarbonyl-2-[(1-(2-chlorobenzyl)indol-3-ylcarbonyl)amino]pentanoyl}amino]-4-(6-ethyl-2-naphthyl)butanamide
ESI-MS : 813 [M+H]

Example 110

(3S)-3-[N-(2-Pyridylmethyl)-{(2S)-5-benzyloxycarbonyl-2-(tert-butoxycarbonylamino)pentanoyl}amino]hexadecanamide
ESI-MS : 695 [M+H]

Example 111

(3S)-3-[N-Benzyl-{(2S)-5-benzyloxycarbonyl-2-(tert-butoxycarbonylamino)pentanoyl}amino]hexadecanamide
ESI-MS : 694 [M+H]

Example 112

(3S)-3-[N-(n-Pentyl)-{(2S)-5-benzyloxycarbonyl-2-(tert-butoxycarbonylamino)pentanoyl}amino]hexadecanamide
ESI-MS : 674 [M+H]

Example 113

(3S)-3-[N-Phenethyl-{(2S)-5-benzyloxycarbonyl-2-(tert-butoxycarbonylamino)pentanoyl}amino]hexadecanamide
ESI-MS : 708 [M+H]

Example 114

(3S)-3-[N-(n-Butyl)-{(2S)-5-benzyloxycarbonyl-2-(tert-butoxycarbonylamino)pentanoyl}amino]hexadecanamide
ESI-MS : 660 [M+H]

Example 115

(3S)-3-[N-Ethyl-{(2S)-5-benzyloxycarbonyl-2-(tert-butoxycarbonylamino)pentanoyl}amino]hexadecanamide
ESI-MS : 632 [M+H]

Example 116

(3S)-3-[N-(n-Propyl)-{(2S)-5-benzyloxycarbonyl-2-(tert-butoxycarbonylamino)pentanoyl}amino]-4-(2-naphthyl)butanamide
ESI-MS : 604 [M+H]

Example 117

(3S)-3-[N-(n-Pentyl)-{(2S)-5-benzyloxycarbonyl-2-(tert-butoxycarbonylamino)pentanoyl}amino]-4-(2-naphthyl)butanamide
ESI-MS : 632 [M+H]

Example 118

(3S)-3-[N-Isobutyl-{(2S)-5-benzyloxycarbonyl-2-(tert-butoxycarbonylamino)pentanoyl}amino]hexadecanamide
ESI-MS : 660 [M+H]

Example 119

(3S)-3-[N-Isopentyl-{(2S)-5-benzyloxycarbonyl-2-(tert-butoxycarbonylamino)pentanoyl}amino]hexadecanamide
ESI-MS : 674 [M+H]

Example 120

(3S)-3-[N-Ethyl-{(2S)-5-benzyloxycarbonyl-2-(tert-butoxycarbonylamino)pentanoyl}amino]-4-(2-naphthyl) butanamide
ESI-MS : 590 [M+H]

Example 121

(3S)-3-[N-(n-Butyl)-{(2S)-5-benzyloxycarbonyl-2-(tert-butoxycarbonylamino)pentanoyl}amino]-4-(2-naphthyl)butanamide
ESI-MS : 618 [M+H]

Example 122

(3S)-3-[N-(n-Propyl)-{(2S)-5-benzyloxycarbonyl-2-(tert-butoxycarbonylamino)pentanoyl}amino]-4-(4-methylphenyl)-butanamide
ESI-MS : 568 [M+H]

Example 123

(3S)-3-[N-(n-Propyl)-{(2S)-5-benzyloxycarbonyl-2-(tert-butoxycarbonylamino)pentanoyl}amino]-4-(4-n-butylphenyl)-butanamide
ESI-MS : 610 [M+H]

Example 124

(3S)-3-[N-(n-Propyl)-{(2S)-4-benzyloxycarbonyl-2-(tert-butoxycarbonylamino)butanoyl}amino]-4-(2-naphthyl)butanamide
ESI-MS : 590 [M+H]

Example 125

(3S)-3-[N-(n-Butyl)-{(2S)-2-(tert-butoxycarbonyl)amino-5-methoxycarbonylpentanoyl}amino]-4-(4-n-heptylphenyl)-butanamide
ESI-MS : 590 [M+H]

Example 126

3-[N-Methyl-{(2S)-5-benzyloxycarbonyl-2-(tert-butoxycarbonylamino)pentanoyl}amino]propanamide
ESI-MS : 436 [M+H]

Example 127

(3S)-3-[N-(n-Pentyl)-{(2S)-5-benzyloxycarbonyl-2-(tert-butoxycarbonylamino)pentanoyl}amino]dodecanamide
ESI-MS : 618 [M+H]

Example 128

(3S)-3-[N-(n-Propyl)-{(2S)-2-(tert-butoxycarbonyl)amino-5-methoxycarbonylpentanoyl}amino]dodecanamide
ESI-MS : 514 [M+H]

Example 129

(3S)-3-[N-(n-Pentyl)-{(2S)-5-benzyloxycarbonyl-2-(tert-butoxycarbonylamino)pentanoyl}amino]nonanamide
ESI-MS : 576 [M+H]

Example 130

(3S)-3-[N-(n-Butyl)-{(2S)-5-benzyloxycarbonyl-2-(tert-butoxycarbonylamino)pentanoyl}amino]nonanamide
ESI-MS : 562 [M+H]

Example 131

(3S)-3-[N-(n-Propyl)-{(2S)-5-benzyloxycarbonyl-2-(tert-butoxycarbonylamino)pentanoyl}amino]nonanamide
ESI-MS : 548 [M+H]

Example 132

(3S)-3-[N-Ethyl-{(2S)-2-(tert-butoxycarbonyl)amino-5-methoxycarbonylpentanoyl}amino]nonanamide
ESI-MS : 458 [M+H]

Example 133

(3S)-3-[N-(n-Propyl)-{(2S)-5-benzyloxycarbonyl-2-(tert-butoxycarbonylamino)pentanoyl}amino]heptanamide
ESI-MS : 520 [M+H]

Example 134

(3S)-3-[N-(n-Butyl)-{(2S)-2-(tert-butoxycarbonyl)amino-5-methoxycarbonylpentanoyl}amino]heptanamide
ESI-MS : 458 [M+H]

Example 135

(3S)-3-[N-(n-Propyl)-{(2S)-5-benzyloxycarbonyl-2-(tert-butoxycarbonylamino)pentanoyl}amino]-4-(6-ethyl-2-naphthyl)-butanamide
ESI-MS : 632 [M+H]

Example 136

(3S)-3-[N-(n-Butyl)-{(2S)-5-benzyloxycarbonyl-2-(tert-butoxycarbonylamino)pentanoyl}amino]-4-(6-ethyl-2-naphthyl)-butanamide

EST-MS : 646 [M+H]

The following compounds (Examples 137 to 194) were obtained according to a similar manner to that of Example 21.

Example 137

(3S)-3-[N-(n-Propyl)-{(2S)-4-carboxy-2-[(1-(2-chlorobenzyl)indol-3-ylcarbonyl)amino]butanoyl}amino]-4-(2-naphthyl)butanamide

ESI-MS : 667 [M+H]

mp : 85-92°C

Example 138

(3S)-3-[N-(n-Butyl)-{(2S)-5-carboxy-2-[(1-(2-chlorobenzyl)indol-3-ylcarbonyl)amino]pentanoyl}amino]-4-(4-n-butylphenyl)butanamide

ESI-MS : 701 [M+H]

mp : 166-168°C

Example 139

(3S)-3-[N-(n-Butyl)-{(2S)-5-carboxy-2-[(1-benzylindol-3-ylcarbonyl)amino]pentanoyl}amino]-4-(4-n-butylphenyl)-butanamide

ESI-MS : 667 [M+H]

mp : 77-82°C

Example 140

(3S)-3-[N-(n-Propyl)-{(2S)-5-carboxy-2-[(1-(2-chlorobenzyl)indol-3-ylcarbonyl) amino]pentanoyl}amino]-4-(4-n-butylphenyl)butanamide

ESI-MS : 685 [M-H]

mp : 83-87°C

Example 141

(3S)-3-[N-(n-Propyl)-{(2S)-5-carboxy-2-[(1-benzylindol-3-ylcarbonyl)amino]pentanoyl}amino]-4-(4-n-butylphenyl)-butanamide

ESI-MS : 651 [M-H]

mp : 82-88°C

Example 142

(3S)-3-[N-(n-Propyl)-{(2S)-5-carboxy-2-[(1-(2-chlorobenzyl)indol-3-ylcarbonyl)amino]pentanoyl}amino]-4-(4-methylphenyl)butanamide

ESI-MS : 643 [M-H]

mp : 79-96°C

Example 143

(3S)-3-[N-(n-Propyl)-{(2S)-2-(1-benzylindol-3-ylcarbonyl)amino-5-carboxypentanoyl}amino]-4-(4-methylphenyl)butanamide

ESI-MS : 609 [M-H]

mp : 83-92°C

Example 144

(3S)-3-[N-(n-Butyl)-{(2S)-5-carboxy-2-[(1-(2-chlorobenzyl) indol-3-ylcarbonyl)amino]pentanoyl}amino]-4-(2-naphthyl)butanamide

ESI-MS : 693 [M-H]

mp : 104-110°C

Example 145

(3S)-3-[N-(n-Butyl)-{(2S)-5-carboxy-2-[(1-benzylindol-3-ylcarbonyl)amino]pentanoyl}amino]-4-(2-naphthyl) butanamide

ESI-MS : 659 [M-H]

mp : 90-96°C

Example 146

(3S)-3-[N-Ethyl-{(2S)-5-carboxy-2-[(1-(2-chlorobenzyl)indol-3-ylcarbonyl)amino]pentanoyl}amino]-4-(2-naphthyl)butanamide

ESI-MS : 665 [M-H]

mp : 110-114°C

Example 147

(3S)-3-[N-Ethyl-{(2S)-5-carboxy-2-[(1-(1-naphthylmethyl)indol-3-ylcarbonyl)amino]pentanoyl}amino]-4-(2-naphthyl)butanamide

ESI-MS : 681 [M-H]

mp : 118-126°C

Example 148

(3S)-3-[N-Isopentyl-{(2S)-5-carboxy-2-(2-quinolylcarbonylamino)pentanoyl}amino]hexadecanamide

ESI-MS : 639 [M+H]

Example 149

(3S)-3-[N-Isobutyl-{(2S)-5-carboxy-2-(2-quinolylcarbonylamino)pentanoyl}amino]hexadecanamide

ESI-MS : 625 [M+H]

mp : 65-66°C

Example 150

(3S)-3-[N-Phenethyl-{(2S)-5-carboxy-2-(2-quinolylcarbonylamino)pentanoyl}amino]hexadecanamide

ESI-MS : 673 [M+H]

Example 151

(3S)-3-[N-Ethyl-{(2S)-5-carboxy-2-(1-benzylindol-3-ylcarbonylamino)pentanoyl}amino]-4-(2-naphthyl)butanamide

ESI-MS : 631 [M-H]

mp : 110-112°C

Example 152

(3S)-3-[N-(n-Pentyl)-{(2S)-5-carboxy-2-[(1-benzylindol-3-ylcarbonyl)amino]pentanoyl}amino]-4-(2-naphthyl) butanamide

ESI-MS : 675 [M+H]

mp : 89-93°C

Example 153

(3S)-3-[N-(n-Pentyl)-{(2S)-5-carboxy-2-[(1-(1-naphthylmethyl)indol-3-ylcarbonyl)amino]pentanoyl}amino]-4-(2-naphthyl)butanamide

ESI-MS : 725 [M+H]

mp : 112-116°C

Example 154

(3S)-3-[N-(n-Propyl)-{(2S)-5-carboxy-2-[(1-(1-naphthylmethyl)indol-3-ylcarbonyl)amino]pentanoyl}amino]-4-(2-naphthyl)butanamide
ESI-MS : 697 [M+H]
mp : 123-126°C

Example 155

(3S)-3-[N-(n-Propyl)-{(2S)-2-(1-benzylindol-3-ylcarbonyl)amino-5-carboxypentanoyl}amino]-4-(2-naphthyl) butanamide
ESI-MS : 647 [M+H]
mp : 91-94°C

Example 156

(3S)-3-[N-(2-Pyridylmethyl)-{(2S)-5-carboxy-2-(2-quinolylcarbonylamino)pentanoyl}amino]hexadecanamide dihydrochloride
ESI-MS : 660 [M+H]
mp : 74-81°C

Example 157

(3S)-3-[N-Ethyl-{(2S)-5-carboxy-2-(2-quinolylcarbonylamino)pentanoyl}amino]hexadecanamide
ESI-MS : 660 [M+H]

Example 158

(3S)-3-[N-(n-Propyl)-{(2S)-5-carboxy-2-(2-quinolylcarbonylamino)pentanoyl}amino]hexadecanamide hydrochloride
mp : 59-62°C

Example 159

(3S)-3-[N-(n-Butyl)-{(2S)-5-carboxy-2-(2-quinolylcarbonylamino)pentanoyl}amino]hexadecanamide
EST-MS : 625 [M+H]

Example 160

(3S)-3-[N-(n-Pentyl)-{(2S)-5-carboxy-2-(2-quinolylcarbonylamino)pentanoyl}amino]hexadecanamide
ESI-MS : 639 [M+H]

Example 161

(3S)-3-[N-Benzyl-{(2S)-5-carboxy-2-(2-quinolylcarbonylamino)pentanoyl}amino]hexadecanamide
ESI-MS : 659 [M+H]

Example 162

(3S)-3-[N-(2-Pyridylmethyl)-{(2S)-5-carboxy-2-(2-quinolylcarbonylamino)pentanoyl}amino]hexadecanamide
ESI-MS : 660 [M+H]

Example 163

(3S)-3-[(2S)-5-Carboxy-2-[{1-(4-methylbenzyl)indol-3-ylcarbonyl}amino]pentanoyl]oxy-4-(2-naphthyl)butanamide

NMR (DMSO-$d_6$, δ) : 8.13-8.28 (3H, m), 7.70-7.86 (4H, m), 7.34-7.57 (5H, m),7.08-7.23 (6H, m), 6.85 (1H, br s), 5.42 (2H, s), 5.39 (1H, m), 4.40 (1H, m), 2.96-3.16 (2H, m), 2.29-2.38 (2H, m), 2.25 (3H, s), 2.11-2.20 (2H, m), 1.44-1.83 (4H, m)
ESI-MS : 620 [M+H]

Example 164

(3S)-3-[(2S)-5-Carboxy-2-[{1-(4-chlorobenzyl)indol-3-ylcarbonyl}amino]pentanoyl]oxy-4-(2-naphthyl)butanamide

NMR (DMSO-$d_6$, δ) : 8.12-8.28 (3H, m), 7.72-7.87 (4H, m), 7.32-7.57 (6H, m), 7.10-7.31 (4H, m), 6.85 (1H, br s), 5.49 (2H, s), 5.42 (1H, m), 4.42 (1H, m), 2.95-3.16 (2H, m), 2.29-2.38 (2H, m), 2.12-2.22 (2H, m), 1.45-1.84 (4H, m)
ESI-MS : 640 [M+H]

Example 165

(3S)-3-[(2S)-5-Carboxy-2-[{1-(4-methylbenzyl)indol-2-ylcarbonyl}amino]pentanoyl]oxy-4-(2-naphthyl)butanamide
ESI-MS : 620 [M+H]
mp : 86-90°C

Example 166

(3S)-3-[(2S)-5-Carboxy-2-(2-quinolylcarbonylamino)-pentanoyl]oxy-5-(2-naphthyl)pentanamide

NMR (DMSO-$d_6$, δ) : 9.12 (1H, d, J=8Hz), 8.60 (1H, d, J=8Hz), 8.22 (1H, t, J=8Hz), 8.20 (1H, d, J=9Hz), 8.12 (1H, d, J=8Hz), 7.90 (1H, t, J=8Hz), 7.68-7.86 (4H, m), 7.62 (1H, br s), 7.38-7.46 (3H, m), 6.88 (1H, br s), 5.20 (1H, m), 4.54 (1H, m), 2.70-2.86 (2H, m), 2.42-2.49 (2H, m), 2.27 (3H, t, J=7Hz), 1.87-2.04 (4H, m), 1.53-1.67 (2H, m)

Example 167

(3S)-3-[(2S)-2-(3-Benzylnaphthalen-2-ylcarbonyl)amino-5-carboxypentanoyl]oxy-4-(4-n-heptylphenyl)butanamide

NMR (DMSO-$d_6$, δ) : 8.82 (1H, d, J=7Hz), 7.95 (1H, m), 7.93 (1H, s), 7.85 (1H, m), 7.75 (1H, s), 7.47-7.59 (2H, m), 7.38 (1H, br s), 7.05-7.28 (9H, m), 6.86 (1H, br s), 5.32 (1H, m), 4.37 (1H, d, J=14Hz), 4.33 (1H, m), 4.29 (1H, d, J=14Hz), 2.78-2.96 (2H, m), 2.24-2.40 (2H, m), 2.12-2.21 (2H, m), 1.42-1.75 (6H, m), 1.13-1.35 (8H, m), 0.78-0.88 (3H, m)
ESI-MS : 665 [M+H]

Example 168

(3S)-3-[(2S)-5-Carboxy-2-[2-((2E)-2-methoxycarbonylvinyl)benzoylamino]pentanoyl]oxy-4-(2-naphthyl)butanamide

NMR (DMSO-$d_6$, δ) : 8.92 (1H, d, J=7Hz), 7.77-7.98 (6H, m), 7.42-7.57 (6H, m), 7.37 (1H, br s), 6.88 (1H, br s), 6.59 (1H, d, J=16Hz), 5.43 (1H, m), 4.23 (1H, m), 3.66 (3H, s), 3.16 (1H, dd, J=14, 6Hz), 3.06 (1H, dd, J=14, 6Hz), 2.37 (2H, d, J=7Hz), 2.1 (3H, t, J=7Hz), 1.42-1.75 (4H, m)
ESI-MS : 561 [M+H]

Example 169

(3S)-3-[(2S)-5-Carboxy-2-[2-(carboxymethyl)-benzoylamino]pentanoyl]oxy-4-(2-naphthyl)butanamide

NMR (DMSO-$d_6$, δ) : 8.75 (1H, d, J=7Hz), 7.81-7.92 (3H, m), 7.77 (1H, s), 7.22-7.54 (8H, m), 6.86 (1H, br s), 5.42 (1H, m), 4.30 (1H, m), 3.83 (1H, d, J=16Hz), 3.71 (1H, d, J=16Hz), 3.13 (1H, dd, J=14, 5Hz), 3.03 (1H, dd, J=14, 6Hz), 2.36 (2H, d, J=7Hz), 2.13 (3H, t, J=7Hz), 1.43-1.74 (4H, m)
ESI-MS : 535 [M+H]

Example 170

(3S)-3-[(2S)-5-Carboxy-2-[2-(methoxycarbonylmethyl)-benzoylamino]pentanoyl]oxy-4-(2-naphthyl)butanamide

NMR (DMSO-$d_6$, δ) : 8.71 (1H, d, J=7Hz), 7.75-7.92 (4H, m), 7.28-7.54 (8H, m), 6.87 (1H, br s), 5.42 (1H, m), 4.28 (1H, m), 3.91 (1H, d, J=16Hz), 3.81 (1H, d, J=16Hz), 3.53 (3H, s), 3.13 (1H, dd, J=14, 5Hz), 3.04 (1H, dd, J=14, 6Hz), 2.36 (2H, d, J=7Hz), 2.14 (3H, t, J=7Hz), 1.42-1.74 (4H, m)
ESI-MS : 549 [M+H]

Example 171

(3S)-3-[(2S)-5-Carboxy-2-(3-quinolylcarbonylamino)-pentanoyl]oxy-4-(2-naphthyl)butanamide

NMR ($CDCl_3$-$CD_3OD$, δ) : 9.36 (1H, s), 8.72 (1H, s), 8.15 (1H, d, J=15Hz), 7.94 (1H, d, J=15Hz), 7.6-7.9 (6H, m), 7.3-7.45 (3H, m), 5.6 (1H, m), 4.62 (1H, m), 3.15 (2H, d, J=7Hz), 2.4-2.6 (2H, m), 2.1-2.4 (2H, m), 1.75-1.9 (2H, m), 1.5-1.6 (2H, m)

Example 172

(3S)-3-[(2S)-5-Carboxy-2-(isoquinolin-3-ylcarbonylamino)pentanoyl]oxy-4-(2-naphthyl)butanamide

NMR ($CDCl_3$, δ) : 9.1 (1H, s), 8.7 (1H, d, J=15Hz), 8.50 (1H, s), 7.9-8.05 (2H, m), 7.6-7.8 (5H, m), 7.25-7.4 (3H, m), 6.9 (1H, br s), 6.3 (1H, br s), 5.6 (1H, m), 4.8 (1H, m), 3.0-3.3 (2H, m), 2.4-2.6 (2H, m), 2.1-2.4 (2H, m), 1.75-1.9 (2H, m), 1.5-1.6 (2H, m)
FAB-MS : 528 [M+H]

Example 173

(3S)-3-[(2S)-5-Carboxy-2-(isoquinolin-1-ylcarbonylamino)pentanoy]oxy-4-(2-naphthyl)butanamide

NMR ($CDCl_3$-$CD_3OD$, δ) : 9.45 (1H, d, J=10Hz), 8.72 (1H, d, J=10Hz), 8.4 (1H, d, J=7Hz), 7.6-7.9 (11H, m), 7.0 (1H, br s), 6.4 (1H, br s), 5.6 (1H, m), 4.7 (1H, m), 3.0-3.3 (2H, m), 2.4-2.6 (2H, m), 2.1-2.4 (2H, m), 1.75-1.9 (2H, m), 1.5-1.6 (2H, m)
FAB-MS : 528 [M+H]

Example 174

(3S)-3-[(2S)-2-(2-Benzylbenzoyl)amino-5-carboxypentanoyl]oxy-4-(2-naphthyl)butanamide

NMR (DMSO-$d_6$, δ) : 8.75 (1H, d, J=10Hz), 7.1-7.9 (17H, m), 6.88 (1H, br s), 5.4 (1H, m), 4.32 (1H, m), 4.1 (2H, ABq), 2.95-3.15 (2H, m), 2.32 (2H, d, J=7Hz), 2.1 (2H, m), 1.4-1.7 (4H, m)
ESI-MS : 567 [M+H]

Example 175

(3S)-3-[(2S)-5-Carboxy-2-(2-naphthylcarbonylamino)-pentanoyl]oxy-4-(2-naphthyl)butanamide

NMR (DMSO-$d_6$, δ) : 8.9 (1H, d, J=10Hz), 8.52 (1H, s), 7.35-8.1 (14H, m), 6.9 (1H, br s), 5.45 (1H, m), 4.44 (1H, m), 3.0-3.2 (2H, m), 2.35 (2H, d, J=7Hz), 2.2 (2H, t, J=7Hz), 1.7-1.85 (2H, m), 1.5-1.7 (2H, m)
ESI-MS : 527 [M+H]

Example 176

(3S)-3-[(2S)-2-Benzoylamino-5-carboxypentanoyl]oxy-4-(2-naphthyl)butanamide

NMR (DMSO-$d_6$, δ) : 8.74 (1H, d, J=10Hz), 7.3-8.0 (13H, m), 6.88 (1H, br s), 5.4 (1H, m), 4.4 (1H, m), 3.0-3.2 (2H, m), 2.32 (2H, d, J=7Hz), 2.18 (2H, t, J=7Hz), 1.4-1.8 (4H, m)

ESI-MS : 477 [M+H]

Example 177

(3S)-3-[(2S)-5-Carboxy-2-(2-phenethylbenzoylamino)-pentanoyl]oxy-4-(2-naphthyl)butanamide

NMR ($CDCl_3$, δ) : 8.8 (1H, d, J=10Hz), 7.1-7.9 (17H, m), 6.85 (1H, br s), 5.44 (1H, m), 4.36 (1H, m), 2.8-3.2 (6H, m), 2.35 (2H, d, J=7Hz), 2.18 (2H, t, J=7Hz), 1.45-1.8 (4H, m)
ESI-MS : 581 [M+H]

Example 178

(3S)-3-[(2S)-2-(3-Benzylbenzoyl)amino-5-carboxypentanoyl]oxy-4-(2-naphthyl)butanamide

NMR (DMSO-$d_6$, δ) : 8.75 (1H, d, J=10Hz), 7.1-7.9 (17H, m), 6.88 (1H, br s), 5.45 (1H, m), 4.4 (1H, m), 4.04 (2H, s), 2.95-3.15 (2H, m), 2.32 (2H, d, J=7Hz), 2.2 (2H, t, J=7Hz), 1.4-1.7 (4H, m)
ESI-MS : 567 [M+H]

Example 179

(3S)-3-[(2S)-2-Benzylnaphthalen-2-ylcarbonyl)amino-5-carboxypentanoyl]oxy-4-(6-ethyl-2-naphthyl)butanamide

NMR (DMSO-$d_6$, δ) : 8.82 (1H, d, J=10Hz), 7.1-7.9 (18H, m), 8.86 (1H, br s), 5.4 (1H, m), 4.32 (1H, m), 4.26 (2H, ABq), 3.0-3.15 (2H, m), 2.72 (2H, q, J=7Hz), 2.36 (2H, d, J=7Hz), 2.12 (2H, t, J=7Hz), 1.4-1.7 (4H, m), 1.22 (3H, t, J=7Hz)
ESI-MS : 645 [M+H]

Example 180

3-[N-Methyl-{(2S)-2-(3-benzylnaphthalen-2-ylcarbonyl)amino-5-carboxypentanoyl}amino]propanamide
ESI-MS : 488 [M-H]
mp : 147-157°C

Example 181

(3S)-3-[N-(n-Pentyl)-{(2S)-2-(1-benzylindol-3-ylcarbonyl)amino-5-carboxypentanoyl}amino]dodecanamide
ESI-MS : 661 [M+H]
mp : 125-127°C

Example 182

(3S)-3-[N-(n-Pentyl)-{(2S)-2-(1-benzylindol-3-ylcarbonyl)amino-5-carboxypentanoyl}amino]nonanamide
ESI-MS : 619 [M+H]
mp : 127-129°C

Example 183

(3S)-3-[N-(n-Butyl)-{(2S)-5-carboxy-2-[(1-benzylindol-3-ylcarbonyl)amino]pentanoyl}amino]nonanamide
ESI-MS : 605 [M+H]
mp : 124-127°C

Example 184

(3S)-3-[N-(n-Butyl)-{(2S)-5-carboxy-2-[(1-(2-chlorobenzyl)indol-3-ylcarbonyl)amino]pentanoyl}amino]nonanamide
ESI-MS : 639 [M+H]
mp : 153-156°C

Example 185

(3S)-3-[N-(n-Propyl)-{(2S)-5-carboxy-2-[(1-(3-chlorobenzyl)indol-3-ylcarbonyl)amino]pentanoyl}amino]nonanamide

ESI-MS : 625 [M+H]
mp : 106-109°C

Example 186

(3S)-3-[N-(n-Propyl)-{(2S)-5-carboxy-2-[(1-(1-naphthylmethyl)indol-3-ylcarbonyl)amino]pentanoyl}amino] nonanamide

ESI-MS : 641 [M+H]
mp : 139-141°C

Example 187

(3S)-3-[N-(n-Propyl)-{(2S)-5-carboxy-2-[(1-(2-pyridylmethyl)indol-3-ylcarbonyl)amino]pentanoyl}amino]nonanamide

ESI-MS : 592 [M+H]
mp : 78-95°C

Example 188

(3S)-3-[N-(n-Propyl)-{(2S)-5-carboxy-2-[(1-(2-chlorobenzyl)indol-3-ylcarbonyl]amino]pentanoyl}amino]nonanamide

ESI-MS : 625 [M+H]
mp : 175-180°C

Example 189

(3S)-3-[N-(n-Propyl)-{(2S)-2-(1-(2-chlorobenzyl)indol-3-ylcarbonyl)amino-5-carboxypentanoyl}amino]heptanamide

ESI-MS : 597 [M+H]
mp : 95-98°C

Example 190

(3S)-3-[N-(n-Propyl)-{(2S)-2-(1-(1-naphthylmethyl)indol-3-ylcarbonyl)amino-5-carboxypentanoyl}amino] heptanamide

ESI-MS : 613 [M+H]
mp : 98-116°C

Example 191

(3S)-3-[N-(n-Propyl)-{(2S)-5-carboxy-2-[(1-(2-pyridylmethyl)indol-3-ylcarbonyl)amino]pentanoyl}amino]-4-(6-ethyl-2-naphthyl)butanamide

ESI-MS : 676 [M+H]
mp : 110-116°C

Example 192

(3S)-3-[N-(n-Propyl)-{(2S)-2-(1-benzylindol-3-ylcarbonyl)amino-5-carboxypentanoyl}amino]-4-(6-ethyl-2-naphthyl)butanamide

ESI-MS : 673 [M-H]
mp : 105-116°C

Example 193

(3S)-3-[N-(n-Butyl)-{(2S)-2-(1-benzylindol-3-ylcarbonyl)amino-5-carboxypentanoyl}amino]-4-(6-ethyl-2-naphthyl)butanamide

ESI-MS : 689 [M+H]

mp : 100-116°C

Example 194

(3S)-3-[N-(n-Butyl)-{(2S)-2-(1-(2-chlorobenzyl)indol-3-ylcarbonyl)amino-5-carboxypentanoyl}amino]-4-(6-ethyl-2-naphthyl)butanamide

ESI-MS : 723 [M+H]

mp : 100-116°C

The following compounds (Examples 195 to 199) were obtained according to a similar manner to that of Example 33.

Example 195

(3S)-3-[(2S)-5-Carboxy-2-(tert-butoxycarbonylamino)-pentanoyl]oxy-5-(n-decyloxy)pentananide

NMR ($CDCl_3$, δ) : 6.90 (1H, br s), 6.24 (1H, br s), 5.40 (1H, m), 5.20 (1H, d, J=8Hz), 4.27 (1H, m), 3.32-3.57 (4H, m), 2.40-2.72 (2H, m), 2.30-2.40 (2H, m), 1.48-2.03 (8H, m), 1.44 (9H, s), 1.16-1.35 (14H, m), 0.87 (3H, t, J=7Hz)

Example 196

(3S)-3-[(2S)-5-Carboxy-2-(tert-butoxycarbonylamino)-pentanoyl]oxy-6-(n-nonyloxy)hexanamide

NMR ($CDCl_3$, δ) : 6.77 (1H, br s), 6.15 (1H, br s), 5.33 (1H, m), 5.22 (1H, d, J=8Hz), 4.26 (1H, m), 3.32-3.46 (4H, m), 2.45-2.56 (2H, m), 2.27-2.40 (2H, m), 1.47-1.93 (8H, m), 1.44 (9H, s), 1.16-1.36 (14H, m), 0.88 (3H, t, J=7Hz)

Example 197

(3S)-3-[(2S)-5-Carboxy-2-(3-quinolylcarbonylamino)-pentanoyl]oxy-10-phenyldecanamide

NMR ($CDCl_3$, δ) : 9.34 (1H, s), 8.62 (1H, s), 7.55-8.25 (4H, m), 7.1-7.4 (11H, m), 6.85 (1H, br s), 6.40 (1H, br s), 5.35 (1H, m), 4.80 (1H, m), 2.4-2.7 (6H, m), 1.5-2.15 (8H, m), 1.2-1.4 (8H, m)

FAB-MS : 562 [M+H]

Example 198

(3S)-3-[(2S)-(tert-Butoxycarbonyl)amino-5-carboxypentanoyl]oxy-10-phenyldecanamide

NMR ($CDCl_3$, δ) : 7.1-7.3 (6H, m), 6.20 (1H, br s), 5.26 (1H, m), 5.20 (1H, d, J=15.0Hz), 4.28 (1H, m), 2.3-2.65 (6H, m), 1.5-1.9 (8H, m), 1.45 (9H, s), 1.2-1.4 (8H, m)

Example 199

(3S)-3-[(2S)-2-(tert-Butoxycarbonyl)amino-5-carboxypentanoyl]oxy-4-(4-biphenylyl)butanamide

NMR (DMSO-$d_6$, δ) : 7.3-7.7 (10H, m), 7.22 (1H, d, J=15.0Hz), 6.84 (1H, br s), 5.32 (1H, m), 3.88 (1H, m), 2.8-3.0 (2H, m), 2.3 (2H, m), 2.14 (2H, m), 1.3-1.7 (13H, m)

FAB-MS : 499 [M+H]

Example 200

To a stirring solution of (3S)-3-[N-(n-propyl)-{(2S)-2-(1-benzylindol-3-ylcarbonyl)amino-5-methoxycarbonylpentanoyl}amino]dodecanamide (0.23 g) in methanol (4.5 ml) was added 1N sodium hydroxide (0.71 ml) at room temperature and allowed to stand overnight. The mixture was diluted with 1N hydrochloric acid (2 ml) and concentrated

under reduced pressure. The residue was extracted with ethyl acetate and the organic layer was washed with water and brine. The organic layer was dried with magnesium sulfate and concentrated in vacuo. The residue was triturated with ethyl ether to give (3S)-3-[N-(n-propyl)-{(2S)-2-(1-benzylindol-3-ylcarbonyl)amino-5-carboxypentanoyl}amino]dodecanamide (114 mg).

ESI-MS : 633 [M+H]

mp : 155-160°C

The following compounds (Examples 201 to 207) were obtained according to a similar manner to that of Example 200.

Example 201

(3S)-3-[N-(n-Propyl)-{(2S)-2-(1-(2-chlorobenzyl)indol-3-ylcarbonyl)amino-5-carboxypentanoyl}amino]dodecanamide

ESI-MS : 667 [M+H]

mp : 145-150°C

Example 202

(3S)-3-[N-(n-Butyl)-{(2S)-5-carboxy-2-[(1-(2-chlorobenzyl)indol-3-ylcarbonyl)amino]pentanoyl}amino]-4-(4-n-heptylphenyl)butanamide

ESI-MS : 743 [M+H]

mp : 79-81°C

Example 203

(3S)-3-[N-(n-Butyl)-{(2S)-5-carboxy-2-(2-quinolylcarbonylamino)pentanoyl}amino]-4-(4-n-heptylphenyl)butanamide

ESI-MS : 631 [M+H]

Example 204

(3S)-3-[N-Ethyl-{(2S)-2-(1-(2-chlorobenzyl)indol-3-ylcarbonyl)amino-5-carboxypentanoyl}amino]nonanamide

ESI-MS : 611 [M+H]

mp : 180-183°C

Example 205

(3S)-3-[N-Ethyl-{(2S)-2-(1-benzylindol-3-ylcarbonyl)amino-5-carboxypentanoyl}amino]nonanamide

ESI-MS : 577 [M+H]

mp : 80-85°C

Example 206

(3S)-3-[N-(n-Butyl)-{(2S)-2-(1-(2-chlorobenzyl)indol-3-ylcarbonyl)amino-5-carboxypentanoyl}amino]heptanamide

ESI-MS : 611 [M+H]

mp : 105-110°C

Example 207

(3S)-3-[N-(n-Butyl)-{(2S)-2-(1-(1-naphthylmethyl)indol-3-ylcarbonyl)amino-5-carboxypentanoyl}amino]heptanamide

ESI-MS : 627 [M+H]

mp : 105-113°C

Example 208

(3S)-3-[N-Ethyl-{(2S)-5-carboxy-2-(2-quinolylcarbonylamino)pentanoyl}amino]hexadecanamide (300 mg) was dissolved in 4N hydrogen chloride in ethyl acetate (2 ml) at room temperature. After being stirred at the same temperature for 10 minutes, the solvent was removed under reduced pressure and the resulting solid was triturated with ethyl acetate to give (3S)-3-[N-ethyl-{(2S)-5-carboxy-2-(2-quinolylcarbonylamino)pentanoyl}amino]hexadecanamide hydrochloride (194 mg).

Example 209

(3S)-3-[N-(n-Butyl)-{(2S)-5-carboxy-2-(2-quinolylcarbonylamino)pentanoyl}amino]hexadecanamide hydrochloride was obtained according to a similar manner to that of Example 208.

Example 210

(3S)-3-[N-(n-Propyl)-{(2S)-2-(1-benzylindol-3-ylcarbonyl)amino-5-carboxypentanoyl}amino]nonanamide was obtained according to a similar manner to that of Example 21.

ESI-MS : 591 [M+H]

mp : 147-157°C

**Claims**

**1.** A fatty acid derivative represented by the following formula :

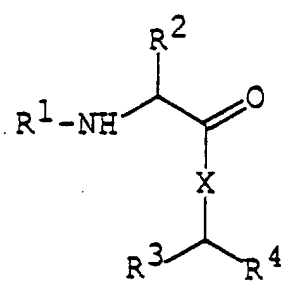

(I)

wherein

$R^1$ is $(C_1-C_4)$alkoxycarbonyl;
phenyl$(C_1-C_4)$alkanoyl or naphthyl$(C_1-C_4)$alkanoyl, each of which may have carboxy$(C_1-C_4)$alkyl, $(C_1-C_4)$alkoxycarbonyl$(C_1-C_4)$alkyl which may be substituted by phenyl, $(C_1-C_4)$alkoxycarbonyl$(C_2-C_4)$alkenyl, carbamoyl$(C_1-C_4)$alkyl or phenyl$(C_1-C_4)$alkyl;
heterocyclic$(C_1-C_4)$alkanoyl which may have pyridyl$(C_1-C_4)$alkyl, naphthyl$(C_1-C_4)$alkyl or phenyl$(C_1-C_4)$alkyl which may have 1 to 3 suitable substituent(s) selected from the group consisting of $(C_1-C_4)$alkyl and halogen, in which the heterocyclic moiety is indolyl, quinolyl or isoquinolyl,

$R^2$ is carboxy$(C_1-C_4)$alkyl, methoxycarbonyl$(C_1-C_4)$alkyl, or benzyloxycarbonyl$(C_1-C_4)$alkyl,

$R^3$ is phenyl$(C_1-C_4)$alkyl which may have $(C_1-C_4)$alkyl, $(C_7-C_{16})$alkyl or phenyl;
naphthyl$(C_1-C_4)$alkyl which may have $(C_1-C_4)$alkyl;
phenyl$(C_7-C_{16})$alkyl;
benzofuranyl$(C_1-C_4)$alkyl;
$(C_7-C_{16})$alkoxy$(C_1-C_4)$alkyl;
$(C_3-C_6)$alkyl; or
$(C_7-C_{16})$alkyl,

$R^4$ is carbamoyl($C_1$-$C_4$)alkyl, and

X is -O-, -NH- or

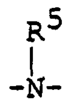

[wherein $R^5$ is ($C_1$-$C_5$)alkyl, phenyl($C_1$-$C_4$)alkyl, or pyridyl($C_1$-$C_4$)alkyl], with proviso that X is

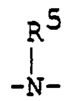

(wherein $R^5$ is as defined above), when $R^3$ is ($C_3$-$C_6$)alkyl or ($C_7$-$C_{16}$)alkyl, and a pharmaceutically acceptable salt thereof.

**2.** A compound of claim 1 wherein

$R^1$ is indolyl($C_1$-$C_4$) alkanoyl which may have a suitable substituent selected from the group consisting of pyridyl ($C_1$- $C_4$) alkyl, naphthyl ($C_1$-$C_4$) alkyl, phenyl ($C_1$ - $C_4$)alkyl, ($C_1$ - $C_4$)alkylphenyl ($C_1$ - $C_4$)alkyl, and halophenyl ($C_1$ - $C_4$)alkyl,
$R^2$ is carboxy ($C_1$ - $C_4$)alkyl,
$R^3$ is ($C_3$ - $C_6$) alkyl or ($C_7$ - $C_{16}$)alkyl,
$R^4$ is carbamoyl ($C_1$ - $C_4$)alkyl, and
X is

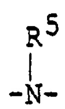

[wherein $R^5$ is ($C_1$-$C_5$)alkyl].

**3.** A compound of claim 2 which is selected from the group consisting of

(1) (3S)-3-[N-(n-Propyl)-{(2S)-5-carboxy-2-[(1-(2-chlorobenzyl) indol-3-ylcarbonyl)amino]pentanoyl}amino] nonanamide,
(2) (3S)-3-[N-(n-Propyl)-{(2S)-2-(1-(2-chlorobenzyl)indol-3-ylcarbonyl) amino-5-carboxypentanoyl}amino] heptanamide,
(3) (3S)-3-[N-(n-Propyl)-{(2S)-2-(1-benzylindol-3-ylcarbonyl)amino-5-carboxypentanoyl}amino]-dodecanamide,
(4) (3S)-3-[N-(n-Propyl)-{(2S)-2-(1-(2-chlorobenzyl)indol-3-ylcarbonyl)amino-5-carboxypentanoyl}amino]dodecanamide,
(5) (3S)-3-[N-Ethyl-{(2S)-2-(1-(2-chlorobenzyl)indol-3-ylcarbonyl)amino-5-carboxypentanoyl}amino]-nonanamide,
(6) (3S)-3-[N-Ethyl-{(2S)-2-(1-benzylindol-3-ylcarbonyl)amino-5-carboxypentanoyl}amino]-nonanamide,
(7) (3S)-3-[N-(n-Butyl)-{(2S)-2-(1-(1-naphthylmethyl)-indol-3-ylcarbonyl)amino-5-carboxypentanoyl}amino]-heptanamide, and
(8) (3S)-3-[N-(n-Propyl)-{(2S)-2-(1-benzylindol-3-ylcarbonyl)amino-5-carboxypentanoyl}amino]-nonanamide,

or a pharmaceutically acceptable salt thereof.

**4.** A process for preparing a compound of the formula :

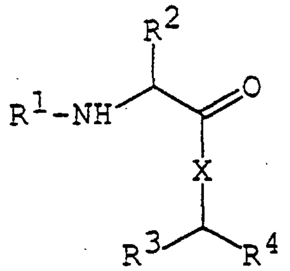

(I)

wherein
$R^1$, $R^2$, $R^3$, $R^4$ and X are each as defined in claim 1, or a salt thereof, which comprises

1) reacting the compound of the formula :

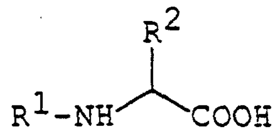

wherein $R^1$ and $R^2$ are each as defined above, or a reactive derivative at the carboxy group or a salt thereof, with the compound of the formula :

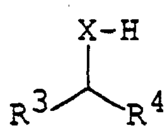

wherein $R^3$, $R^4$ and X are each as defined above, or a salt thereof, or

2) reacting the compound of the formula :

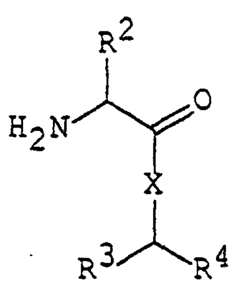

wherein $R^2$, $R^3$, $R^4$ and X are each as defined above, or a reactive derivative at the amino group or a salt thereof, with the compound of the formula :

$R^1$ - OH

wherein $R^1$ is as defined above,
or a reactive derivative or a salt thereof, or

3) subjecting the compound of the formula :

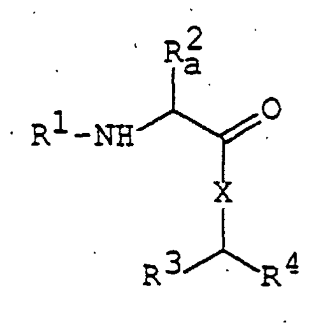

wherein

| | |
|---|---|
| $R^1$, $R^3$, $R^4$ and X | are each as defined above, and |
| $R^2_a$ | is protected carboxy ($C_1$-$C_4$) alkyl, |

or a salt thereof, to elimination reaction of carboxy protective group, to give the compound of the formula :

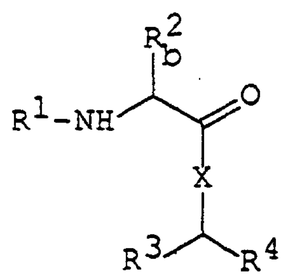

wherein

| | |
|---|---|
| $R^1$, $R^3$, $R^4$ and X | are each as defined above, and |
| $R^2_b$ | is carboxy ($C_1$-$C_4$) alkyl, |

or a salt thereof.

**5.** A pharmaceutical composition which comprises, as an active ingredient, a fatty acid derivative of claim 1 or a pharmaceutically acceptable salt thereof in admixture with pharmaceutically acceptable carriers or excipients.

**6.** Use of a fatty acid derivative of claim 1 or a pharmaceutically acceptable salt thereof for the manufacture of a medicament.

**7.** A fatty acid derivative of claim 1 or a pharmaceutically acceptable salt thereof for use as a medicament.

**8.** Use of a fatty acid derivative or a pharmaceutically acceptable salt thereof according to claim 1 for the manufacture of a medicament for the prevention and/or the treatment of pancreatitis, hepatitis, chronic renal failure, shock, arthritis, respiratory disease, heart disease, allergic disease, thrombosis, arteriosclerosis, pain, autoimmune disease, dermal disease, inflammatory bowel disease, ophthalmic disease, nasal diseases, gout, trauma induced inflammation or liver diseases in a human being or an animal.

**Patentansprüche**

**1.** Durch die folgende Formel dargestelltes Fettsäure-Derivat;

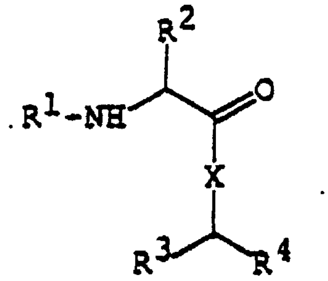

wobei $R^1$ $(C_1-C_4)$Alkoxycarbonyl; Phenyl$(C_1-C_4)$alkanoyl oder Naphthyl$(C_1-C_4)$alkanoyl, wobei jeder Carboxy$(C_1-C_4)$alkyl, $(C_1-C_4)$Alkoxycarbonyl$(C_1-C_4)$alkyl, der durch Phenyl substituiert sein kann, $(C_1-C_4)$Alkoxycarbonyl$(C_2-C_4)$alkenyl, Carbamoyl$(C_1-C_4)$alkyl oder Phenyl$(C_1-C_4)$alkyl aufweisen kann; Heterocyclo$(C_1-C_4)$alkanoyl, der Pyridyl$(C_1-C_4)$alkyl, Naphthyl$(C_1-C_4)$alkyl oder Phenyl$(C_1-C_4)$alkyl aufweisen kann, der 1 bis 3 geeignete Substituent(en) haben kann, die ausgewählt werden aus der aus $(C_1-C_4)$Alkyl und Halogen bestehenden Gruppe, wobei die heterocyclische Einheit Indolyl, Chinolyl oder Isochinolyl ist, ist

$R^2$ Carboxy$(C_1-C_4)$alkyl, Methoxycarbonyl$(C_1-C_4)$alkyl, oder Benzyloxycarbonyl$(C_1-C_4)$alkyl ist.

$R^3$ Phenyl$(C_1-C_4)$alkyl, der $(C_1-C_4)$Alkyl, $(C_7-C_{16})$Alkyl oder Phenyl aufweisen kann;
Naphthyl$(C_1-C_4)$alkyl, der $(C_1-C_4)$Alkyl aufweisen kann;
Phenyl$(C_7-C_{16})$alkyl;
Benzofuranyl$(C_1-C_4)$alkyl;
$(C_7-C_{16})$Alkoxy$(C_1-C_4)$alkyl;
$(C_3-C_6)$Alkyl; oder
$(C_7-C_{16})$Alkyl ist

$R^4$ Carbamoyl$(C_1-C_4)$alkyl ist, und

X -O-, -NH- oder

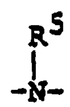

ist
[wobei $R^5$ $(C_1-C_5)$Alkyl, Phenyl$(C_1-C_4)$alkyl oder Pyridyl$(C_1-C_4)$alkyl ist],
unter der Voraussetzung, dass X

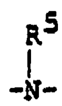

ist (wobei $R^5$ wie oben definiert ist), wenn $R^3$ $(C_3-C_6)$Alkyl oder $(C_7-C_{16})$Alkyl ist, und ein pharmazeutisch verträgliches Salz davon.

**2.** Verbindung nach Anspruch 1, wobei

$R^1$ Indolyl($C_1$-$C_4$)alkanoyl ist, der einen geeigneten Substituenten aufweisen kann, der ausgewählt wird aus der aus Pyridyl($C_1$-$C_4$)alkyl, Naphthyl($C_1$-$C_4$)alkyl, Phenyl($C_1$-$C_4$)alkyl, ($C_1$-$C_4$)Alkylphenyl($C_1$-$C_4$)alkyl, und Halophenyl($C_1$-$C_4$)alkyl bestehenden Gruppe,

$R^2$ Carboxy($C_1$-$C_4$)alkyl ist,

$R^3$ ($C_3$-$C_6$)Alkyl oder ($C_7$-$C_{16}$)Alkyl ist,

$R^4$ Carbamoyl($C_1$-$C_4$)alkyl ist, und

X

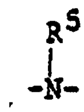

ist, [wobei $R^5$ ($C_1$-$C_5$)Alkyl ist].

**3.** Verbindung nach Anspruch 2, welche ausgewählt wird aus der Gruppe, die besteht aus

(1) (3S)-3-[N-(n-Propyl)-{(2S)-5-carboxy-2-[(1-(2-chlorbenzyl)indol-3-ylcarbonyl)-amino]pentanoyl}amino]nonanamid,

(2) (3S)-3-[N-(n-Propyl)-{(2S)-2-(1-(2-chlorbenzyl)indol-3-ylcarbonyl)amino-5-carboxypentanoyl}amino]heptanamid,

(3) (3S)-3-[N-(n-Propyl)-{(2S)-2-(1-benzylindol-3-ylcarbonyl)amino-5-carboxypentanoyl}amino]dodecanamid,

(4) (3S)-3-[N-(n-Propyl)-{(2S)-2-(1-(2-chlorbenzyl)indol-3-ylcarbonyl)amino-5-carboxypentanoyl}amino]dodecanamid,

(5) (3S)-3-[N-Ethyl-{(2S)-2-(1-(2-chlorbenzyl)indol-3-ylcarbonyl)amino-5-carboxypentanoyl}amino]nonanamid,

(6) (3S)-3-[N-Ethyl-{(2S)-2-(1-benzylindol-3-ylcarbonyl)amino-5-carboxypentanoyl}amino]-nonanamid,

(7) (3S)-3-[N-(n-Butyl)-{(2S)-2-(1-(1-naphthylmethyl)indol-3-ylcarbonyl)amino-5-carbaxypentanoyl}amino]heptanamid, und

(8) (3S)-3-[N-(n-Propyl)-{(2S)-2-( 1-benzylindol-3-ylcarbonyl)amino-5-carboxypentanoyl}amino]nonanamid,

oder ein pharmazeutisch verträgliches Salz davon.

**4.** Verfahren zur Herstellung einer Verbindung der Formel

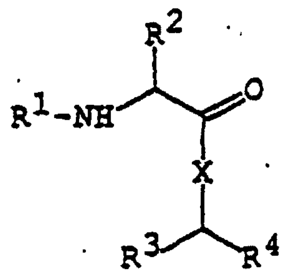

(I)

wobei $R^1$, $R^2$, $R^3$, $R^4$ und X jeweils wie in Anspruch 1 definiert sind, oder eines Salzes davon, welches umfasst

1) Umsetzung der Verbindung der Formel:

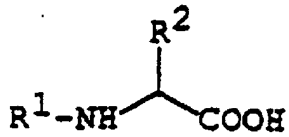

wobei $R^1$ und $R^2$ jeweils wie oben definiert sind, oder eines reaktiven Derivates an der Carboxygruppe oder eines Salzes davon, mit der Verbindung der Formel:

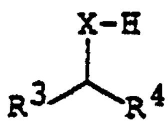

wobei $R^3$, $R^4$ und X jeweils wie oben definiert sind, oder einem Salz davon, oder

2) Umsetzung der Verbindung der Formel:

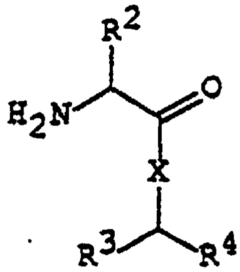

wobei $R^2$, $R^3$, $R^4$ und X jeweils wie oben definiert sind, oder eines reaktiven Derivates an der Aminogruppe oder eines Salzes davon, mit der Verbindung der Formel:

$$R^1 - OH$$

wobei $R^1$ wie oben definiert ist, oder eines reaktiven Derivates oder eines Salzes davon, oder

3) Durchführung einer Eliminierungsreaktion der Carboxyschutzgruppe mit einer Verbindung der Formel:

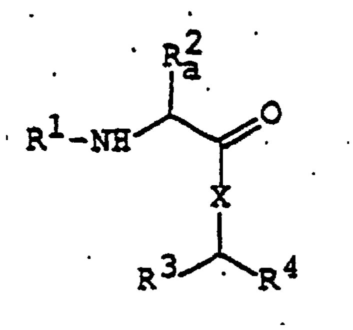

wobei $R^1$, $R^3$, $R^4$ und X jewells wie oben definiert sind, und
$R^2_a$ geschütztes Carboxy($C_1$-$C_4$)alkyl ist, oder mit einem Salz davon, zur Herstellung einer Verbindung der Formel:

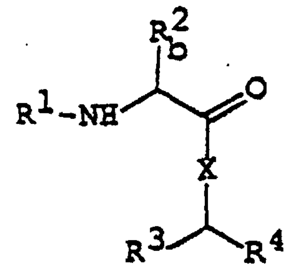

wobei $R^1$, $R^3$, $R^4$ und X jeweils wie oben definiert sind, und
$R^2_b$ Carboxy($C_1$-$C_4$)alkyl ist oder eines Salzes davon.

**5.** Pharmazeutische Zusammensetzung, welche als aktiven Bestandteil ein Fettsäure-Derivat nach Anspruch 1 oder ein pharmazeutisch verträgliches Salz davon im Gemisch mit pharmazeutisch verträglichen Trägern oder Exzipienten umfasst.

**6.** Verwendung eines Fettsäure-Derivates nach Anspruch 1 oder eines pharmazeutisch verträglichen Salzes davon zur Herstellung eines Medikaments.

**7.** Fettsäure-Derivat nach Anspruch 1 oder pharmazeutisch verträgliches Salz davon zur Verwendung als Medikament.

**8.** Verwendung eines Fettsäure-Derivates oder eines pharmazeutisch verträglichen Salzes davon nach Anspruch 1 zur Herstellung eines Medikaments zur Vorbeugung und/oder Behandlung von Pankreatitis, Hepatitis, chronischem Nierenversagen, Schock, Arthritis, Atemwegserkrankungen, Herzerkrankungen, allergischen Erkrankungen, Thrombose, Arteriosklerose, Schmerzen, Autoimmunerkrankungen, Hauterkrankungen, entzündlichen Darmerkrankungen, ophthalmischen Erkrankungen, Nasenerkrankungen, Gicht, traumainduzierten Entzündungen oder Lebererkrankungen beim Menschen oder Tier.

## Revendications

**1.** Dérivé d'acide gras représenté par la formule suivante :

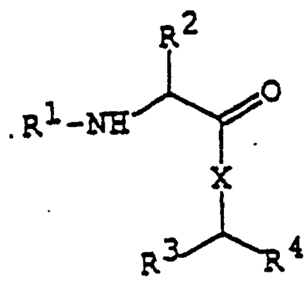

(I)

dans laquelle

$R^1$ est alcoxy(en $C_1$-$C_4$)carbonyle;
un phénylalcanoyle en $C_1$-$C_4$ ou un naphtylalcanoyle en $C_1$-$C_4$, chacun de ceux-ci peut avoir un carboxyalkyle en $C_1$-$C_4$, un alcoxy(en $C_1$-$C_4$)carbonylalkyle en $C_1$-$C_4$ qui peut être substitué par un phényle, un alcoxy (en $C_1$-$C_4$)carbonylalcényle en $C_2$-$C_4$, un carbamoylalkyle en $C_1$-$C_4$ ou un phénylalkyle en $C_1$-$C_4$;
un hétérocycle-alcanoyle en $C_1$-$C_4$ qui peut avoir un pyridylalkyle en $C_1$-$C_4$, un naphtylalkyle en $C_1$-$C_4$ ou un phénylalkyle en $C_1$-$C_4$ qui peut avoir 1 à 3 substituants appropriés choisis dans le groupe constitué par un alkyle en $C_1$-$C_4$ et un halogène, dans lequel le fragment hétérocyclique est un indolyle, un quinolyle ou un isoquinolyle,
$R^2$ est carboxyalkyle en $C_1$-$C_4$, un méthoxycarbonylalkyle en $C_1$-$C_4$, ou un benzyloxycarbonylalkyle en $C_1$-$C_4$,
$R^3$ est un phénylalkyle en $C_1$-$C_4$ qui peut avoir un alkyle en $C_1$-$C_4$, un alkyle en $C_7$-$C_{16}$ ou un phényle;
un naphtylalkyle en $C_1$-$C_4$ qui peut avoir un alkyle en $C_1$-$C_4$;
un phénylalkyle en $C_7$-$C_{16}$;
un benzofuranylalkyle en $C_1$-$C_4$;
un alcoxy(en $C_7$-$C_{16}$)alkyle en $C_1$-$C_4$;
un alkyle en $C_3$-$C_6$; ou
un alkyle en $C_7$-$C_{16}$,
$R^4$ est un carbamoylalkyle en $C_1$-$C_4$, et
X est -O-, -NH- ou

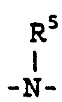

[où $R^5$ est un alkyle en $C_1$-$C_5$, un phénylalkyle en $C_1$-$C_4$, ou un pyridylalkyle en $C_1$-$C_4$],

avec la condition que X soit

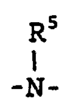

(où $R^5$ est tel que défini ci-dessus), lorsque $R^3$ est un alkyle en $C_3$-$C_6$ ou un alkyle en $C_7$-$C_{16}$,
et un sel pharmaceutiquement acceptable de celui-ci.

**2.** Composé selon la revendication 1, dans lequel

$R^1$ est un indolylalcanoyle en $C_1$-$C_4$ qui peut avoir un substituant approprié choisi dans le groupe constitué par un pyridylalkyle en $C_1$-$C_4$, un naphtylalkyle en $C_1$-$C_4$, un phénylalkyle en $C_1$-$C_4$, un alkyl(en $C_1$-$C_4$)phénylalkyle en $C_1$-$C_4$, et un halophénylalkyle en $C_1$-$C_4$,
$R^2$ est un carboxyalkyle en $C_1$-$C_4$,
$R^3$ est un alkyle en $C_3$-$C_6$ ou un alkyle en $C_7$-$C_{16}$,
$R^4$ est un carbamoylalkyle en $C_1$-$C_4$, et
X est

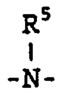

[où $R^5$ est un alkyle en $C_1$-$C_5$].

3. Composé selon la revendication 2 qui est choisi dans le groupe constitué par

(1) le (3S)-3-[N-(n-propyl)-{(2S)-5-carboxy-2-[(1-(2-chlorobenzyl)indol-3-ylcarbonyl)amino]pentanoyl}amino] nonanamide,
(2) le (3S)-3-[N-(n-propyl)-{(2S)-2-(1-(2-chlorobenzyl)indol-3-ylcarbonyl)amino-5-carboxypentanoyl}amino]-heptanamide,
(3) le (3S)-3-[N-(n-propyl)-{(2S)-2-(1-benzylindol-3-yl-carbonyl)amino-5-carboxypentanoyl}amino]dodécanamide,
(4) le (3S)-3-[N-(n-propyl)-{(2S)-2-(1-(2-chlorobenzyl)indol-3-ylcarbonyl)amino-5-carboxypentanoyl}amino]-dodécanamide,
(5) le (3S)-3-[N-éthyl-{(2S)-2-(1-(2-chlorobenzyl)indol-3-yl-carbonyl)amino-5-carboxypentanoyl}amino]nonanamide,
(6) le (3S)-3-[N-éthyl-{(2S)-2-(1-benzylindol-3-yl-carbonyl)amino-5-carboxypentanoyl}amino]nonanamide,
(7) le (3S)-3-[N-(n-butyl)-{(2S)-2-(1-(1-naphtylméthyl)-indol-3-ylcarbonyl)amino-5-carboxypentanoyl}amino]-heptanamide, et
(8) le (3S)-3-[N-(n-propyl)-{(2S)-2-(1-benzylindol-3-yl-carbonyl)amino-5-carboxypentanoyl}amino]nonanamide,

ou un sel pharmaceutiquement acceptable de celui-ci

4. Procédé pour préparer un composé de formule :

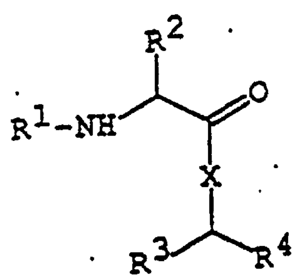

(I)

dans laquelle
$R^1$, $R^2$, $R^3$, $R^4$ et X sont chacun tels que définis dans la revendication 1,
ou un sel de celui-ci, qui comprend les étapes consistant à

1) faire réagir le composé de formule :

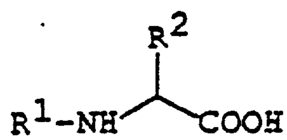

dans laquelle $R^1$ et $R^2$ sont chacun tels que définis ci-dessus,
ou un dérivé réactif au groupe carboxy
ou un sel de celui-ci, avec le composé de formule :

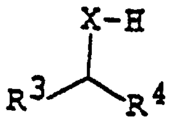

dans laquelle $R^3$, $R^4$ et X sont chacun tels que définis ci-dessus, ou un sel de celui-ci, ou

2) faire réagir le composé de formule :

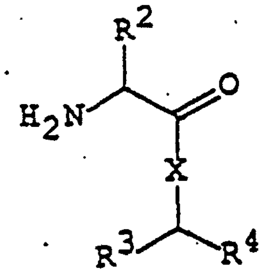

dans laquelle $R^2$, $R^3$, $R^4$ et X sont chacun tels que définis ci-dessus, ou un dérivé réactif au groupe amino ou un sel de celui-ci, avec le composé de formule :

$$R^1 - OH$$

dans laquelle $R^1$ est tel que défini ci-dessus, ou un dérivé réactif ou un sel de celui-ci, ou

3) soumettre le composé de formule :

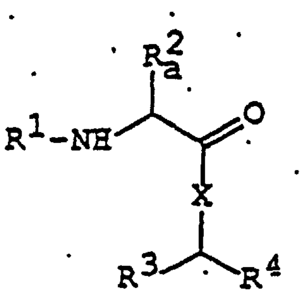

dans laquelle

| | |
|---|---|
| $R^1$, $R^3$, $R^4$ et X | sont chacun tels que définis ci-dessus, et |
| $R^2_a$ | est un groupe carboxy(protégé)alkyle en $C_1$-$C_4$, |

ou un sel de celui-ci, à une réaction d'élimination du groupe protecteur du carboxy, pour donner le composé de formule :

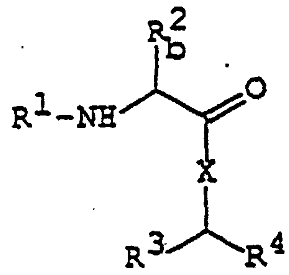

dans laquelle

| | |
|---|---|
| $R^1$, $R^3$, $R^4$ et X | sont chacun tels que définis ci-dessus, et |
| $R^2_b$ | est un carboxyalkyle en $C_1$-$C_4$, |

ou un sel de celui-ci.

**5.** Composition pharmaceutique qui comprend, en tant qu'ingrédient actif, un dérivé d'acide gras de la revendication 1 ou un sel pharmaceutiquement acceptable de celui-ci en mélange avec des supports ou excipients pharmaceutiquement acceptables.

**6.** Utilisation d'un dérivé d'acide gras de la revendication 1 ou d'un sel pharmaceutiquement acceptable de celui-ci pour la fabrication d'un médicament.

**7.** Dérivé d'acide gras de la revendication 1 ou sel pharmaceutiquement acceptable de celui-ci pour utilisation comme médicament.

**8.** Utilisation d'un dérivé d'acide gras ou d'un sel pharmaceutiquement acceptable de celui-ci selon la revendication 1 pour la fabrication d'un médicament pour la prévention et/ou le traitement d'une pancréatite, d'une hépatite, d'une insuffisance rénale, d'un choc, d'une arthrite, d'une maladie respiratoire, d'une maladie cardiaque, d'une maladie allergique, d'une thrombose, d'une artériosclérose, d'une douleur, d'une maladie auto-immune, d'une maladie de la peau, d'une maladie inflammatoire de l'intestin, d'une maladie ophtalmique, de maladies nasales, de la goutte, de maladies du foie ou inflammation provoquées par un trauma chez un être humain ou un animal.